# EUROPEAN PATENT SPECIFICATION

(11) **EP 2 530 148 B1**
(45) Date of publication and mention of the grant of the patent: **29.04.2015**
(21) Application number: 11004424.5
(22) Date of filing: 30.05.2011
(51) Int. Cl.: C12N 9/38, A23C 9/12

(54) **Novel beta-galactosidases useful for the production of lactose depleted milk products**
Neue beta-Galactosidasen zur Herstellung von lactoseverarmten Milchprodukten
Nouveaux béta-galactosidases utiles pour la production de produits laitiers appauvris en lactose

(43) Date of publication of application: 05.12.2012
(73) Proprietor: B.R.A.I.N. Biotechnology Research And Information Network AG, 64673 Zwingenberg (DE)
(72) Inventor: Niehaus, Frank, 64646 Heppenheim (DE); Eck, Jürgen, 64625 Bensheim-Auerbach (DE)
(74) Representative: Vossius & Partner Patentanwälte Rechtsanwälte mbB

(56) References cited:
- WO-A1-2010/092057
- DATABASE UniProt [Online] 8 March 2011 (2011-03-08), "SubName: Full=Beta-galactosidase;", XP002663059, retrieved from EBI accession no. UNIPROT:E5YRW7 Database accession no. E5YRW7 & SIROTA-MADI ALEXANDRA ET AL: "Genome sequence of the pattern forming Paenibacillus vortex bacterium reveals potential for thriving in complex environments", BMC GENOMICS, BIOMED CENTRAL, LONDON, GB, vol. 11, no. 1, 17 December 2010 (2010-12-17), page 710, XP021086311, ISSN: 1471-2164, DOI: 10.1186/1471-2164-11-710
- DATABASE UniProt [Online] 23 March 2010 (2010-03-23), "SubName: Full=Beta-galactosidase; EC=3.2.1.23;", XP002663060, retrieved from EBI accession no. UNIPROT:D3EDU4 Database accession no. D3EDU4
- COKER J A ET AL: "Biochemical Characterization of a ss-Galactosidase with a Low Temperature Optimum Obtained from an Antarctic Arthrobacter Isolate", JOURNAL OF BACTERIOLOGY, AMERICAN SOCIETY FOR MICROBIOLOGY, WASHINGTON, DC; US, vol. 185, no. 18, 1 September 2003 (2003-09-01), pages 5473-5482, XP002579364, ISSN: 0021-9193, DOI: 10.1128/JB.185.18.5473-5482.2003
- HUSAIN QAYYUM: "beta Galactosidases and their potential applications: a review", CRITICAL REVIEWS IN BIOTECHNOLOGY, vol. 30, no. 1, March 2010 (2010-03), pages 41-62, XP9153894, ISSN: 0738-8551

## Description

The invention relates to a nucleic acid molecule encoding (I) a polypeptide having the activity of a beta-galactosidase (EC 3.2.1.23), which beta-galactosidase is characterized in that it has (i) sufficient enzymatic activity in the range 5 to 50°C to catalyze the hydrolysis of ß-galactosidases into monosaccharides, (ii) sufficient enzymatic activity at a pH range from 6 to 8 to catalyze the hydrolysis of ß-galactosidases into monosaccharides, and (iii) an enzymatic activity that is not inhibited by reaction products such as galactose, or other products substantially present in milk, such as calcium, which beta-galactosidase is encoded by (a) a nucleic acid molecule encoding a polypeptide comprising or consisting of the amino acid sequence of SEQ ID NO: 1; (b) a nucleic acid molecule comprising or consisting of the nucleotide sequence of SEQ ID NO: 2; (c) a nucleic acid molecule encoding a beta-galactosidase the amino acid sequence of which is at least 70% identical to the amino acid sequence of SEQ ID NO: 1; (d) a nucleic acid molecule comprising or consisting of a nucleotide sequence which is at least 70% identical to the nucleotide sequence of SEQ ID NO: 2; or (e) a nucleic acid molecule corresponding to the nucleic acid molecule of any one of (a) to (d) wherein T is replaced by U; (II) a fragment of the polypeptide of (I) having the activity of a beta-galactosidase as defined in (I) and comprising at least 800 amino acids.

Beta-Galactosidases (or lactases or beta-D-galactoside hydrolases, EC 3.2.1.23) are enzymes that catalyze the hydrolysis of the disaccharide lactose into its monosaccharide components beta-D-glucose and beta-D-galactose. Besides some enzymes in this group hydrolyse alpha-L-arabinosides and some animal enzymes also hydrolyse beta-D-fucosides and beta-D-glucosides such as cellobiose. Lactose is a component of mammalian milk. It is also contained in the products that have been prepared from milk for consumption by humans like yoghurt, cheese, fresh milk, ice cream, whipped cream or other dairy products. Fresh cow's milk contains between 4.5 % and 5 % w/w lactose.

The presence of lactose in food compositions can have two unwanted effects after consumption. While infants have the ability to digest lactose, because it is an important ingredient of the mother milk, a huge part of the human population loose this ability with increasing age due to a loss of the production of beta-galactosidase in the digestive tract. As a result, these individuals experience adverse well-being effects like bloating, flatulence and diarrhea. Removal of lactose from milk and milk products leads to disappearance of these symptoms.

Since lactose intolerance is widely distributed throughout the world population, a labeling of milk-containing food with "lactose-free" or "lactose-depleted" will be of significant economic importance.

Another effect of lactose in milk products is the unwanted change of the organoleptic feeling of some products. In "dulce de leche" products, which are mainly produced and consumed in the Rio de la Plata region in South America, the presence of lactose can lead to a gritty mouth feeling, which is also described as "sandiness". This effect is mainly caused by the crystallization of lactose in these products. A similar effect can be observed in ice cream, where lactose crystallizes itself or forms seed crystals for the crystallization of water. In both cases the crystals of either the lactose or, in case of ice cream, the crystals of lactose and/or water lead to the unwanted texture and adverse mouth feeling.

Lactose can be removed from milk and dairy products by physical and chemical methods.

One way of depleting lactose from dairy products is a chromatographic process as disclosed e.g. in EP 226035 B1. Here, a specific chromatographic separation of lactose from milk is described. Milk is treated in a column with a cation exchange resin at a temperature of about 50 to about 80°C yielding lactose depleted milk and after elution of the column a pure lactose solution. The advantage of this process is that all ingredients which are relevant for taste remain in the milk.

A common physical removal is the use of filtration based techniques. It is either used to perform an ultrafiltration step in which the lactose is enriched in the filtrate, while most of the other milk ingredients are retained in the treated milk. The modified milk can then be added to milk products, which leads in fact to a milk or dairy product with a reduced lactose concentration. Diafiltration is a modification of ultrafiltration, which leads to a milk-derived product that can be lactose-free. The combination of ultrafiltration and diafiltration leads to milk products that have a high concentration of fat and protein, but a very low lactose and salt concentration. Thus, it leads to an enrichment of water insoluble milk components. A selective filtration procedure to separate lactose from milk is described in e.g. WO 2003/094623

However, all methods of lactose depletion employing separation processes require complex machinery and are not applicable to all dairy products. Consequently, the enzymatic hydrolysis of lactose has been a topic in applied research for several decades. The enzyme beta-galactosidase catalyzes the hydrolysis of lactose disaccharide into the monosaccharides glucose and galactose. beta-Galactosidases have been obtained from microorganisms such as fungi, bacteria and yeasts, plants, animals cells, and from recombinant sources. The enzyme has two main applications: the removal of lactose from milk products for lactose intolerant people and the production of galactosylated products. Free and immobilized preparations of β galactosidases have been exploited in various processes such as in industrial, biotechnological, medical and analytical, and in different other applications. Immobilized β-galactosidases are employed for the continuous hydrolysis of lactose from whey and milk in a number of reactors (Q. Husain, β-Galactosidases and their potential applications: a review, Critical Reviews in Biotechnology, March 2010, Vol. 30, No. 1, pages 41-62). Besides the hydrolysis of lactose with beta-galactosidases, it is also possible to employ beta-galactosidase for the synthesis of beta-galacto-oligosacharides from beta-D-galactose and/or lactose. Beta-Galacto-oligosaccharides consist mainly of beta(1-6) linked galactopyranosil units linked to a terminal glucopyranosyl residue via an alpha(1-4) glycosidic bond. In general oligosaccharides have a chain length of between two to ten monosaccharides. While beta-galacto-oligosaccharides are only found in trace amounts in cow's milk, their concentration is relatively high in human milk. Human milk contains between 5 to 12 g beta-galacto-oligosacharides per liter. Beta-Galacto-oligosacharides are used as prebiotic compounds in food preparation. Beta-Galacto-oligosacharides are a very important ingredient of artificial infant milk. The addition to these products allows a composition, which is close to human milk. It is therefore of great interest to have an efficient production route for beta-galacto-oligosacharides, which is done by applying beta-galactosidases under the appropriate conditions.

Enzymatic treatment of milk with beta-galactosidase leads under the currently used conditions to a sweet milk product with a lactose concentration of less than 0.1% lactose. Almost all beta-galactosidases, that are used in the dairy industry as of today are of microbial origin. Depending on the manufacturing and down-stream processing procedures the beta-galactosidases can have unwanted proteolytic side activities on milk proteins, which can lead to an off-taste in the lactose free milk or milk products. The beta-galactosidase treated milk products do also taste much sweeter than untreated milk, because the glucose concentration is increased by the treatment. Another side effect of the beta-galactosidase treatment caused by the increased concentration of reducing sugars is the higher tendency to browning due an increased occurrence of Maillard reactions between the sugars and the proteins.

Many beta-galactosidases show inhibition by their reaction products. Mainly D-galactose, but also D-glucose inhibit lactose hydrolysis above a certain concentration. Product inhibition can be a severe problem in technical applications, because the formation of the reaction product leads to a complete inhibition of the hydrolysis reaction and prevents degradation of lactose in milk.

The reported pH-range of beta-galactosidases varies between 1 and 10. The optimal pH-values of lactose hydrolysis are often found in the slightly acidic to neutral values, but there are also a number of enzymes that optimally function in the slightly alkaline range between pH 8 and 9.

The reported temperature range for beta-galactosidase activity spans almost the complete temperature range of liquid water between 0° and 95° C. The majority of the beta-galactosidases exhibit optimal activity at 37°C.

Beta-galactosidases can be found in a variety of organisms that include but are not limited to *Achatina achatina, Actinomyces viscosus, Aeromonas punctata, Alicyclobacillus acidocaldarius, Alternaria alternata, Arabidopsis thaliana, Arthrobacter psychrolactophilus, Aspergillus aculeatus, Aspergillus awamori, Aspergillus carbonarius, Aspergillus cellulosae, Aspergillus flavus, Aspergillus japonicus, Aspergillus niger, Aspergillus oryzae, Averrhoa carambola, Bacillus circulans, Bacillus coagulans, Bacillus licheniformis, Bacillus megaterium, Bacillus subtilis, Bacteroides polypragmatus, Bifidobacterium adolescentis, Bifidobacterium animalis, Bifidobacterium bifidum, Bifidobacterium longum, Bifidobacterium longum subsp. infantis, Bos taurus, Bullera singularis, Caldariella acidophila, Caldicellulosiruptor saccharolyticus, Canis lupus familiaris, Capra hircus, Clostridium perfringens, Coffea sp., Corynebacterium murisepticum, Cryptococcus laurentii, Cupressus sempervirens, Curvularia inaequalis, Daucus carota, Enterobacter aerogenes, Enterobacter cloacae, Erythrina variegata, Escherichia coli, Felis catus, Flavobacterium sp., Fusarium oxysporum, Galactomyces geotrichum, Geobacillus stearothermophilus, Gibberella moniliformis, Guehomyces pullulans, Helix pomatia, Hesperocyparis arizonica, Homo sapiens, Hordeum vulgare, Humicola grisea, Hymenaea courbaril, Hypocrea jecorina, Irpex lacteus, Kerstingiella geocarpa, Kluyveromyces lactis, Kluyveromyces marxianus, Kluyveromyces marxianus CDBBL 278*, *Lactis kluveromyces, Lactobacillus acidophilus, Lactobacillus amylovorus, Lactobacillus casei, Lactobacillus crispatus, Lactobacillus delbrueckii, Lactobacillus delbrueckii subsp. bulgaricus, Lactobacillus delbrueckii subsp. lactis, Lactobacillus fermentum, Lactobacillus helveticus, Lactobacillus johnsonii, Lactobacillus mucosae, Lactobacillus murinus, Lactobacillus oris, Lactobacillus plantarum, Lactobacillus plantarum WCFS1, Lactobacillus reuteri, Lactobacillus salivarius, Lactobacillus thermophilus, Lactobacillus vaginalis, Lactococcus lactis, Leuconostoc citrovorum, Loktanella hongkongensis, Malus sp., Marchantia polymorpha, Medicago sativa, Mucor javanicus, Mucor mucedo, Mus musculus, Myceliophthora thermophila, Neurospora crassa, Olea europaea, Oreochromis niloticus, Oryctolagus cuniculus, Oryza sativa, Ovis aries, Paecilomyces variotii, Paenibacillus macerans, Pantoea agglomerans, Paracolobactrum aerogenoides, Penicillium canescens, Penicillium chrysogenum, Penicillium citrinum, Penicillium cyclopium, Penicillium glabrum, Penicillium glaucum, Penicillium multicolor, Penicillium roqueforti, Penicillium toxidarium, Phaseolus vulgaris, Pisum sativum, Planococcus sp., Prunus armeniaca, Prunus dulcis, Prunus persica, Pseudoalteromonas haloplanktis, Pseudomonas fluorescens, Pseudomonas putida, Pycnoporus cinnabarinus, Pyrococcus woesei, Raphanus sativus, Rattus norvegicus, Rhizobium leguminosarum, Rhizomucor miehei, Rhizomucor pusillus, Rhizomucor sp., Rhizopus acidus, Rhizopus formosciensis, Rhizopus javanicus, Rhizopus microsporus var. chinensis, Rhizopus niveus, Rhizopus oryzae, Rhizopus stolonifer, Saccharomyces anamensis, Saccharomyces cerevisiae, Saccharomyces fragilis, Saccharomyces lactis, Saccharopolyspora rectivirgula, Saccharum officinarum, Scopulariopsis sp., Shigella dysenteriae, Shigella sonnei, Sinorhizobium meliloti, Sporobolomyces singularis, Sporotrichum sp., Sterigmatomyces elviae, Streptococcus mitis, Streptococcus pneumoniae, Streptococcus thermophilus, Streptomyces coelicolor, Sulfolobus solfataricus, Talaromyces luteus, Talaromyces thermophilus, Tenebrio molitor, Thalassobius gelatinovorus, Thalassococcus halodurans,*

*Thermoanaerobacterium thermosulfurigenes, Thermoanaerobium sp., Thermomyces lanuginosus, Thermotoga maritima, Thermus aquaticus, Torula thermophila, Torulopsis sphaerica, Torulopsis versatilis, Treponema phagedenis, Trichoderma viride, Triticum aestivum, Tritrichomonas foetus, Umbelopsis vinacea, Vigna radiata, Xanthomonas axonopodis pv. manihotis, Xanthomonas campestris, Yersinia pestis, Zygosaccharomyces lactis.*

Despite of a plenitude of microbial beta-galactosidases described in the literature, industrial use of microbial beta-galactosidases is restricted to a small number of enzyme preparations especially from yeast such as Kluyveromyces lactis and Kluyveromyces fragilis (see e.g. Review by Rubio-Texeira M., Endless versatility in the biotechnological applications of Kluyveromyces LAC genes, Biotechnol Adv. 2006 Mar-Apr; 24(2):212-25) or from Aspergillus spec. (e.g. US 5821350).

Beta-galactosidases are selected by their respective application area. Beta-galactosidases for the treatment of whey based milk products operate preferably at slightly acidic pH (4.5 to 6), which is the natural pH of whey, while beta-galactosidases for the treatment of milk operate mainly under neutral conditions around pH 7.

Commercial enzyme products for industrial applications are either supplied as liquid or solid formulations. Immobilized beta-galactosidases are employed for the continuous hydrolysis of lactose from whey and milk in a number of reactors (Q. Husain, β-Galactosidases and their potential applications: a review, Critical Reviews in Biotechnology, March 2010, Vol. 30, No. 1, pages 41-62).

The Maxilact® product line from DSM comprises enzymes from the yeast *Kluyveromyces lactis.* Maxilact® LX5000 is a highly purified product. The product Maxilact®LGX has been purified in order to be completely arylsulphatase free, which enhances the taste of the treated milk. Tolerase® is an *Aspergillus oryzae* derived enzyme product from DSM for the treatment of whey based materials. The Danish Chr. Hansen is selling a *K. lactis*-derived product under the trade name Ha-lactase. Lactozym pure® is the lactase product line of Novozymes®. The products in this line are sold in a variety of enzyme concentrations. Godo YNL-2 is another technical beta-galactosidase derived form *K*. *lactis* sold by the Japanese company GODO Shushei.

Beta-Galactosidases are not only sold as technical enzyme products for the dairy industry, but these enzymes are also made available as over the counter products in drug stores in pill or capsule form for consumer use as digestive supplement. The contents of the pills or capsules can be mixed with the fresh home prepared milk products, which still contain lactose, to enable symptom free consumption of these products through in situ lactose hydrolysis. Examples for these pills are "Digestive Advantage", "Lactase Enzyme", "Say Yes to Dairy", "Life like Lactase", "Biolabor Laktase", "Lactrase" or the like.

The current commercial beta-galactosidases show some characteristics that are rather unfavourable for the establishment of an industrial process of treating milk or milk-containing products. These are amongst others a) a low affinity for the substrate lactose (high Km-value), resulting in high amount of enzyme needed and a long reaction time, which in turn leads to high costs for enzyme and hygiene measures; b) inhibition of the beta-galactosidase enzyme by the products of lactose hydrolysis, i.e. glucose and galactose, and by calcium ions; c) a temperature optimum of 35-37°C involving an increased risk of contamination by microorganisms; d) an undesired protease activity leading to the generation of bitter taste caused by peptides from milk proteins; e) an activity restricted to neutral pH.

The conventional approach in food processing is to carry out the hydrolysis of lactose at 40°C during approximately four hours. However, milk or lactose solution as a raw material is a preferable nutrition source for bacteria. As the result, the putrefaction owing to the saprophyte contamination during the treatment is a serious problem in the food production. To overcome problems of contamination one approach is to run the process of lactose cleavage at elevated temperatures using thermophilic enzymes as described for example in EP 0006035 B1, US 4237230 and US 4007283. One drawback of this procedure is high energy costs.

Several cold-active beta-D-galactosidases dervied from different organisms have been described, e.g. from *Arthrobacter* as descibed by Hildebrand et al. (A new cold-adapted beta-D-Galactosidase from the Antarctic Arthrobacter ap. 32c - gene cloning, overexpression, purification and properties. BMC Microbiol., 2009, 9:151), from the psychrophilic bacterium *Pseudoalteromonas* (WO 2001/004276 A1) or from the psychrophilic bacterium *Alkalilactibacillus* in WO 2010/092057 A1. However, these beta-galactosidases generally known in the prior art, when used for food processing, all have one or more disadvantages such as low enzyme activity and low stability at a temperature above 20°C, narrow range of optimum pH and the inhibition of enzymatic action by a reaction product, such as galactose or others products or ingredients of milk, in particular calcium.

As it is evident from the above there is an ongoing need for further beta-galactosidases, in particular novel beta-galactosidases which overcome one ore more of the deficiencies of the existing beta-galactosidases describes herein above. This need is addressed by the present invention.

Accordingly, the present invention relates in a first embodiment to a nucleic acid molecule encoding (I) a polypeptide having the activity of a beta-galactosidase (EC 3.2.1.23), which beta-galactosidase is characterized in that it has (i) sufficient enzymatic activity in the range 5 to 50°C to catalyze the hydrolysis of ß-galactosidases into monosaccharides, (ii) sufficient enzymatic activity at a pH range from 6 to 8 to catalyze the hydrolysis of ß-galactosidases into monosaccharides, and (iii) an enzymatic activity that is not inhibited by reaction products such as galactose, or other products substantially present in milk, such as calcium, which beta-galactosidase is encoded by (a) a nucleic acid molecule encoding a polypeptide comprising or consisting of the amino acid sequence of SEQ ID NO: 1; (b) a nucleic acid molecule comprising or consisting of the nucleotide sequence of SEQ ID NO: 2; (c) a nucleic acid molecule encoding a beta-galactosidase the amino acid sequence of which is at least 70% identical to the amino acid sequence of SEQ ID NO: 1; (d) a nucleic acid molecule comprising or consisting of a nucleotide sequence which is at least 70% identical to the nucleotide sequence of SEQ ID NO: 2; or (e) a nucleic acid molecule corresponding to the nucleic acid molecule of any one of (a) to (d) wherein T is replaced by U; (II) a fragment of the polypeptide of (I) having the activity of a beta-galactosidase as defined in (I) and comprising at least 800 amino acids.

Also described herein is a nucleic acid molecule encoding (I) a polypeptide having the activity of a beta-galactosidase (EC 3.2.1.23), which is (a) a nucleic acid molecule encoding a polypeptide comprising or consisting of the amino acid sequence of SEQ ID NO: 3; (b) a nucleic acid molecule comprising or consisting of the nucleotide sequence of SEQ ID NO: 4; (c) a nucleic acid molecule encoding a beta-galactosidase the amino acid sequence of which is at least 68% identical to the amino acid sequence of SEQ ID NO: 3, (d) a nucleic acid molecule comprising or consisting of a nucleotide sequence which is at least 68% identical to the nucleotide sequence of SEQ ID NO: 4, (e) a nucleic acid molecule which is degenerate with respect to the nucleic acid molecule of (d); or (f) a nucleic acid molecule corresponding to the nucleic acid molecule of any one of (a) to (e) wherein T is replaced by U; (II) a fragment of the polypeptide of (I) having the activity of a beta-galactosidase.

Also described herein is a nucleic acid molecule encoding (I) a polypeptide having the activity of a beta-galactosidase (EC 3.2.1.23), which is (a) a nucleic acid molecule encoding a polypeptide comprising or consisting of the amino acid sequence of SEQ ID NO: 5; (b) a nucleic acid molecule comprising or consisting of the nucleotide sequence of SEQ ID NO: 6; (c) a nucleic acid molecule encoding a beta-galactosidase the amino acid sequence of which is at least 64% identical to the amino acid sequence of SEQ ID NO: 5, (d) a nucleic acid molecule comprising or consisting of a nucleotide sequence which is at least 63% identical to the nucleotide sequence of SEQ ID NO: 6, (e) a nucleic acid molecule which is degenerate with respect to the nucleic acid molecule of (d); or (f) a nucleic acid molecule corresponding to the nucleic acid molecule of any one of (a) to (e) wherein T is replaced by U; (II) a fragment of the polypeptide of (I) having the activity of a beta-galactosidase.

Also described herein is a nucleic acid molecule encoding (I) a polypeptide having the activity of a beta-galactosidase (EC 3.2.1.23), which is (a) a nucleic acid molecule encoding a polypeptide comprising or consisting of the amino acid sequence of SEQ ID NO: 7; (b) a nucleic acid molecule comprising or consisting of the nucleotide sequence of SEQ ID NO: 8; (c) a nucleic acid molecule encoding a beta-galactosidase the amino acid sequence of which is at least 75% identical to the amino acid sequence of SEQ ID NO: 7, (d) a nucleic acid molecule comprising or consisting of a nucleotide sequence which is at least 71% identical to the nucleotide sequence of SEQ ID NO: 8, (e) a nucleic acid molecule which is degenerate with respect to the nucleic acid molecule of (d); or (f) a nucleic acid molecule corresponding to the nucleic acid molecule of any one of (a) to (e) wherein T is replaced by U; (II) a fragment of the polypeptide of (I) having the activity of a beta-galactosidase.

In accordance with the present invention the term "nucleic acid molecule" defines a linear molecular chain consisting of preferably more than 2400 nucleotides. The group of molecules designated herein as "nucleic acid molecules" also comprises complete genes. The term "nucleic acid molecule" is interchangeably used herein with the term "polynucleotide".

The term "nucleic acid molecule" in accordance with the present invention includes DNA, such as cDNA or double or single stranded genomic DNA and RNA. In this regard, "DNA" (deoxyribonucleic acid) means any chain or sequence of the chemical building blocks adenine (A), guanine (G), cytosine (C) and thymine (T), called nucleotide bases, that are linked together on a deoxyribose sugar backbone. DNA can have one strand of nucleotide bases, or two complimentary strands which may form a double helix structure. "RNA" (ribonucleic acid) means any chain or sequence of the chemical building blocks adenine (A), guanine (G), cytosine (C) and uracil (U), called nucleotide bases, that are linked together on a ribose sugar backbone. RNA typically has one strand of nucleotide bases. Included are also single- and double-stranded hybrid molecules, i.e., DNA-DNA, DNA-RNA and RNA-RNA. The nucleic acid molecule may also be modified by many means known in the art. Non-limiting examples of such modifications include methylation, "caps", substitution of one or more of the naturally occurring nucleotides with an analog, and internucleotide modifications such as, for example, those with uncharged linkages (e.g., methyl phosphonates, phosphotriesters, phosphoroamidates, carbamates, etc.) and with charged linkages (e.g., phosphorothioates, phosphorodithioates, etc.). Polynucleotides may contain one or more additional covalently linked moieties, such as, for example, proteins (e.g., nucleases, toxins, antibodies, signal peptides, poly-L-lysine, etc.), intercalators (e.g., acridine, psoralen, etc.), chelators (e.g., metals, radioactive metals, iron, oxidative metals, etc.), and alkylators. The polynucleotides may be derivatized by formation of a methyl or ethyl phosphotriester or an alkyl phosphorarnidate linkage. Further included are nucleic acid mimicking molecules known in the art such as synthetic or semi-synthetic derivatives of DNA or RNA and mixed polymers. Such nucleic acid mimicking molecules or nucleic acid derivatives according to the invention include phosphorothioate nucleic acid, phosphoramidate nucleic acid, 2'-O-methoxyethyl ribonucleic acid, morpholino nucleic acid, hexitol nucleic acid (HNA), peptide nucleic acid (PNA) and locked nucleic acid (LNA) (see Braasch and Corey, Chem Biol 2001, 8: 1). LNA is an RNA derivative in which the ribose ring is constrained by a methylene linkage between the 2'-oxygen and the 4'-carbon. Also included are nucleic acids containing modified bases, for example thio-uracil, thio-guanine and fluoro-uracil. A nucleic acid molecule typically carries genetic information, including the information used by cellular machinery to make proteins and/or polypeptides. The nucleic acid molecule of the invention may additionally comprise promoters, enhancers, response elements, signal sequences, polyadenylation sequences, introns, 5'- and 3'- non-coding regions, and the like.

The term "polypeptide" as used herein interchangeably with the term "protein" describes linear molecular chains of amino acids, including single chain proteins, preferably containing more than 800 amino acids. Polypeptides may further form oligomers consisting of at least two identical or different molecules. The corresponding higher order structures of such multimers are, correspondingly, termed homo- or heterodimers, homo- or heterotrimers etc.. The polypeptides of the invention may form heteromultimers or homomultimers, such as heterodimers or homodimers. Furthermore, peptidomimetics of such proteins/polypeptides where amino acid(s) and/or peptide bond(s) have been replaced by functional analogues are also encompassed by the invention. Such functional analogues include all known amino acids other than the 20 gene-encoded amino acids, such as selenocysteine. The terms "polypeptide" and "protein" also refer to naturally modified polypeptides and proteins where the modification is effected e.g. by glycosylation, acetylation, phosphorylation, ubiqitinylation and similar modifications which are well known in the art.

The term "a polypeptide having the activity of a beta-galactosidase" as used herein means a polypeptide which catalyzes the hydrolysis of β-galactosides into monosaccharides. Substrates of β-galactosidases include, for example, ganglioside GM1, lactosylceramides, lactose, and various glycoproteins.

A β-galactoside is a type of galactoside in which the glycosidic bond lies above the plane of the galactose residue. Two of the most commonly recognized and used β-galactosides are lactose and beta-D-galactose. Lactose is a disaccharide sugar that is found most notably in milk and is formed from galactose and glucose. Lactose makes up around 2-8% of milk (by weight), although the amount varies among species and individuals. It is, for example, extracted from sweet or sour whey. Beta-D-galactose and glucose are the two monosaccharide sugar components that make up the disaccharide sugar, lactose. They form a β-1→4 glycosidic linkage.

According to the invention, an enzymatic activity is considered as stable when, in the respective conditions, the enzyme is capable of lasting long enough to obtain the desired effect, for example, the hydrolysis of a substrate. In this regard it is noted that beta-galactosidase activity can be assayed as described in the examples of the specification or by methods well known in the art.

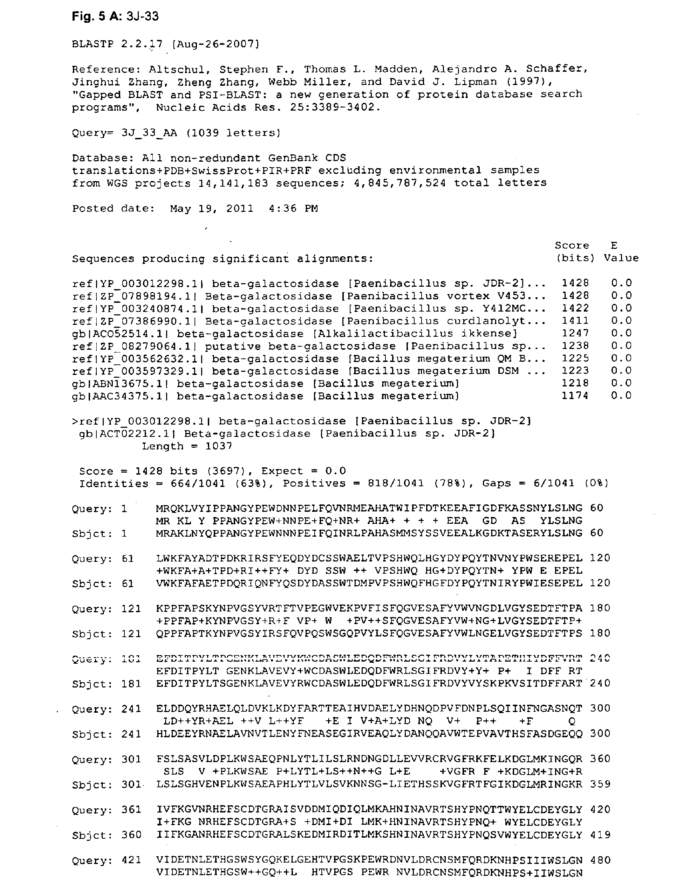

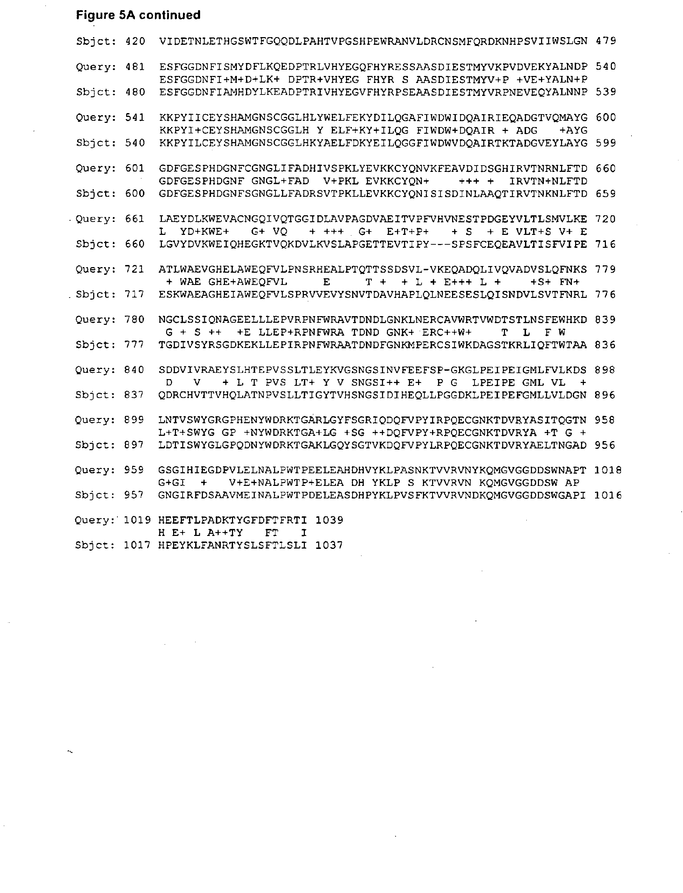

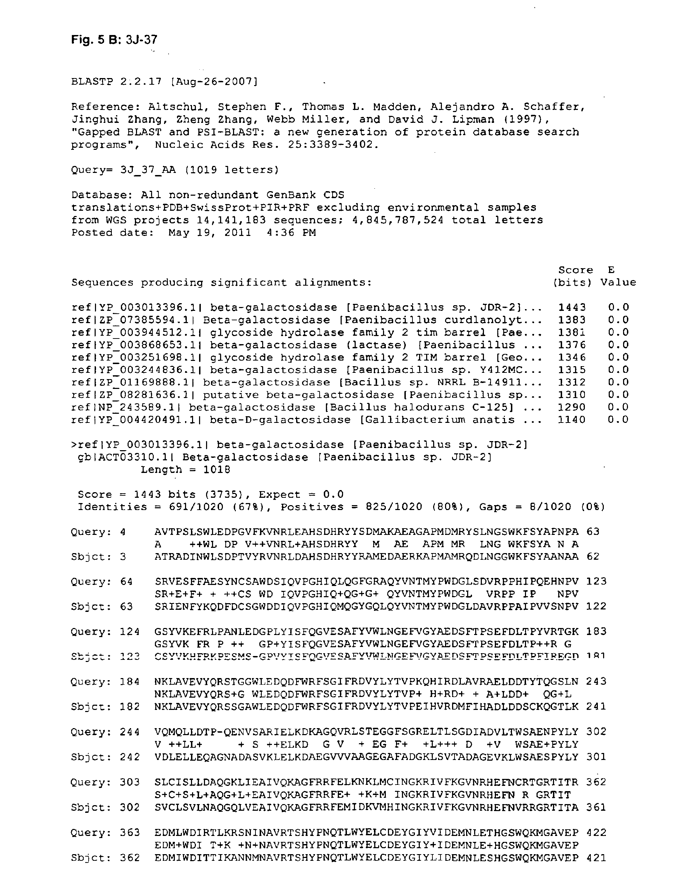

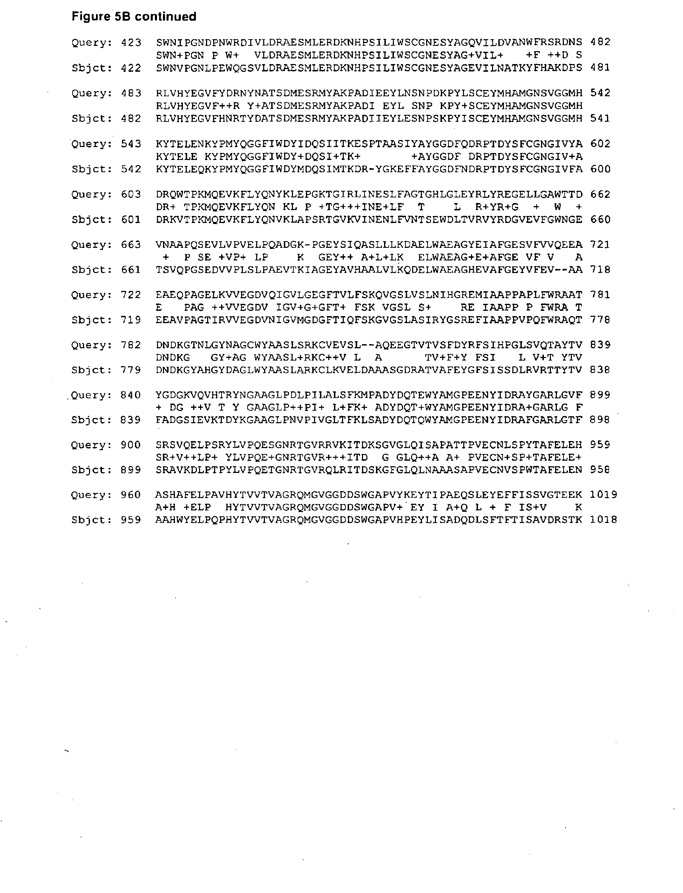

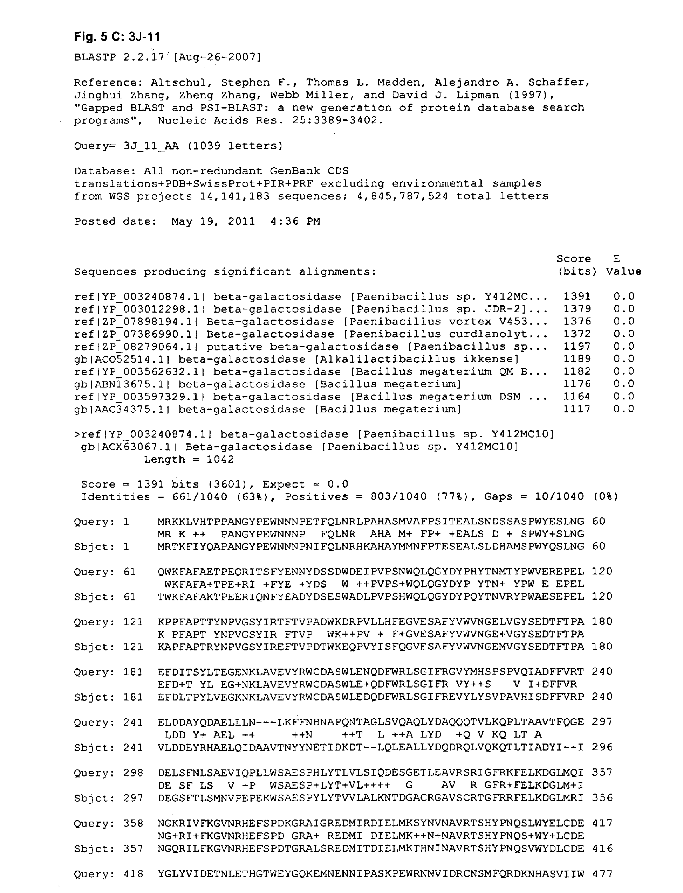

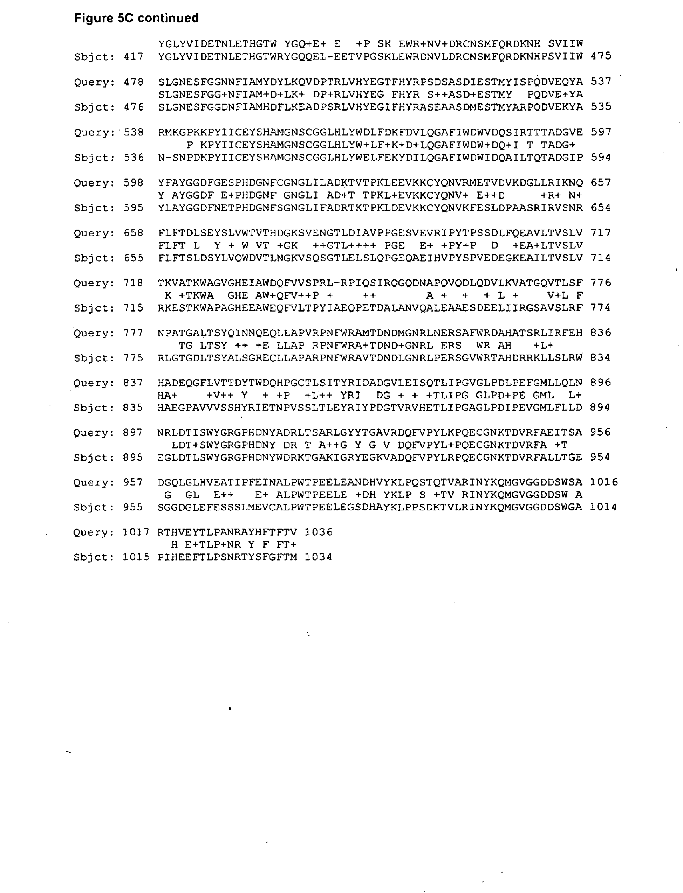

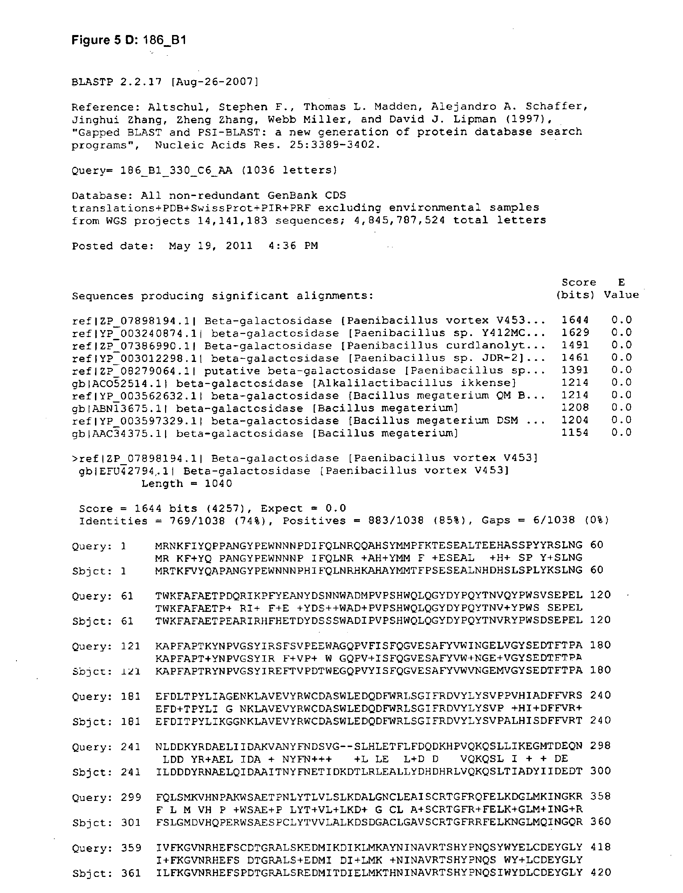

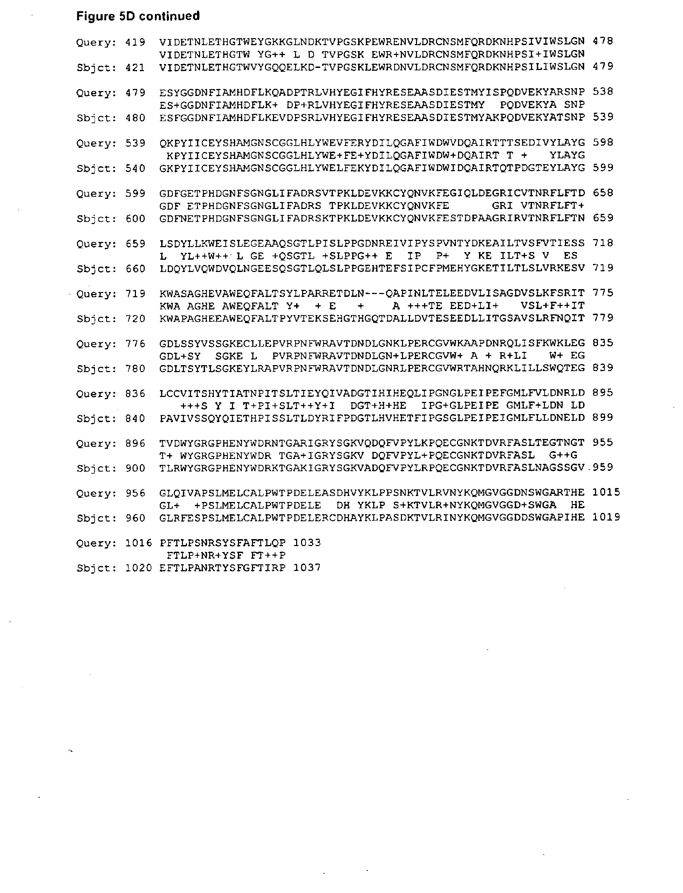

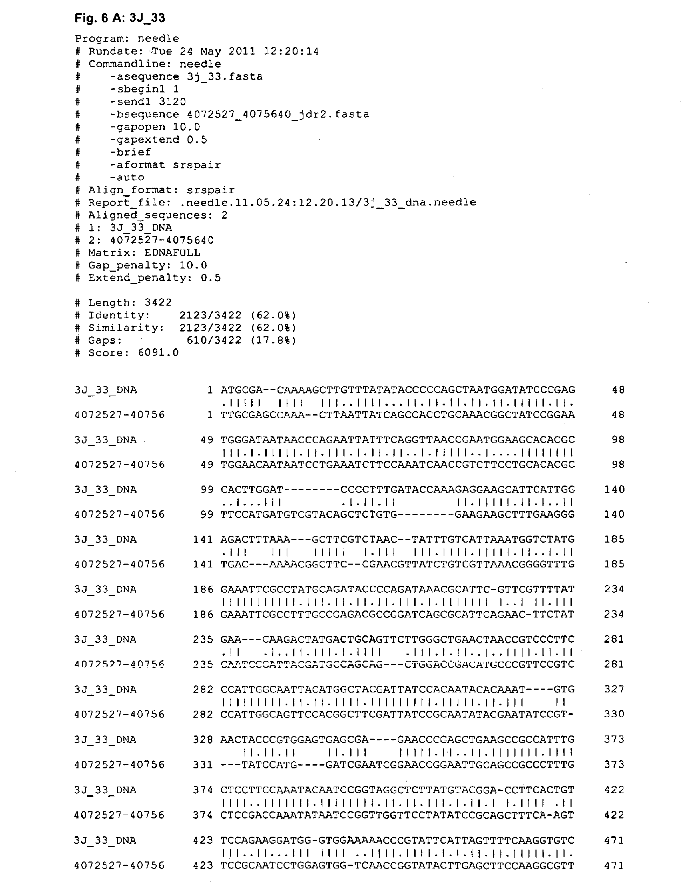

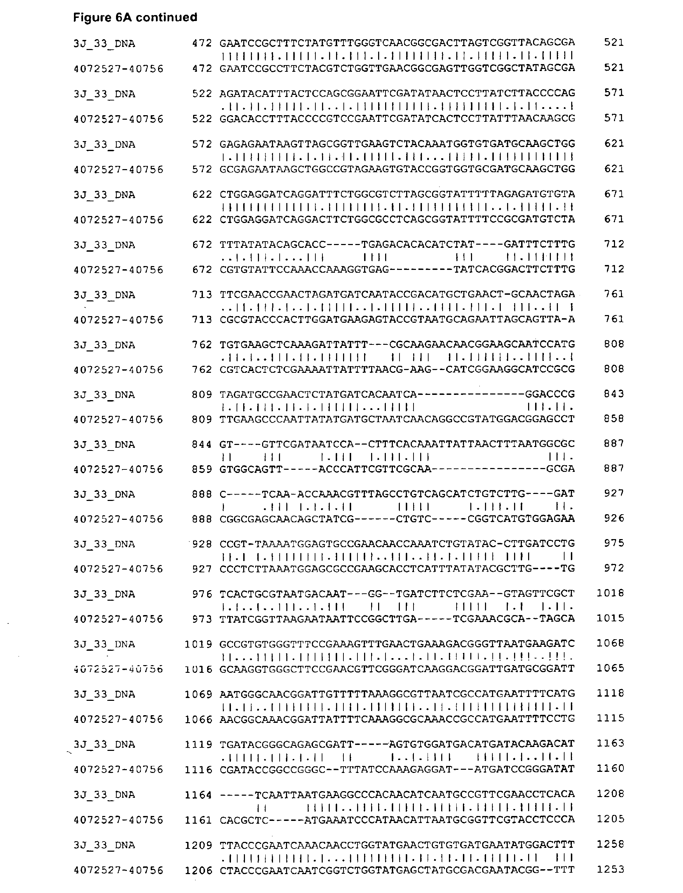

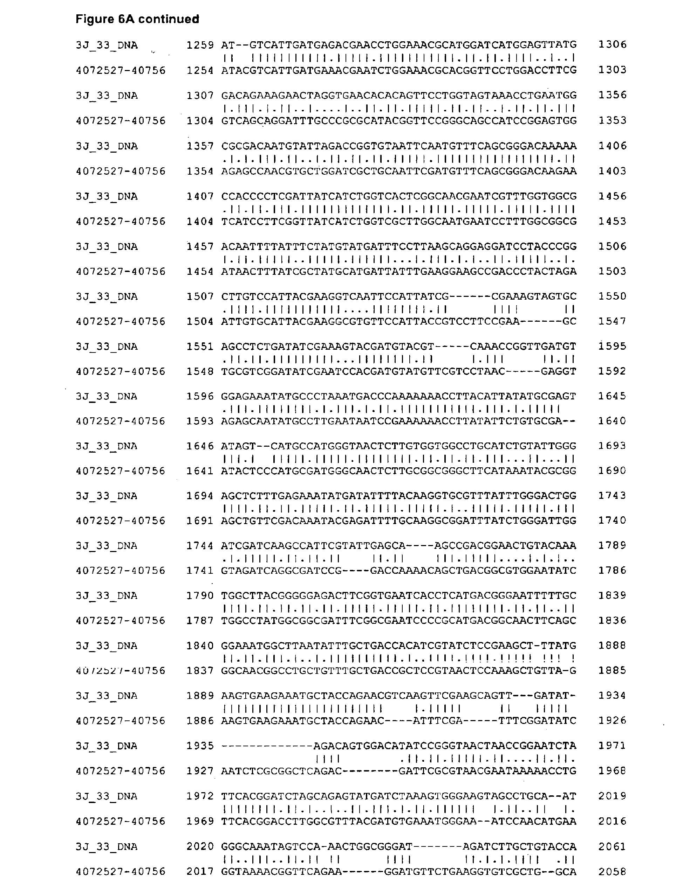

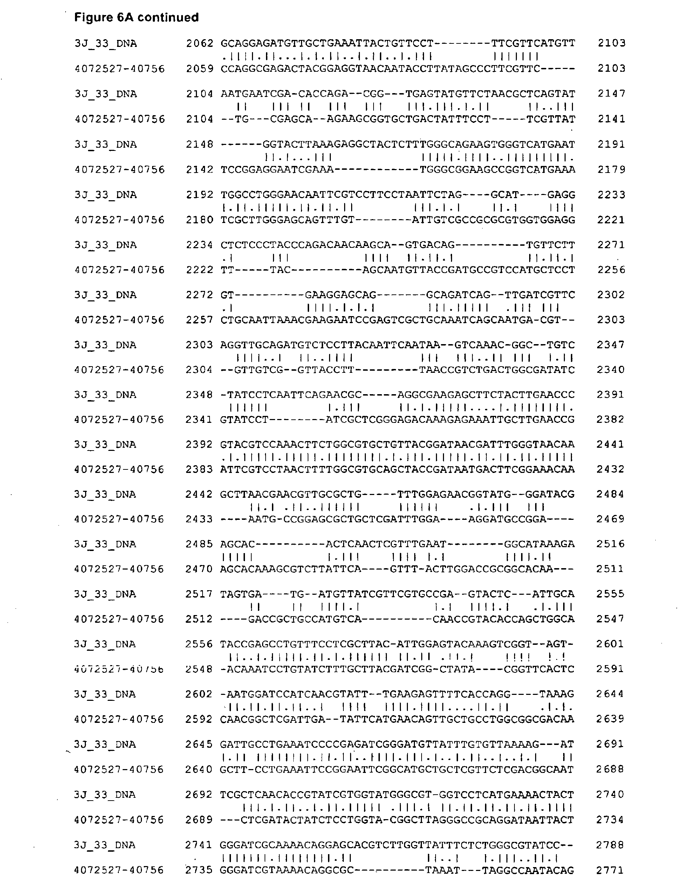

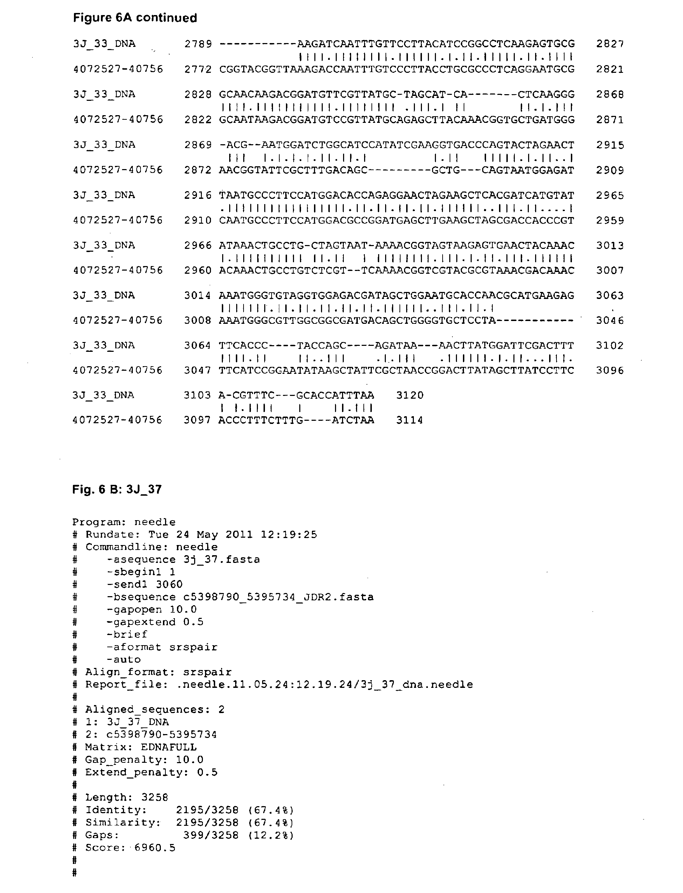

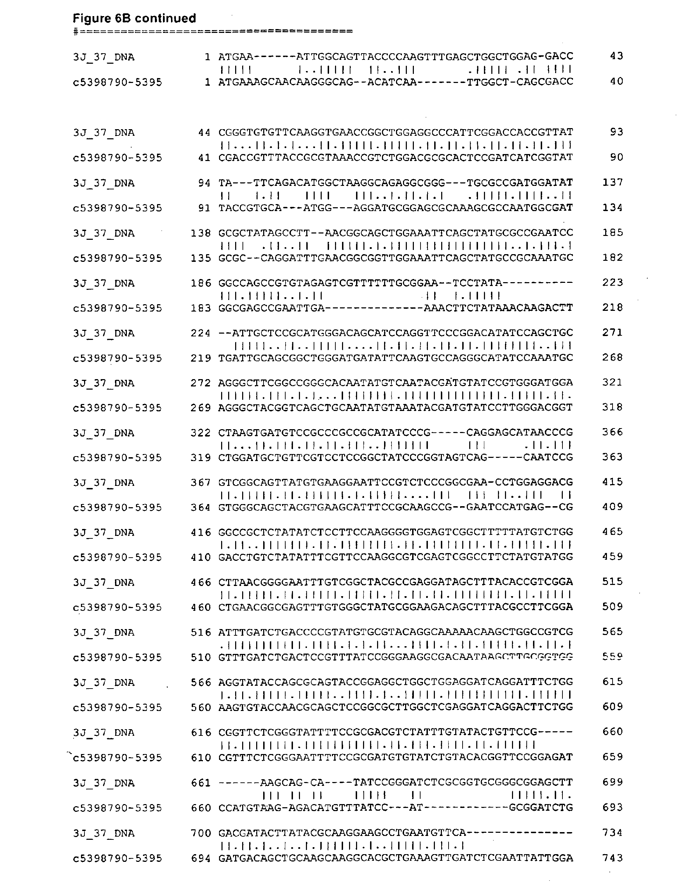

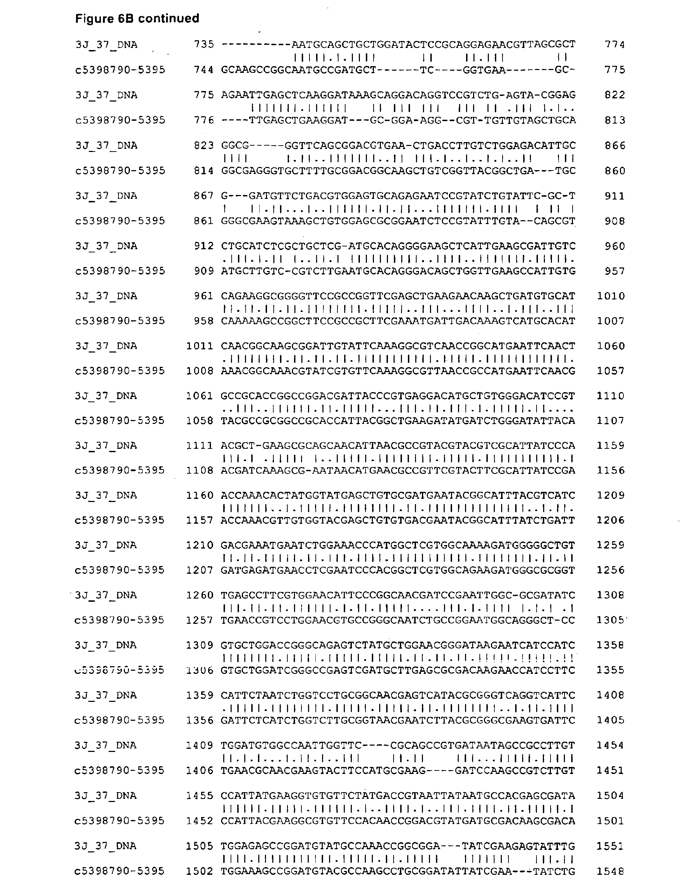

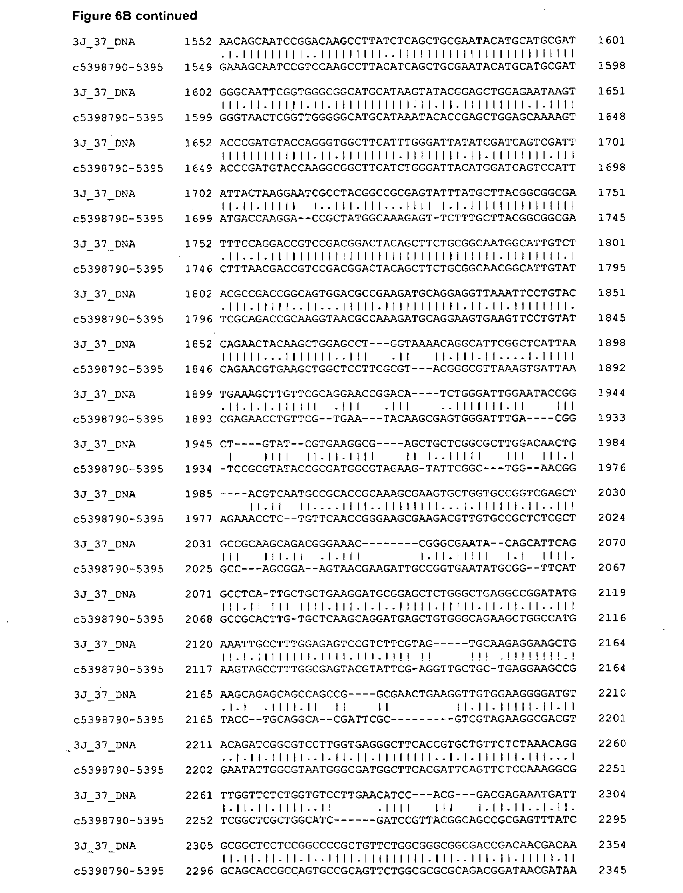

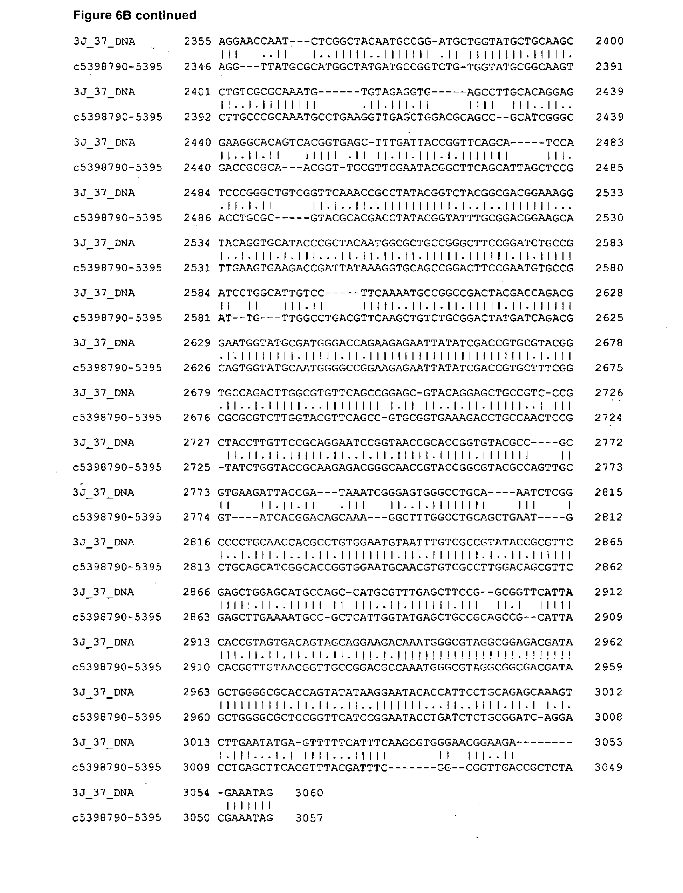

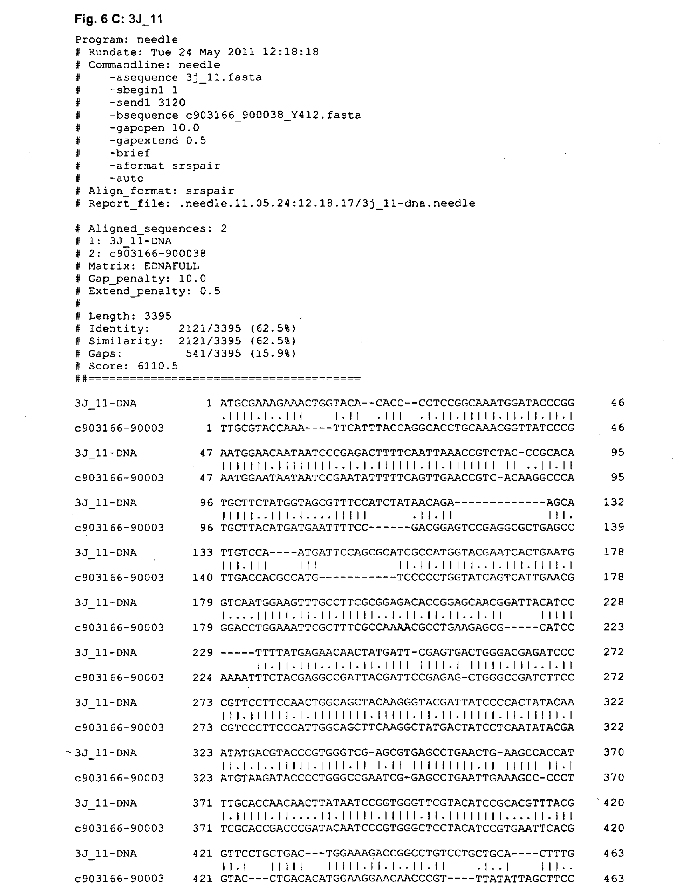

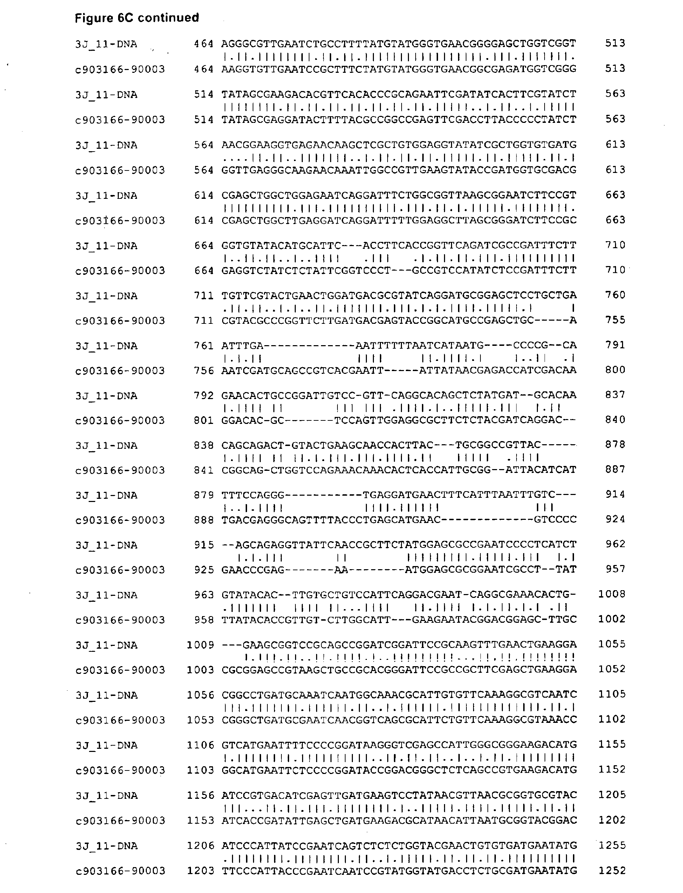

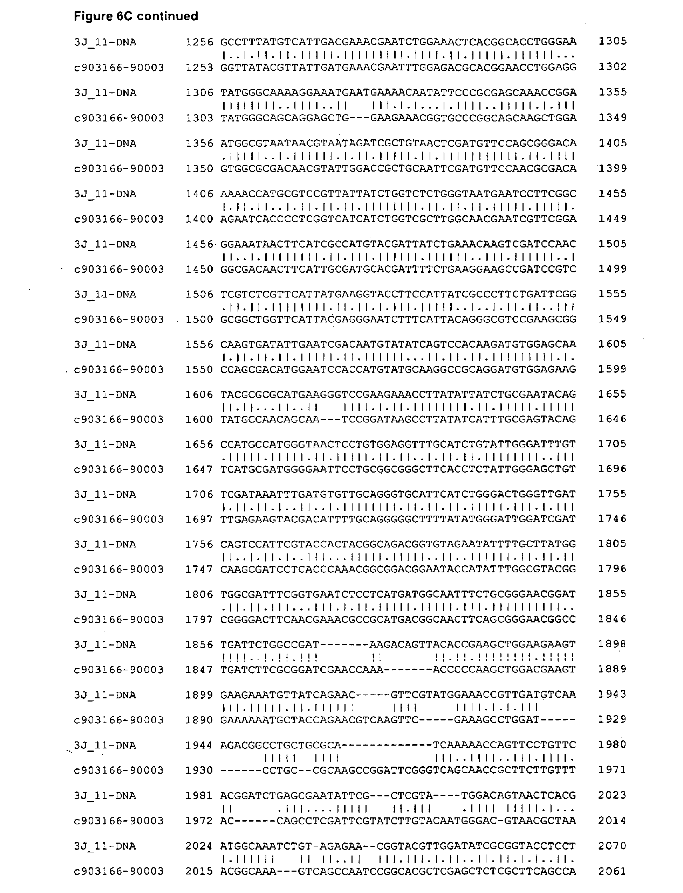

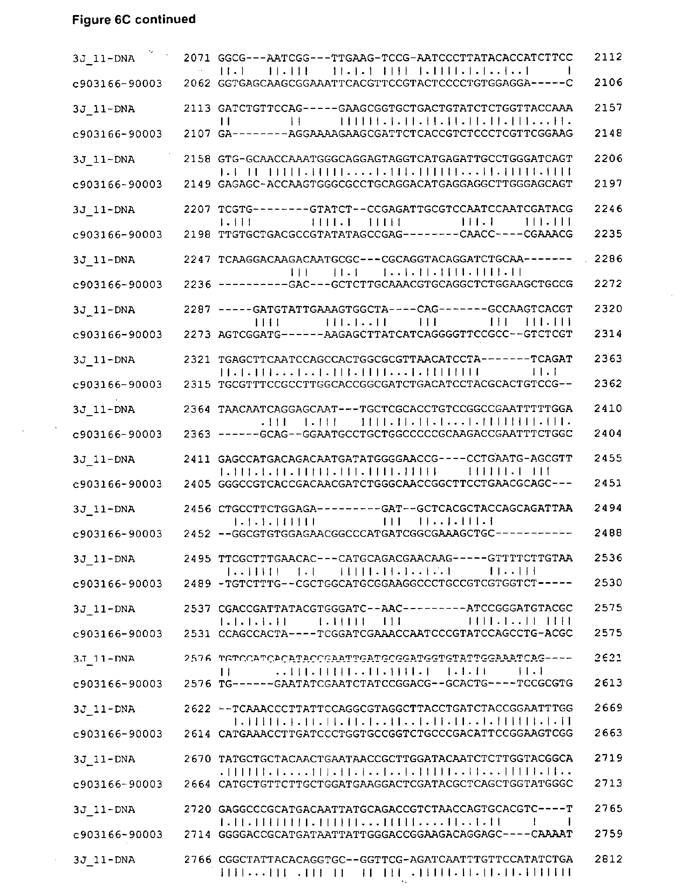

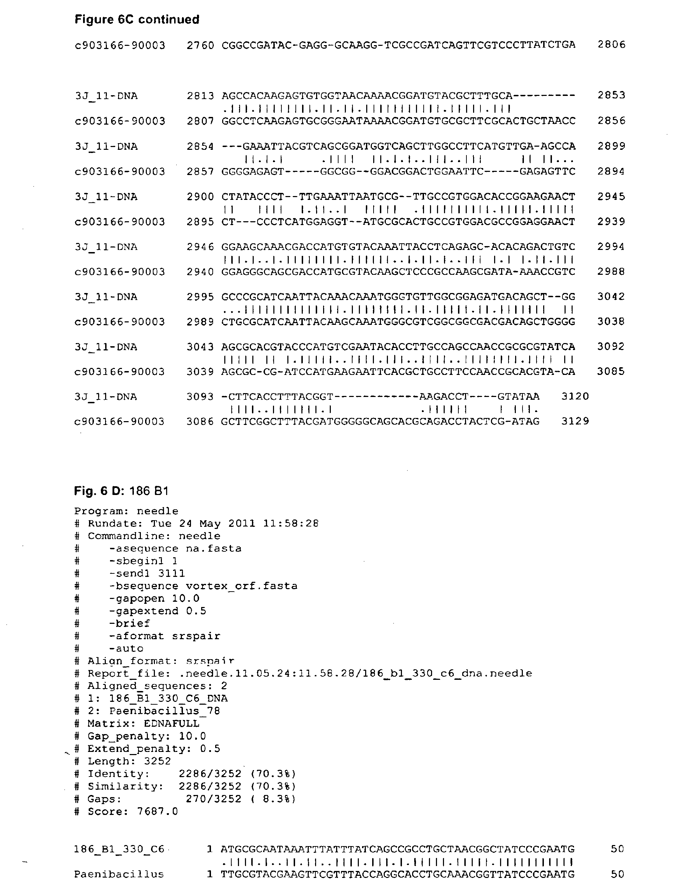

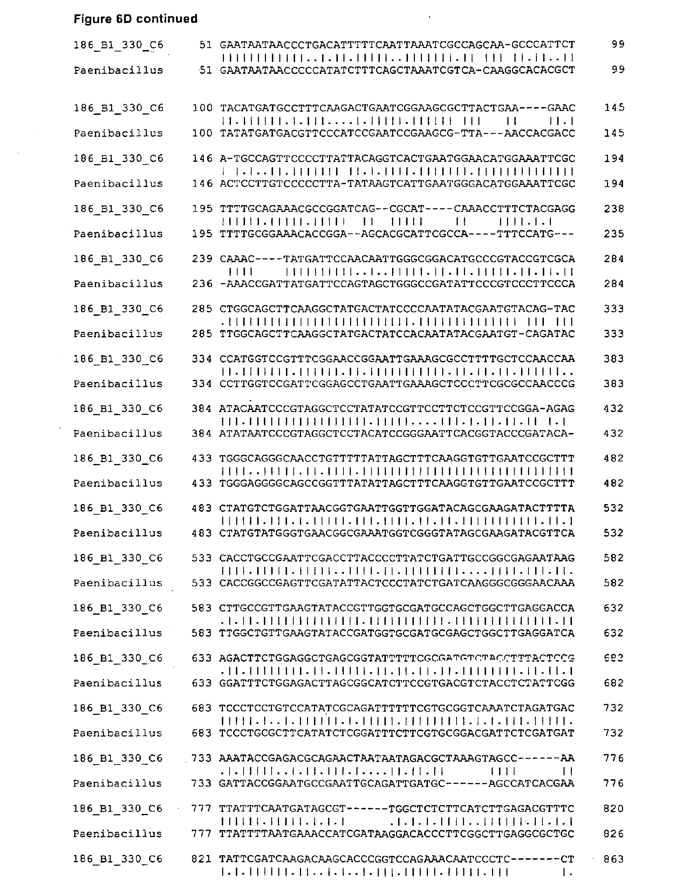

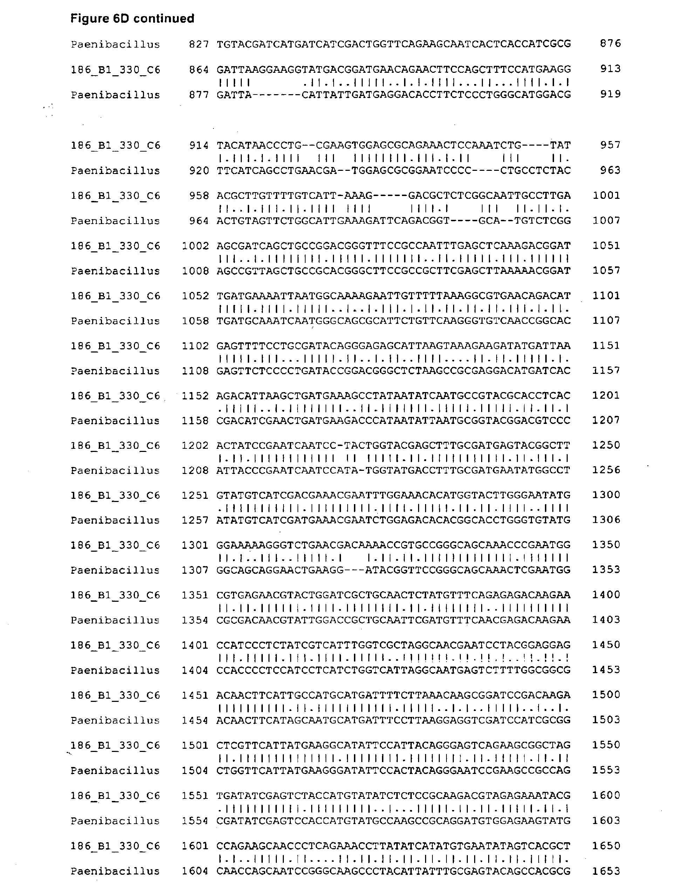

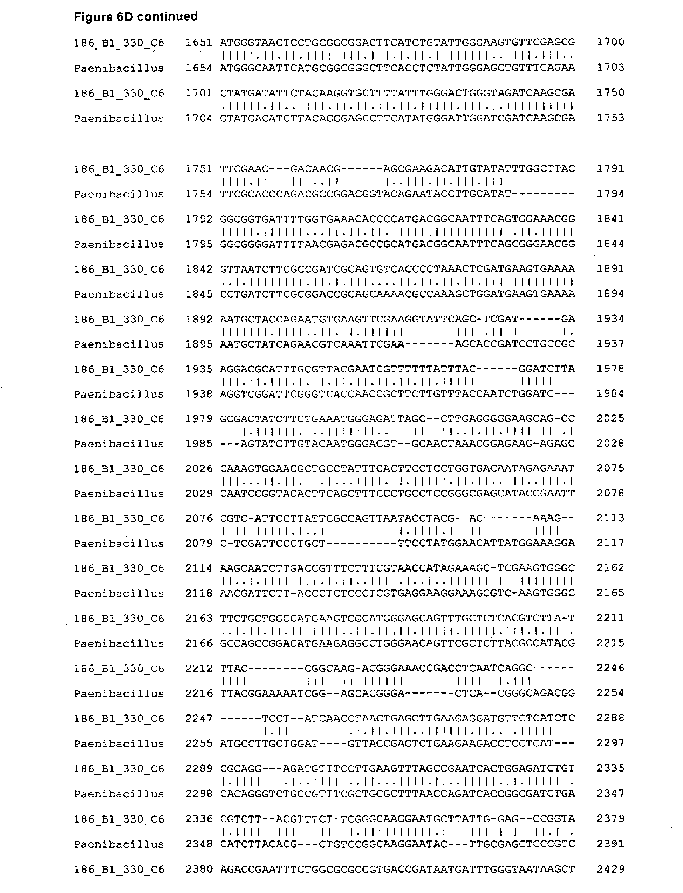

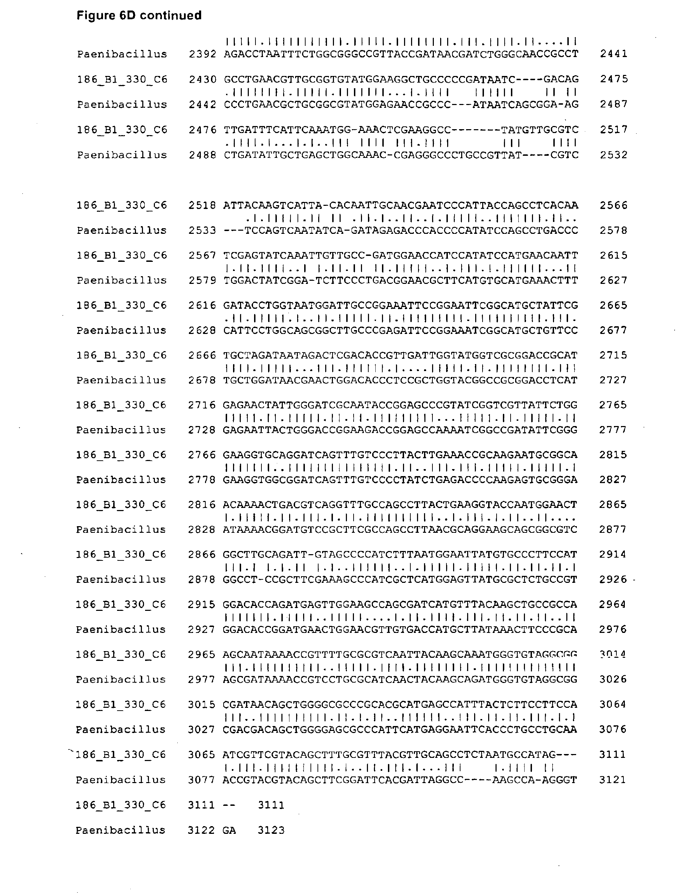

In accordance with the present invention, the term "percent (%) sequence identity" describes the number of matches ("hits") of identical nucleotides/amino acids of two or more aligned nucleic acid or amino acid sequences as compared to the number of nucleotides or amino acid residues making up the overall length of the template nucleic acid or amino acid sequences. In other terms, using an alignment, for two or more sequences or subsequences the percentage of amino acid residues or nucleotides that are the same (e.g. 95% identity) may be determined, when the (sub)sequences are compared and aligned for maximum correspondence over a window of comparison, or over a designated region as measured using a sequence comparison algorithm as known in the art, or when manually aligned and visually inspected. This definition also applies to the complement of any sequence to be aligned. Amino acid sequence analysis and alignment in connection with the present invention was carried out using the NCBI BLAST algorithm (Stephen F. Altschul, Thomas L. Madden, Alejandro A. Schäffer, Jinghui Zhang, Zheng Zhang, Webb Miller, and David J. Lipman (1997), "Gapped BLAST and PSI-BLAST: a new generation of protein database search programs", Nucleic Acids Res. 25:3389-3402) which is preferably employed in accordance with this invention. Preferably published standard parameters are used; see, e.g., Figure 5. The skilled person is aware of additional suitable programs to align nucleic acid sequences. A preferred program for nucleic acid sequence alignment in accordance with the invention is the needle program; preferably, the Needleman-Wunsch algorithm is used. Guidance for preferred parameters may be found in Figure 6.

As defined in the embodiments herein above, certain amino acid sequence identities are envisaged by the invention. Also envisaged are with increasing preference amino acid sequence identities of at least 75%, at least 80%, at least 85%, at least 90%, at least 95%, at least 97,5%, at least 98%, at least 98.5%, at least 99%, at least 99,5%, at least 99,8 %, and 100% identity by the invention. Also described herein is an amino acid sequence identity of at least 68% with regard to SEQ ID NO: 3, at least 63% with regard to SEQ ID NO: 5, and at least 71% with regard to SEQ ID NO: 7. Further described amino acid sequence identities are the sequence identities among SEQ ID NOs. 1, 3, 5 and 7 as shown in the table in Example 3 herein below.

With regard to the amino acid sequences of the invention consisting of or comprising SEQ ID NO: 2, it is particularly preferred that the amino acid sequences of the invention do not comprise the amino acid "M" at the N-terminus as shown in the sequence listing.

As defined in the embodiments herein above, certain nucleotide sequence identities are envisaged by the invention. Also envisaged are with increasing preference nucleotide sequence identities of at least 75%, at least 80%, at least 85%, at least 90%, at least 95%, at least 97,5%, at least 98%, at least 98.5%, at least 99%, at least 99,5%, at least 99,8 %, and 100% identity by the invention. Also described herein is a nucleotide sequence identity of at least 67% with regard to SEQ ID NO: 4, at least 63% with regard to SEQ ID NO: 6, and at least 74% with regard to SEQ ID NO: 8. Further described nucleotide sequence identities are the sequence identities among SEQ ID NOs. 2, 4, 6 and 8 as shown in the table in Example 3 herein below.

With regard to the nucleotide sequences of the invention consisting of or comprising SEQ ID NO: 2 it is particularly preferred that the nucleotide sequences of the invention does not comprise the start codon "ATG" as shown in the sequence listing.

Fragments according to the present invention are polypeptides having the activity of a beta-galactosidase as defined herein above and comprise at least 800 amino acids. In this regard, it is preferred with increasing preference that the fragments according the present invention are polypeptides of at least 850, at least 900, at least 950, at least 1000 amino acids, at least 1010 amino acids, at least 1020 amino acids. Fragments of the polypeptide of the invention, which substantially retain beta-galactosidase activity, include N-terminal truncations, C-terminal truncations, amino acid substitutions, internal deletions and addition of amino acids (either internally or at either terminus of the protein). For example, conservative amino acid substitutions are known in the art and may be introduced into the beta-galactosidase of the invention without substantially affecting beta-galactosidase activity.

As it is evident from the data shown in the Examples herein below, the metagenome-derived ß-galactosidases of the invention are much more active at low and moderate temperatures when compared to commercially available enzymes enzymes from K. lactis. Importantly, no product inhibition of activity by the cleavage product galactose (i.e. monosaccharides) could be observed in in vitro assays and under application conditions in milk. Thus, the invention describes the discovery of novel beta-galactosidases, which show low or no inhibition by their reaction products (in particular mononsaccharides glucose and galactose). This property leads to a better degradation of lactose and opens the possibility to prepare lactose-depleted milk with a lower residual lactose concentration as is possible with current state of the art beta-galactosidase products. Moreover the optimum pH range of 6-8 is ideal for lactose hydrolysis in milk and dairy products. Advantageously, the beta-galactosidase according to the invention has a stable enzymatic activity at a pH range from 6 to 8, preferably from 6.5 to 7.5.

Preferably, the beta-galactosidase according to the invention has a stable enzymatic activity in presence of calcium and/or galactose, meaning that the activity of this enzyme is neither inhibited by its reaction product nor by products being present in milk. This property allows to efficiently use this enzyme in milk treatment.

Taken together, the beta-galactosidase according to the invention attains the level of superior practical application by having the following three advantageous properties: (1) sufficient enzymatic activity in the range 5 to 50°C, preferably 8 to 45°C, (2) sufficient enzymatic activity at a pH range from 6 to 8 (3) an enzymatic activity that is not inhibited by reaction products, such as galactose, or other products substantially present in milk, such as calcium. To the best knowledge of the inventor no beta-galactosidase described in the prior art has all these advantageous properties in combination.

According to an advantageous embodiment of the invention, the enzyme can be inactivated at a pasteurisation temperature. This property of the enzyme according to the present invention allows to apply the beta-galactosidase according to the invention and to stop the enzymatic reaction of lactose hydrolysis without any additional step during a current milk treatment.

Therefore, the object of the present invention was to hydrolyse lactose in a broad temperature range by using beta-galactosidases, which could overcome the above-mentioned drawbacks of prior art beta-galactosidases, especially product inhibition, while advantageously avoiding contamination problems during the hydrolysis process and lowering the energy consumption.

In a further embodiment the invention relates to a vector encoding the nucleic acid molecule of the invention.

The term "vector" in accordance with the invention preferably means a plasmid, cosmid, virus, bacteriophage or another vector used e.g. conventionally in genetic engineering which carries the nucleic acid molecule of the invention either encoding the peptide or the fusion protein of the invention. Accordingly, the nucleic acid molecule of the invention may be inserted into several commercially available vectors. Non-limiting examples include prokaryotic plasmid vectors, such as of the pUC-series, pBluescript (Stratagene), the pET-series of expression vectors (Novagen) or pCRTOPO (Invitrogen) and vectors compatible with an expression in mammalian cells like pREP (Invitrogen), pcDNA3 (Invitrogen), pCEP4 (Invitrogen), pMC1neo (Stratagene), pXT1 (Stratagene), pSG5 (Stratagene), EBO-pSV2neo, pBPV-1, pdBPVMMTneo, pRSVgpt, pRSVneo, pSV2-dhfr, pIZD35, pLXIN, pSIR (Clontech), pIRES-EGFP (Clontech), pEAK-10 (Edge Biosystems) pTriEx-Hygro (Novagen) and pCINeo (Promega). Examples for plasmid vectors suitable for *Pichia pastoris* comprise e.g. the plasmids pAO815, pPIC9K and pPIC3.5K (all Intvitrogen).

The nucleic acid molecule of the present invention referred to above may also be inserted into vectors such that a translational fusion with another nucleic acid molecule is generated. To this aim, overlap extension PCR can be applied (e.g. Wurch, T., Lestienne, F., and Pauwels, P.J., A modified overlap extension PCR method to create chimeric genes in the absence of restriction enzymes, Biotechn. Techn. 12, 9, Sept. 1998, 653-657). The products arising therefrom are termed fusion proteins and will be described further below. The other nucleic acid molecules may encode a protein which may e.g. increase the solubility and/or facilitate the purification of the protein encoded by the nucleic acid molecule of the invention. Non-limiting examples include pET32, pET41, pET43. The vectors may also contain an additional expressible nucleic acid coding for one or more chaperones to facilitate correct protein folding. Suitable bacterial expression hosts comprise e. g. strains derived from BL21 (such as BL21(DE3), BL21(DE3)PlysS, BL21(DE3)RIL, BL21(DE3)PRARE) or Rosetta®.

Particularly preferred plasmids which can be used to introduce the nucleic acid encoding the polypeptide of the invention having the activity of a beta-galactosidase into the host cell are: pUC18/19 (Roche Biochemicals), pKK-177-3H (Roche Biochemicals), pBTac2 (Roche Biochemicals), pKK223-3 (Amersham Pharmacia Biotech), pKK-233-3 (Stratagene) and pET (Novagen). Further suitable plasmids are listed in PCT/EP03/07148. Very particular preference is given to an expression system which is based on plasmids belonging to the pET series.

For vector modification techniques, see Sambrook and Russel, 2001. Generally, vectors can contain one or more origins of replication (ori) and inheritance systems for cloning or expression, one or more markers for selection in the host, e.g., antibiotic resistance, and one or more expression cassettes. Suitable origins of replication include, for example, the Col E1, the SV40 viral and the M13 origins of replication.

The coding sequences inserted in the vector can e.g. be synthesized by standard methods, or isolated from natural sources. Ligation of the coding sequences to transcriptional regulatory elements and/or to other amino acid encoding sequences can be carried out using established methods. Transcriptional regulatory elements (parts of an expression cassette) ensuring expression in prokaryotes or eukaryotic cells are well known to those skilled in the art. These elements comprise regulatory sequences ensuring the initiation of the transcription (e. g., translation initiation codon, transcriptional termination sequences, promoters, enhancers, and/or insulators), internal ribosomal entry sites (IRES) (Owens *et al.,* 2001) and optionally poly-A signals ensuring termination of transcription and stabilization of the transcript. Additional regulatory elements may include transcriptional as well as translational enhancers, and/or naturally-associated or heterologous promoter regions. The regulatory elements may be native to the beta-galactosidase of the invention or heterologous regulatory elements. Preferably, the nucleic acid molecule of the invention is operably linked to such expression control sequences allowing expression in prokaryotes or eukaryotic cells. The vector may further comprise nucleotide sequences encoding secretion signals as further regulatory elements. Such sequences are well known to the person skilled in the art. Furthermore, depending on the expression system used, leader sequences capable of directing the expressed polypeptide to a cellular compartment may be added to the coding sequence of the nucleic acid molecule of the invention. Such leader sequences are well known in the art. Specifically designed vectors allow the shuttling of DNA between different hosts, such as bacteria-fungal cells or bacteria-animal cells.

The co-transfection with a selectable marker such as kanamycin or ampicillin resistance genes for culturing in *E*. *coli* and other bacteria allows the identification and isolation of the transfected cells. Selectable markers for mammalian cell culture are the dhfr, gpt, neomycin, hygromycin resistance genes. The transfected nucleic acid can also be amplified to express large amounts of the encoded (poly)peptide. The DHFR (dihydrofolate reductase) marker is useful to develop cell lines that carry several hundred or even several thousand copies of the gene of interest. Another useful selection marker is the enzyme glutamine synthase (GS) (Murphy et al. 1991; Bebbington et al. 1992).

Using such markers, the cells are grown in selective medium and the cells with the highest resistance are selected.

In another embodiment the invention relates to a host cell transformed, transduced or transfected with the nucleic acid molecule or the vector of the invention, and a heterologous expression regulatory element which is operably linked to the nucleic acid molecule of the invention.

A variety of host-expression systems may be conceived to express the beta-galactosidase coding sequence in a host cell using a suitable vector.

The "host cell" in accordance with the invention may be produced by introducing the nucleic acid molecule or vector(s) of the invention into the host cell which upon its/their presence mediates the expression of the nucleic acid molecule of the invention encoding the enzyme of the invention. The host from which the host cell is derived may be any prokaryote or eukaryotic cell.

A suitable eukaryotic host cell may be a vertebrate cell, an amphibian cell, a fish cell, an insect cell, a fungal/yeast cell, a nematode cell or a plant cell. The insect cell may be a *Spodoptera frugiperda* cell, a Drosophila S2 cell or a Spodoptera Sf9 cell, the fungal/yeast cell may a *Saccharomyces cerevisiae* cell, *Pichia pastoris* cell or an *Aspergillus* cell. It is preferred that the vertebrate cell is a mammalian cell such as a human cell, CHO, COS, 293 or Bowes melanoma cell. The plant cell is preferably selected independently from a cell of *Anacardium, Anona, Arachis, Artocarpus, Asparagus, Atropa, Avena, Brassica, Carica, Citrus, Citrullus, Capsicum, Carthamus, Cocos, Coffea, Cucumis, Cucurbita, Daucus, Elaeis, Fragaria, Glycine, Gossypium, Helianthus, Heterocallis, Hordeum, Hyoseyamus, Lactuca, Linum, Lolium, Lupinus, Lycopersicon, Malus, Manihot, Majorana, Medicago, Nicotiana, Olea, Oryza, Panieum, Pannesetum, Passiflora, Persea, Phaseolus, Pistachia, Pisum, Pyrus, Prunus, Psidium, Raphanus, Ricinus, Secale, Senecio, Sinapis, Solanum, Sorghum, Theobromus, Trigonella, Triticum, Vicia, Vitis, Vigna* and *Zea*. The cell may be a part of a cell line. The cell from plant may, e.g., be derived from root, leave, bark, needle, bole or caulis.

Suitable prokaryotes (bacteria) useful as hosts for the invention are those generally used for cloning and/or expression like *E*. *coli* (e.g., *E coli* strains BL21, HB101, DH5a, XL1 Blue, Y1090 and JM101), *Salmonella typhimurium, Serratia marcescens, Burkholderia glumae, Pseudomonas putida, Pseudomonas fluorescens, Pseudomonas stutzeri, Streptomyces lividans, Lactococcus lactis, Mycobacterium smegmatis, Streptomyces* or *Bacillus subtilis.* Appropriate culture mediums and conditions for the above-described host cells are known in the art.

Preferred examples for host cell to be genetically engineered with the nucleic acid molecule or the vector(s) of the invention is a cell of yeast, *E. coli* and/or a species of the genus *Bacillus* (e.g., *B. subtilis*). The most preferred host cell is *Bacillus spec..*

In a further embodiment the invention relates to a method of producing a protein having the activity of a beta-galactosidase (EC 3.2.1.23) comprising (a) culturing the host cell of the invention and (b) isolating the produced protein having the activity of a beta-galactosidase.

Suitable conditions for culturing a prokaryotic or eukaryotic host are well known to the person skilled in the art. Suitable conditions for culturing *E*. *coli* DH18BΔkat E (Invitrogen), *Pichia pastoris* or *Aspergillus niger* are, for example provided in the examples of the invention. In general, suitable conditions for culturing bacteria are growing them under aeration in Luria Bertani (LB) medium. To increase the yield and the solubility of the expression product, the medium can be buffered or supplemented with suitable additives known to enhance or facilitate both. *E*. *coli* can be cultured from 4 to about 37 °C, the exact temperature or sequence of temperatures depends on the molecule to be overexpressed. In general, *Aspergillus sp.* may be grown on Sabouraud dextrose agar, or potato dextrose agar at about to 10°C to about 40°C, and preferably at about 25°C. Suitable conditions for yeast cultures are known, for example from Guthrie and Fink, "Guide to Yeast Genetics and Molecular Cell Biology" (2002); Academic Pr Inc.. The skilled person is also aware of all these conditions and may further adapt these conditions to the needs of a particular host species and the requirements of the polypeptide expressed. In case an inducible promoter controls the nucleic acid of the invention in the vector present in the host cell, expression of the polypeptide can be induced by addition of an appropriate inducing agent. Suitable expression protocols and strategies are known to the skilled person.

Depending on the cell type and its specific requirements, mammalian cell culture can e.g. be carried out in RPMI or DMEM medium containing 10% (v/v) FCS, 2mM L-glutamine and 100 U/ml penicillin/streptomycin. The cells can be kept at 37 °C in a 5% CO₂, water saturated atmosphere.

Suitable expression protocols for eukaryotic cells are well known to the skilled person and can be retrieved e.g. from in Sambrook, 2001.

Methods of isolation of the polypeptide produced are well-known in the art and comprise without limitation method steps such as ion exchange chromatography, gel filtration chromatography (size exclusion chromatography), affinity chromatography, high pressure liquid chromatography (HPLC), reversed phase HPLC, disc gel electrophoresis or immunoprecipitation, see, for example, in Sambrook, 2001.

The step of protein isolation is preferably a step of protein purification. Protein purification in accordance with the invention specifies a process or a series of processes intended to further isolate the polypeptide of the invention from a complex mixture preferably to homogeneity. Purification steps, for example, exploit differences in protein size, physico-chemical properties and binding affinity. For example, proteins may be purified according to their isoelectric points by running them through a pH graded gel or an ion exchange column. Further, proteins may be separated according to their size or molecular weight via size exclusion chromatography or by SDS-PAGE (sodium dodecyl sulfate-polyacrylamide gel electrophoresis) analysis. In the art, proteins are often purified by using 2D-PAGE and are then further analysed by peptide mass fingerprinting to establish the protein identity. This is very useful for scientific purposes and the detection limits for protein are very low and nanogram amounts of protein are sufficient for their analysis. Proteins may also be separated by polarity/hydrophobicity via high performance liquid chromatography or reversed-phase chromatography. Thus, methods for protein purification are well known to the skilled person and are exemplified in the examples of the invention.

Furthermore, the invention relates in one embodiment to a protein having the activity of a beta-galactosidase (EC 3.2.1.23) encoded by the nucleic acid molecule of the invention or produced by the method of the invention.

In a preferred embodiment, the polypeptide of the invention is a fusion protein.

Exemplary fusion proteins of the polypeptide of the invention may assist in expression and/or purification of the protein. Accordingly, it is preferred that the fusion protein comprises a signal peptide and/or secretion peptide.

In addition to the amino acid sequence of the polypeptide of the present invention (which has the activity of a beta-galactosidase), a fusion protein according to the present invention contains at least one additional, heterologous amino acid sequence. Often, but not necessarily, these additional sequences will be located at the N- or C-terminal end of the polypeptide. It may e.g. be convenient to initially express the polypeptide as a fusion protein from which the additional amino acid residues can be removed, e.g. by a proteinase (e.g. thrombin, factor VIII, factor Xa protease, or PreScission Protease) capable of specifically trimming the polypeptide of the present invention.

The term "fusion protein" in accordance with the invention refers to an amino acid sequence genetically grafted onto a recombinant protein, in the present case the protein having beta-galactosidase activity. Such way a heterologous fusion protein of the invention is preferably experimentally generated and not normally produced (expressed) by a cell. These peptides, preferably "tags" may be removable by chemical agents or by enzymatic means, such as proteolysis or intein splicing. Tags are attached to proteins for various purposes. Affinity tags are appended to proteins so that they can be purified from their crude biological source using an affinity technique. These include but are not limited to chitin binding protein (CBP), maltose binding protein (MBP), glutathione-S-transferase (GST), and poly(His) tag. The poly(His) tag is a widely-used protein tag; it binds to metal matrices. Solubilization tags are used, especially for recombinant proteins expressed in chaperone-deficient species such as *E. coli,* to assist in the proper folding in proteins and keep them from precipitating. These include but are not limited to thioredoxin (TRX) and poly(NANP). Some affinity tags have a dual role as a solubilization agent, such as MBP, and GST. Chromatography tags are used to alter chromatographic properties of the protein to afford different resolution across a particular separation technique. Chromatography tags comprise but are not limited to of polyanionic amino acids, such as FLAG-tag. Epitope tags are short peptide sequences which are chosen because high-affinity antibodies can be reliably produced in many different species. These are usually derived from viral genes, which explain their high immunoreactivity. Epitope tags include but are not limited to V5-tag, c-myc-tag, and HA-tag. These tags are particularly useful for western blotting and immunoprecipitation experiments, although they also find use in antibody purification. Fluorescence tags are used to give visual readout on a protein. For example, GFP and its variants are the most commonly used fluorescence tags. More advanced applications of GFP include using it as a folding reporter (fluorescent if folded, colorless if not). Moreover, tags find many other usages, such as specific enzymatic modification (such as biotin ligase tags) and chemical modification (FIAsH) tag. Examples of suitable tags to be used in accordance with the invention comprise but are not limited to lacZ, GST, maltose-binding protein, NusA, BCCP, c-myc, CaM, His, FLAG, GFP, YFP, cherry, thioredoxin, poly(NANP), V5, Snap, HA, chitin-binding protein, Softag 1, Softag 3, Strep, or S-protein.

In another embodiment the present invention relates to a composition comprising the nucleic acid, the vector, the host cell, and/or the protein of the invention, or combinations thereof.

According to a preferred embodiment the composition is a food composition, a cosmetic composition, or a pharmaceutical composition.

The term "food composition" as used herein describes a solid or liquid formulation which can therefore be eaten or drunk by a human subject for nutrition. It s preferred that the food composition of the invention comprises milk or whey.

In addition to the above recited ingredients further ingredients of the food composition of the invention may be selected from lipids, minerals, carbohydrates, amino acids, amino acid chelates, anabolic nutrients, vitamins, antioxidants, probiotic bacterial strain and lipotropic agents in order to provide an optimal sustained energy and anabolic food composition. The food composition may be a nutritional supplement or may provide complete nutrition. Preferably the food composition is in the form of a liquid.

A non-therapeutic composition which may be a preferred example of a food composition are pills or capsules comprising the nucleic acid, the vector, the host cell, and/or the protein of the invention, or combinations thereof. These pills or capsules can be used as digestive supplement, in particular by individual having a lactose intolerance. In practical terms, the contents of the pills or capsules can be mixed, for example, with milk products, which still contain lactose, to thereby enable symptom free consumption of these products through in situ lactose hydrolysis. Examples of such pills are "Digestive Advantage", "Lactase Enzyme", "Say Yes to Dairy", "Life like Lactase", "Biolabor Laktase", "Lactrase" or the like.

In accordance with the present invention, the term "pharmaceutical composition" relates to a composition for administration to a patient, preferably a human patient. The pharmaceutical composition is preferably used in a disease associated with the loss of function or reduced function of a beta-galactosidase. A non-limiting example of a disease associated with the loss of function or reduced function of beta-galactosidase is galactosidase intolerance as described herein below.

A cosmetic composition according to the invention is for use in non-therapeutic applications. In particular, the may be used in the formulation of cosmetic composition which enhance the cell longevity (Moreau et al, Journal of Drugs in Dermatology, Articles in June, 2007 issue).

Cosmetic compositions may also be defined by their intended use, as compositions intended to be rubbed, poured, sprinkled, or sprayed on, or otherwise applied to the human body for cleansing, beautifying, promoting attractiveness, or altering the appearance.

The particular formulation of the cosmetic composition according to the invention is not limited. Envisaged formulations include rinse solutions, emulsions, creams, milks, gels such as hydrogels, ointments, suspensions, dispersions, powders, solid sticks, foams, sprays and shampoos. For this purpose, the cosmetic composition according to the invention may further comprise cosmetically acceptable diluents and/or carriers. Choosing appropriate carriers and diluents in dependency of the desired formulation is within the skills of the skilled person. Suitable cosmetically acceptable diluents and carriers are well known in the art and include agents referred to in Bushell et al. (WO 2006/053613). Preferred formulations for said cosmetic composition are rinse solutions and creams.

According to a more preferred embodiment, the composition of the invention comprises whey, milk and/or a milk product.

In accordance with an even more preferred embodiment, the milk product of the invention is yoghurt, curd, cheese, ice cream, cream, whipped cream, or "dulce de leche".

(Sour and sweet) whey and milk are the main natural resources of β-galactoside, and in particular lactose.

According to a further embodiment the pharmaceutical composition of the invention is for use in treating or preventing the symptoms of a lactose intolerance.

The term "lactose intolerance" as used herein specifies the inability to metabolize lactose, because of a lack of the required enzyme lactase in the digestive system. It is estimated that 75% of adults worldwide show some decrease in lactase activity during adulthood (Durand, P. (1959). "Lactosurie et saccharosurie". In Ed. E. Rossi e al.. Paediat. IV. Carbohydrate Metabolism in Children. Basel. pp.496-502). The frequency of decreased lactase activity ranges from as little as 5% in northern Europe, up to 71% for Sicily, to more than 90% in some African and Asian countries (Holzel et al. (1959). "Defective lactose absorption causing malnutrition in infancy". Lancet 1 (7083): 1126-8).

Also encompassed by the present invention is the use of the protein of the invention for the hydrolysis of a beta-galactoside, beta-glucoside and/or the synthesis of beta-galacto-oligosaccharides.

In accordance with an even more preferred embodiment, the beta-galactoside is beta-D-galactose or lactose.

In a further embodiment the invention relates to a process for reducing the lactose concentration in milk or a milk product, comprising contacting the host cell of the invention, the protein of the invention, or combinations thereof with milk or a milk product.

As described herein above, may individuals suffer from lactose intolerance. Therefore, beta-galactosides are important in food industry to produce food compositions which comprise lactose-free or at least lactose-depleted milk or a milk products.

In a further embodiment the invention relates to a process for the production of beta-galacto-oligosaccharides, comprising contacting the host cell of the invention, the protein of the invention, or combinations thereof with beta-D-galactose and/or lactose.

As described herein above, beta-galacto-oligosaccharides consist mainly of beta(1-6) linked galactopyranosil units linked to a terminal glucopyranosyl residue via an alpha(1-4) glycosidic bond. In general, oligosaccharides have a chain length of between two to ten monosaccharides. While beta-galacto-oligosaccharides are only found in trace amounts in cow's milk, their concentration is relatively high in human milk. Human milk contains between 5 to 12 g beta-galacto-oligosacharides per liter. Beta-galacto-oligosacharides are used as prebiotic compounds in food preparation. Beta-galacto-oligosacharides are a very important ingredient of artificial infant milk. The addition to these products allows, for example to design a composition, which is close to human milk. It is therefore of interest to have an efficient production route for beta-galacto-oligosacharides, which is done by applying beta-galactosidases under the appropriate conditions.

An overview of the industrial importance and applicability of beta-galactosidases, in particular in food industry is provided in MLlCHOVÁ and ROSENBERG, Journal of Food and Nutrition Research Vol. 45, 2006, No. 2, pp. 47-54. In more detail, the enzymatic hydrolysis of lactose proposes several benefits and advantages of industrial application, including the development of lactose hydrolysed products to present one of the possible approaches to diminish the lactose intolerance problem, prevalent in more than half of the world's population, formation of galacto-oligosaccharides during lactose hydrolysis to favour the growth of intestinal bacterial microflora, improvement in the technological and sensorial characteristics of foods containing hydrolysed lactose, and better biodegradability of whey after lactose hydrolysis .

Furthermore, encompassed by the present invention are uses and methods of the β-galactosidase of the invnetion in assay, which are, for example, frequently used in genetics, molecular biology (see X-gal) for a blue white screen, and other life sciences (http://openwetware.org/wiki/Beta-Galactosidase_Assay_(A_better_Miller). In more detail, IPTG induces production of β-galactosidase by binding and inhibiting the lac repressor.

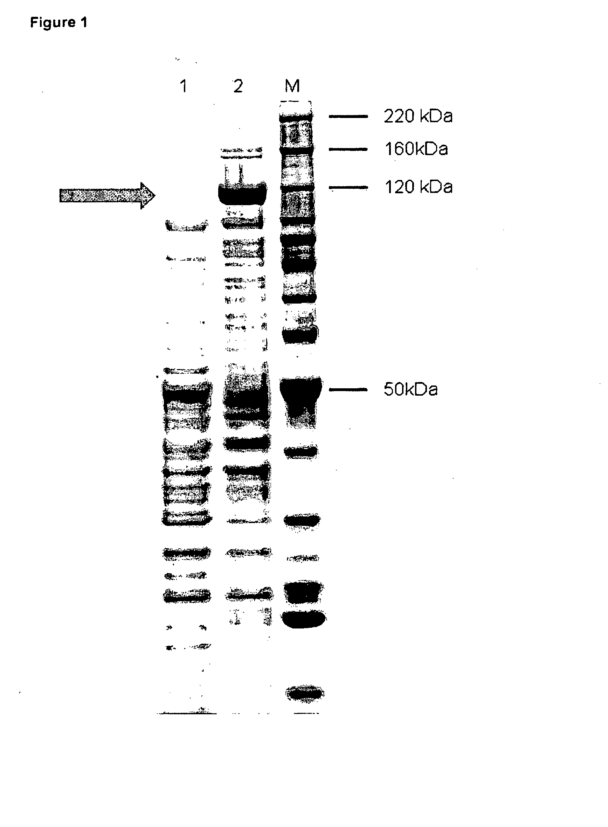

The Figures show
Fig. 1: Expression of the metagenome beta-galactosidase 3J_33 in Escherichia coli. Lane 1: *E*. *coli* with empty vector (negative control), lane 2: *E*. *coli* carrying beta-galactosidase 3J_33, lane M: molecular weight standard
Fig. 2: Enzymatic properties of beta-galactosidases
   A: Enzyme paramters as determined in *in vitro* assays; B: Ratios of relative enzymatic activities (EA) with regard to temperature and absence/presence of galactose
Fig. 3: pH-profile of the metagenome-beta-galactosidase 3J_33 in potassium phosphate buffer at 30°C

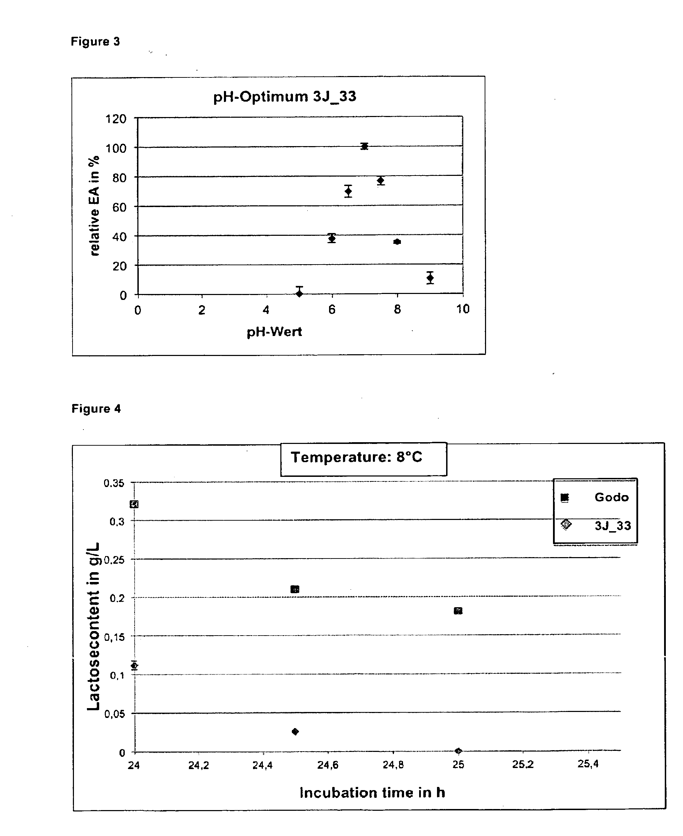

Fig. 4: Comparison of lactose hydrolysis of crude extract of 3J_33 and commercial preparations of Godo-YNL^{®}. Hydrolysis was carried out in pasteurised milk with a lactose concentration of 45 g/l. Reaction temperature was adjusted to 8°C and 40 nkat_{(ortho-nitrophenyl-p-galactopyranoside,25°C,pH7)}/ml of the different enzyme preparations was added in a total reaction volume of 30ml. Lactose concentration was measured after 24 h at 8°C. At the end of the reaction temperature was increased up to 40°C and remaining lactose concentration was determined 30min and 60min after reaching this temperature.
Fig. 5: Sequence Alignments
   Next neighbour analysis (SEQ ID NO: 9) and alignment of the amino acid sequences of 3J_33 (SEQ ID NO: 1) (A), 3J_37 (SEQ ID NO: 3) (B); 3J_11 (SEQ ID NO: 5) (C) and 186_B1 (SEQ ID NO: 7) (D)
Fig. 6: Sequence Alignments
   Next neighbour alignment (SEQ ID NO: 10) of the nucleotide sequences of 3J_33 (SEQ ID NO: 2) (A), 3J_37 (SEQ ID NO: 4) (B); 3J_11(SEQ ID NO: 6) (C) and 186_B1 (SEQ ID NO: 8) (D).

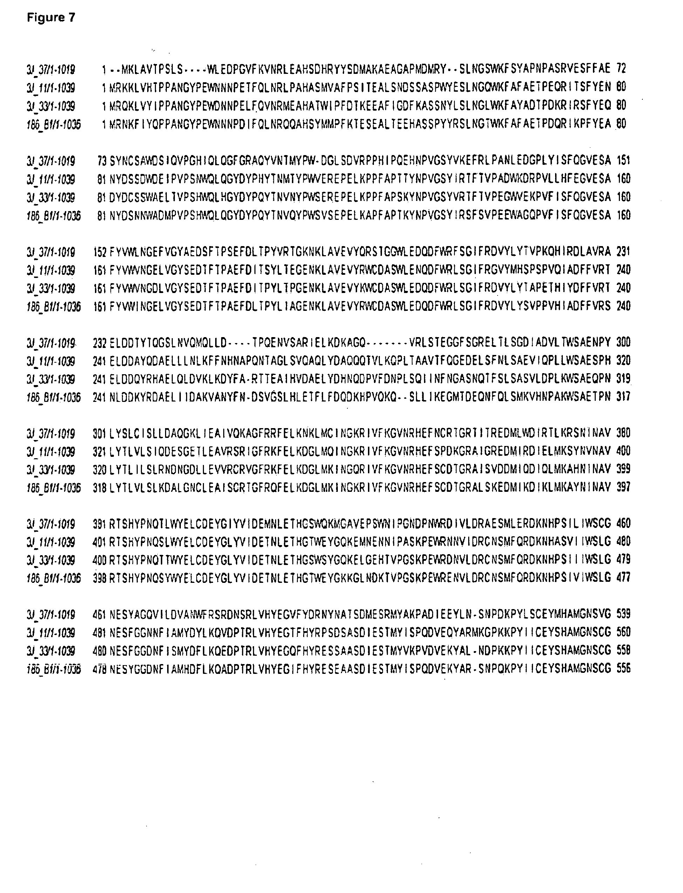

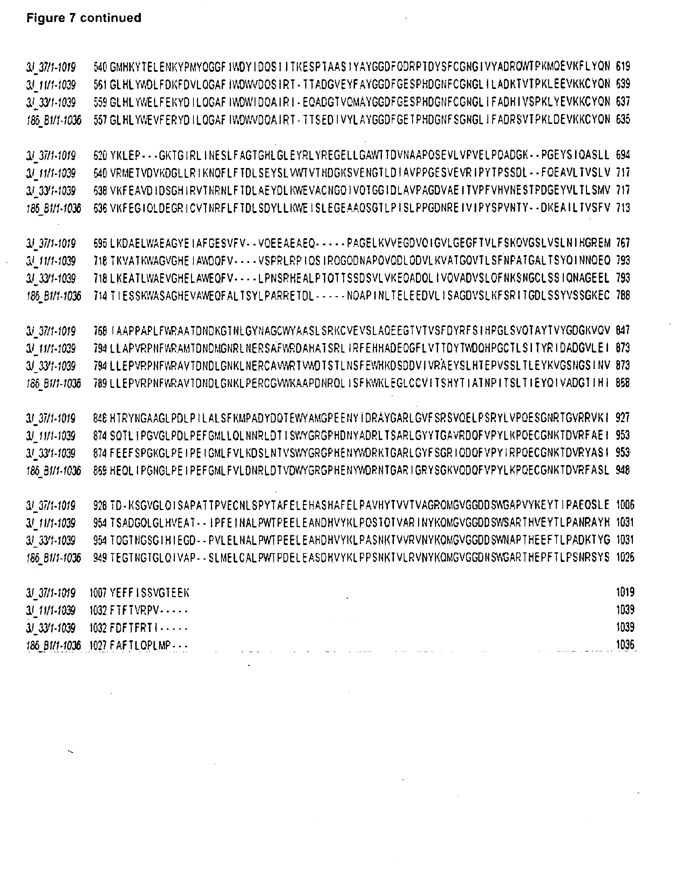

Fig. 7: Sequence Alignment
   Multiple sequence alignment of the amino acid sequences of 3J_33 (SEQ ID NO: 1) (A), 3J_37 (SEQ ID NO: 3) (B); 3J_11 (SEQ ID NO: 5) (C) and 186_B1 (SEQ ID NO: 7) (D).

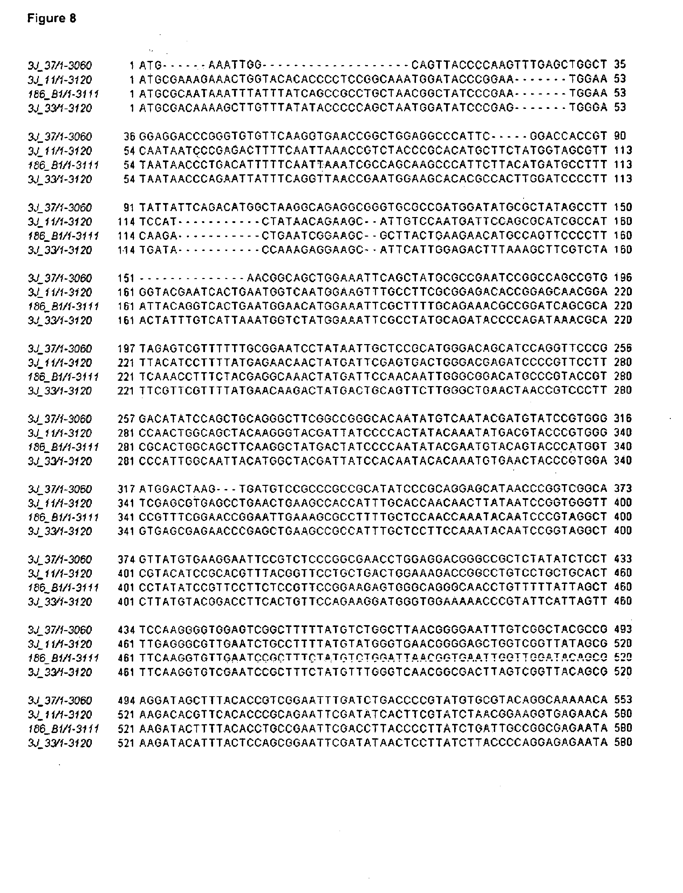

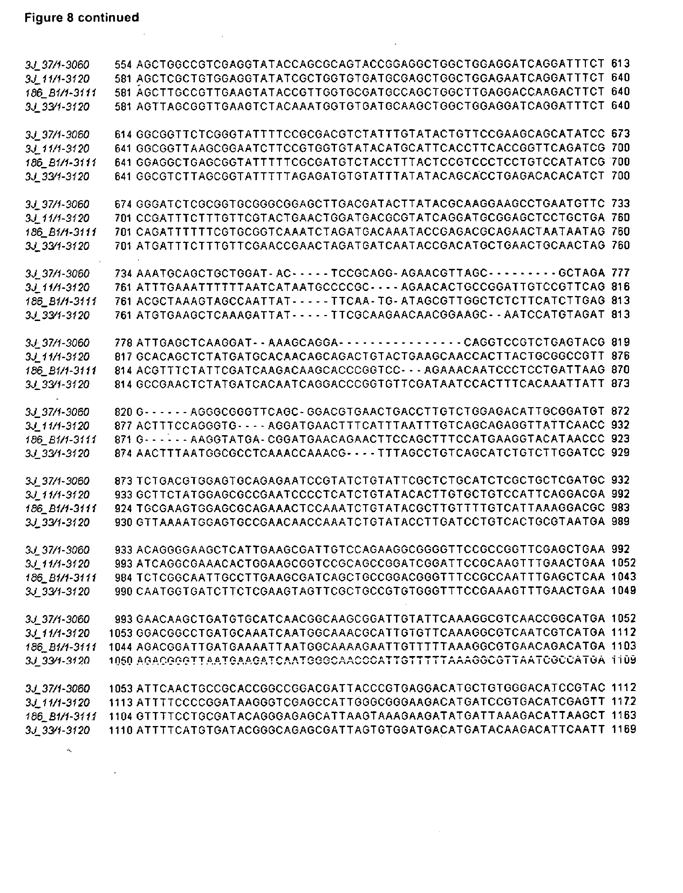

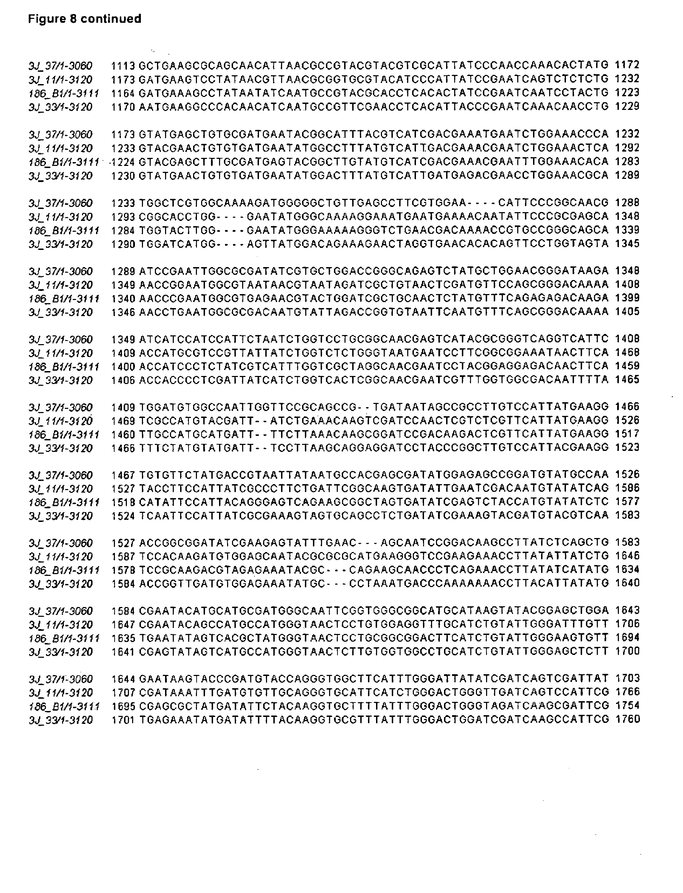

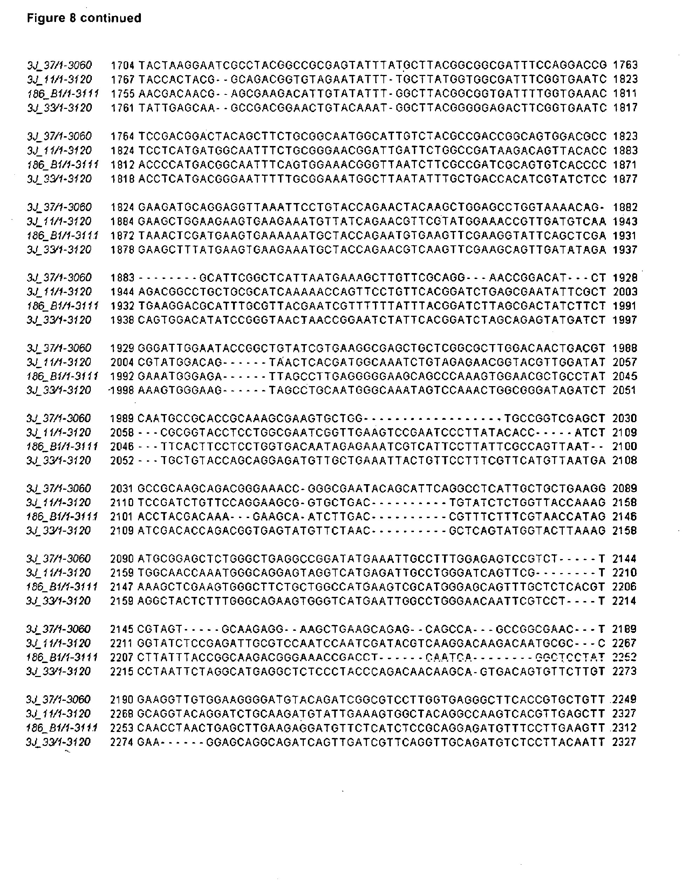

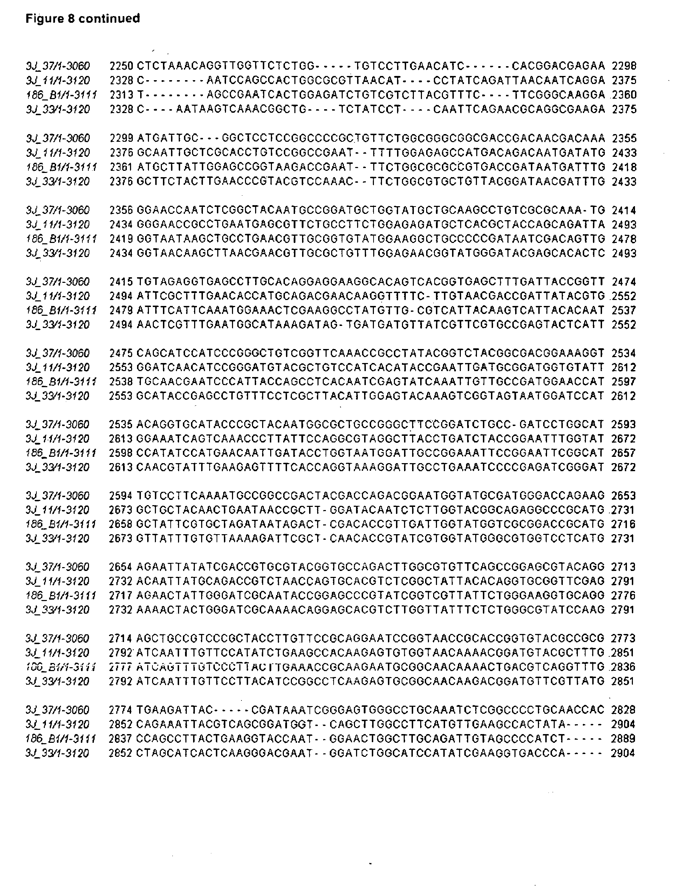

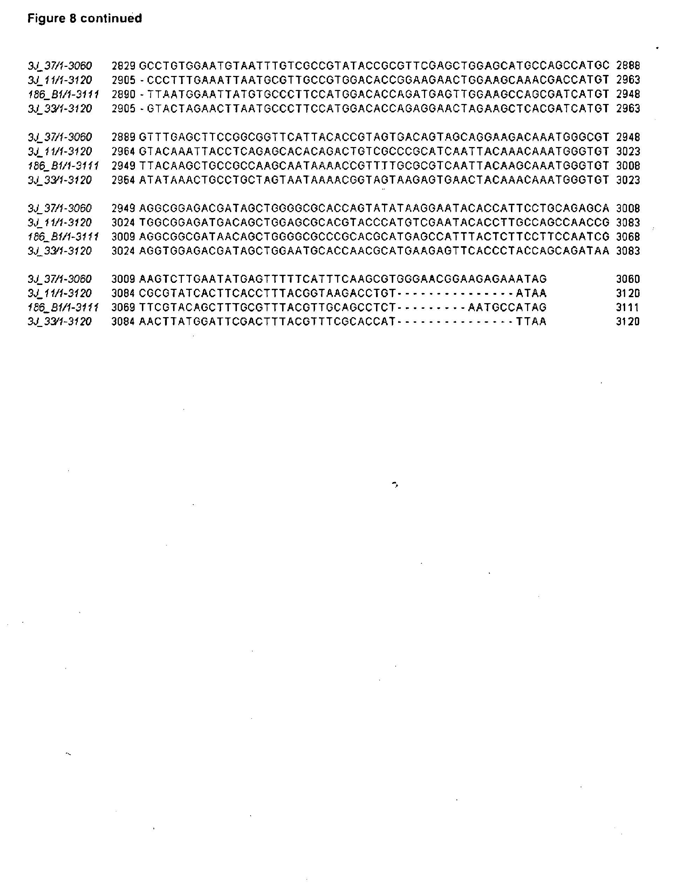

Fig. 8: Sequence Alignment
   Multiple sequence alignment of the nucleotide sequences of 3J_33 (SEQ ID NO: 2) (A), 3J_37 (SEQ ID NO: 4) (B); 3J_11(SEQ ID NO: 6) (C) and 186_B1 (SEQ ID NO: 8) (D).

The Examples illustrate the invention.

Beta-galactosidases isolated from K. lactis were used as control enzymes: Maxilact^{®} (DSM Food Specialties, The Netherlands) and Godo-YNL^{®} (Godo Shusei Co. Ltd., Japan).

### Example 1

### Screening of metagenome library with X-Gal as surrogate substrate: Identification of lactose degrading clones

The identification of beta-galactosidase harboring metagenome clones was carried out using X-Gal (5-bromo-4-chloro-3-indolyl-beta-D-galactopyranoside) as a substrate in an agar plate assay. Hereby plasmid metagenome libraries were plated and incubated on standard Luria-Bertani media at 37°C over night using Chloramphenicol (12.5µg/ml medium) as a selective agent until colonies with 1 mm in diameter were visible. Activity screen was carried out at 12°C until blue colonies were visible. The occurance of the blue colour indicates beta-galactosidase activity. Positive candidates were recultured in liquid media. Briefly, cultivation was done in 50 ml standard A5 medium supplemented with 10g/l Lactose and cultures were agitated at 30°C for 48h at 200rpm. Plasmids were isolated according to standard procedures. After retransformation, verified X-Gal degrading clones were cultivated for long-term storage at-80°C in microplates with 96 cavities (copies of master- and working cultures).

### Example 2

### Liquid screening assay for beta-Galactosidase activity

Two main parameters were targeted in the modification of the manufacturing process for lactose-free milk regarding the application of lactose degrading enzymes. In the first place it was considered desirable for the enzyme to be active at storage temperatures of fresh unprocessed milk. Therefore a liquid screening assay for the quantification of enzymatic activity at a) 8°C and b) 30°C was established.

Of capital importance was the susceptibility of the enzyme for product inhibition. To quantify this effect, enzymatic activity on lactose in a) the presence and b) absence of galactose was measured. The ratio of b) to a) was consulted as the degree of product inhibition. Ratios close to 1 indicate negligible inhibitory effects for galactose being the hydrolysis product.

### a) Cultivation of galactosidase-positive recombinant E. coli cultures and preparation of enzymatically active cell crude extract

Frozen precultures were inoculated in deep well plates with 96 cavities with LB medium containing 12.5µg/ml chloramphenicol as selection agent in quadruplicate cultures. Cultivation was carried out for 40h at 30°C and shaking at 250 rpm on a microplate shaker. All four cultures per clone were pooled and centrifuged at 720g for 30min at 4°C and culture supernatant was removed. Cells were resuspended in 1ml 100mM potassium-phosphate buffer (pH 6.7) and disintegrated using a silica sand mill device (Fast Prep, Bio101, MP Biomedicals LLC) and 0.1 mm zirconia/silica beads (BioSpec Products Inc., Bartlesville, USA) for 30sec. Cell debris was pelleted by centrifugation (720g, 30min, 4°C) and the cell free enzmatically active cell extracts were immediately used for subsequent enzymatic testing (s. Example 4).

### b) Liquid assay on lactose using crude extract of beta-galactosidase positive clones (temperature assay)

Temperature assay was carried out in 96 well MTP devices at 8°C and 30°C performing the assays in four replicates. The assay consisted of 160µl 100mM Potassium-Phosphate buffer pH 6.7 and 40µl of crude extract (see section a). The lactose concentration in this liquid assay was adjusted to 200mM. Lactose hydrolysis was carried out at 8°C and 30°C for 1h and the reaction was stopped by adding 95µl of the enzymatic reaction mixture to 190µl 1 M perchloric acid on ice. After centrifugation (740g, 10min), samples were neutralised by addition of 160µl of the centrifuged supernatant into 61µl 2M KOH on ice. After a final centrifugation step, released glucose was quantified in the supernatant.

### c) Determination of glucose release using chromogenic ABTS assay (temperature assay)

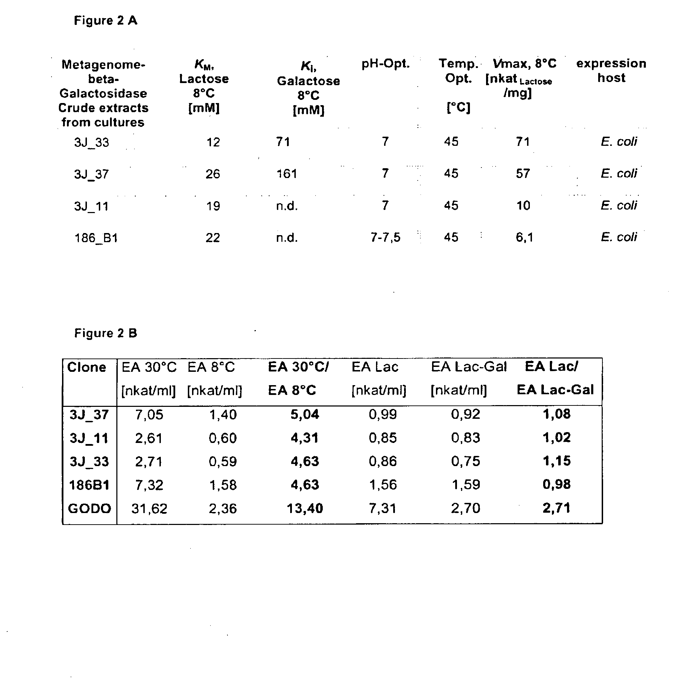

The amount of lactose hydrolysis at different temperatures was measured in an indirect manner by detection of released glucose molecules. In this assay, glucose is first converted enzymatically to gluconolactone by glucose oxidase. During this reaction hydrogen peroxide is formed in equimolar amounts as the second reaction product. In a coupled reaction using Horseradish peroxidase the final chromogenic substrate 2,2'-azino-bis(3-ethylbenzthiazoline-6-sulphonic acid) is reduced to a radical cation with an absorption peak at 420nm. Calibration was performed using increasing concentrations of a glucose solution. For results see Fig. 2B.

### d) Liquid assay on lactose using crude extract of beta-galactosidase positive clones (assay for product inhibition)

The product inhibition assay was carried out in microplates with 96 cavities in quadruplicates. The assay consisted of two different reactions containing 40µl of crude extract (see section a). In a first measurement, lactose hydrolysis was carried out adding 160µl 100mM Potassium-Phosphate buffer pH 6.7 containing 20mM lactose. In a second approach, in addition 150mM galactose was supplemented representing the potential product inhibitor. Lactose hydrolysis was carried out at 30°C for 1h and the reaction was stopped by adding 95µl of the enzymatic reaction mixture to 190µl 1 M perchloric acid on ice. After centrifugation (740g, 10min), samples were neutralised by addition of 160µl of the centrifuged supernatant into 61µl 2M KOH on ice. After a final centrifugation step, released glucose was quantified in the supernatant.

### e) Determination of glucose release using Hexokinase/Glucose-6-P-Dehydrogenase assay (assay for product inhibition)

Since glucose oxidase does also convert galactose, a less sensitive, but galactose-independent assay was used for the quantitative determination of product inhibition of beta-galactosidases. Hereby glucose is converted to glucose-6-phosphate by hexokinase using ATP as cosubstrate. In a coupled reaction glucose-6-phosphate is oxidised to gluconat-6-phosphate by G-6-P dehydrogenase. Related reduction of NADP⁺ to NADPH + H⁺ was quantified photometrically at 340nm. Calibration was performed using glucose solutions with increasing concentrations.

The results of these assays are summarized in Fig. 2B. These results clearly indicate advantageous properties of the described beta-galactosidases of the invention according to the temperature range of activity as well as the absence of product inhibition by galactose and any components of milk.

To conclude, the metagenome-derived beta-galactosidases show favourable enzymatic characteristics especially as compared to the commercial product Godo-YNL^{®}.

### Example 3

### Sequencing of recombinant insert fragments of isolated plasmids derived from priorised lactose hydrolysing clones and identification of corresponding genes

Plasmids of the identified clones were isolated using commercial plasmid isolation kits and sequenced according to standard Sanger sequencing procedures. In all cases open reading fragments related to the observed enzymatic activity could be identified. Protein primary structures and the respective full-length nucleotide sequences and corresponding are shown in SEQ ID NOs 1 to 8. Next neighbours on amino acid level were identified by BLAST analysis using the "Basic Local Alignment Search Tool" (BLAST, http://blast.ncbi.nlm.nih.gov). For parameters of BLAST analysis s. Fig. 5. Alignment of the nucleic acid sequences of the beta-galactosidases of the invention with the next neighbours (SEQ ID NOs 9 and 10) as identified by BLAST analysis was done using the "Needle" program (Saul B. Needleman, Christian D. Wunsch: A general method applicable to the search for similarities in the amino acid sequence of two proteins. In: Journal of Molecular Biology. 48, 1970, S. 443-453). Figures 6A-D show the respective nucleic acid alignments.

The highest identities of the metagenome beta-galactosidase sequences of the invention in a pairwise alignment with the next neigbour in the NCBI database are shown in figurers 5A-D (amino acid) and Figures 6A-D (nucleic acid), respectively.

Identity values are summarized in the following tables:

| SEQ ID amino acid / nucleic acid | Designation | Next neigbour | Identity (%) amino acid / nucleic acid |
|---|---|---|---|
| NO 1 / NO 2 | 3J_33 | Paenibacillus spec., beta-galactosidase | 63.0 / 62.0 |
| NO 3 / NO 4 | 3J_37 | Paenibacillus spec., beta-galactosidase | 67.0 / 67.4 |
| NO 5/ NO 6 | 3J_11 | Paenibacillus spec., beta-galactosidase | 63.0 / 62.5 |
| NO 7 / NO 8 | 186_B1 | Paenibacillus vortex, beta-galactosidase | 74.0 / 70.3 |

Identity (identity level x 100 = % identity) amino acid

| **Seq->** | **3J_37** | **3J_11** | **3J_33** | **186_B1** |
|---|---|---|---|---|
| **3J_37** | - | 0,410 | 0,431 | 0,419 |
| **3J_11** | | - | 0,629 | 0,626 |
| **3J_33** | | | - | 0,656 |
| 186_B1 | | | | - |

Identity (identity level x 100 = % identity) nucleotide

| **Seq->** | **3J_37** | **3J_11** | **186_B1** | **3J_33** |
|---|---|---|---|---|
| **3J_37** | - | 0,498 | 0,495 | 0,516 |
| **3J_11** | | - | 0,625 | 0,628 |
| **186_B1** | | | - | 0,648 |
| **3J_33** | | | | - |

### Example 4

### Determination of temperature- and pH-profiles and measrurement of enzyme parameters

The assay for beta-galactosidase was carried out with enzmatically active cell extracts using 3mM ONPG (ortho-nitrophenyl-p-galactopyranoside) as a chromogenic substrate in 100 mM potassium phosphate buffer containing 1 mM MgCl₂. To determine the pH-profile, the assay was carried out in a pH range from 5 to 9 at 30°C. The temperature profile was determined using the described assay at a constant pH of 7 with temperatures ranging from 8°C to 60°C. Activities towards the chromogenic substrate were recorded in a spectrophotometer (Spectramax, MDS Analytical Technologies Molecular Devices, Ismaning, Germany) and calculated on the basis of an extinction coefficient for o-nitrophenol of 3.5 mM⁻¹cm⁻¹ at 410 nm (Miller, J. H. and Reznikoff, W. S., Eds. 1978; The Operon. Cold Spring Harbor Laboratory Press, NY.). Enzyme kinetic parameters were measured according to state of the art methods to determine Kₘ, Kᵢ, Vₘₐₓ, pH- and temperature-range (for reference see e.g. Andrés Illanes,: Enzyme biocatalysis: principles and applications. Springer, Dordrecht 2008).

Results are shown in Fig. 2A. The beta-galactosidase 3J_33 showed activity from 8 to 50°C (optimum at 45°C) in a pH Range from 5.5 to 9. Optimal activity was measured at pH 7 (s. Fig. 3).

### Example 5

### Comparison of lactose hydrolysis of crude extract of 3J_33 and commercial preparations of Godo-YNL^{®}.

Hydrolysis was carried out in pasteurised milk with a lactose concentration of 45 g/I. Reaction temperature was adjusted to 8°C and 40 nkat_{(ortho-nitrophenyl-p-galactopyranoside,25°C,pH7)}/ml of the respective enzyme preparations were added in a total reaction volume of 30ml. Lactose concentration was measured after 24 h at 8°C. At the end of the 24 hours incubation period, temperature was increased up to 40°C and remaining lactose concentration was determined 30min and 60min after reaching this temperature. It was shown, that under these assay conditions, the beta-galactosidase 3J_33, in contrast to Godo-YNL^{®}, completely degraded residual lactose, implicating the absence of product inhibition for 3J_33 (s. Fig. 4).

To conclude, the beta-galactosidases of the invention, especially 3J_33, are much more active at low and moderate temperatures when compared to a commercially available enzyme from *K. lactis* (Godo-YNL^{®}). Moreover the optimum pH range of 6-8 is ideal for lactose hydrolysis in milk and dairy products

## Claims

1. A nucleic acid molecule encoding
(I) a polypeptide having the activity of a beta-galactosidase (EC 3.2.1.23) which beta-galactosidase is **characterized in that** it has (i) sufficient enzymatic activity in the range 5 to 50°C to catalyze the hydrolysis of ß-galactosidases into monosaccharides, (ii) sufficient enzymatic activity at a pH range from 6 to 8 to catalyze the hydrolysis of ß-galactosidases into monosaccharides, and (iii) an enzymatic activity that is not inhibited by reaction products such as galactose, or other products substantially present in milk, such as calcium, which beta-galactosidase is encoded by
(a) a nucleic acid molecule encoding a polypeptide comprising or consisting of the amino acid sequence of SEQ ID NO: 1;
(b) a nucleic acid molecule comprising or consisting of the nucleotide sequence of SEQ ID NO: 2;
(c) a nucleic acid molecule encoding a beta-galactosidase the amino acid sequence of which is at least 70% identical to the amino acid sequence of SEQ ID NO: 1;
(d) a nucleic acid molecule comprising or consisting of a nucleotide sequence which is at least 70% identical to the nucleotide sequence of SEQ ID NO: 2; or
(e) a nucleic acid molecule corresponding to the nucleic acid molecule of any one of (a) to (d) wherein T is replaced by U;
(II) a fragment of the polypeptide of (I) having the activity of a beta-galactosidase as defined in (I) and comprising at least 800 amino acids.

2. A vector encoding the nucleic acid molecule of claim 1.

3. A host cell transformed, transduced or transfected with the nucleic acid molecule of claim 1 or the vector of claim 2, and a heterologous expression regulatory element which is operably linked to the nucleic acid molecule as defined in claim 1.

4. A method of producing a protein having the activity of a beta-galactosidase (EC 3.2.1.23) comprising
(a) culturing the host cell of claim 3 and
(b) isolating the produced protein having the activity of a beta-galactosidase.

5. A protein having the activity of a beta-galactosidase (EC 3.2.1.23) encoded by the nucleic acid molecule of claim 1 or produced by the method of claim 4.

6. The protein of claim 5 which is a fusion protein.

7. A composition comprising the nucleic acid of claim 1, the vector of claim 2, the host cell of claim 3, and/or the protein of claim 5 or 6, or combinations thereof.

8. The composition of claim 7, which is a food composition, a cosmetic composition, or a pharmaceutical composition.

9. The food composition or cosmetic composition of claim 8, wherein the composition comprises whey, milk and/or a milk product.

10. The food composition of claim 9, wherein the milk product is yoghurt, curd, cheese, ice cream, cream, whipped cream, or "dulce de leche".

11. The pharmaceutical composition of claim 8 for use in treating or preventing the symptoms of a lactose intolerance.

12. Use of the protein of claim 5 or 6 for the hydrolysis of a beta-galactoside, beta-glucoside and/or the synthesis of beta-galacto-oligosaccharides.

13. The use of claim 12, wherein the beta-galactoside is beta-D-galactose or lactose.

14. A process for reducing the lactose concentration in milk or a milk product, comprising contacting the host cell of claim 3, the protein of claim 5 or 6, or combinations thereof with milk or a milk product.

15. A process for the production of beta-galacto-oligosaccharides, comprising contacting the host cell of claim 3, the protein of claim 5 or 6, or combinations thereof with beta-D-galactose and/or lactose.

## Patentansprüche

1. Nukleinsäuremolekül, kodierend
(I) ein Polypeptid, das die Aktivität einer beta-Galaktosidase (EC 3.2.1.23) hat, wobei die beta-Galaktosidase **dadurch gekennzeichnet ist, dass** sie (i) ausreichende enzymatische Aktivität im Bereich von 5 bis 50°C hat, um die Hydrolyse von β-Galaktosidasen in Monosaccharide zu katalysieren, (ii) ausreichende enzymatische Aktivität im pH-Bereich von 6 bis 8 hat, um die Hydrolyse von β-Galaktosidasen in Monosaccharide zu katalysieren und (iii) eine enzymatische Aktivität hat, die nicht durch Reaktionsprodukte wie Galaktose oder andere Produkte, die substantiell in der Milch vorliegen, wie Kalzium, gehemmt wird, wobei die beta-Galaktosidase kodiert wird durch
(a) ein Nukleinsäuremolekül, das ein Polypeptid kodiert, das die Aminosäuresequenz der SEQ ID NO: 1 umfasst oder daraus besteht;
(b) ein Nukleinsäuremolekül, das die Nukleinsäuresequenz der SEQ ID NO: 2 umfasst oder daraus besteht;
(c) ein Nukleinsäuremolekül, das eine beta-Galaktosidase kodiert, deren Aminosäuresequenz mindestens zu 70% identisch mit der Aminosäuresequenz der SEQ ID NO: 1 ist;
(d) ein Nukleinsäuremolekül, das eine Nukleinsäuresequenz umfasst oder daraus besteht, die mindestens zu 70% identisch mit der Nukleinsäuresequenz der SEQ ID NO: 2 ist; oder
(e) ein Nukleinsäuremolekül, das dem Nukleinsäuremolekül nach einer der Varianten (a) bis (d) entspricht, wobei T durch U ausgetauscht ist;
(II) ein Fragment des Polypeptids aus (I), das die Aktivität einer wie in (I) definierten beta-Galaktosidase hat und mindestens 800 Aminosäuren umfasst.

2. Vektor, der das Nukleinsäuremolekül nach Anspruch 1 kodiert.

3. Wirtszelle, transformiert, transduziert oder transfiziert mit dem Nukleinsäuremolekül nach Anspruch 1 oder dem Vektor nach Anspruch 2 und einem heterologen, expressionsregulatorischen Element, das funktionell mit dem in Anspruch 1 definierten Nukleinsäuremolekül verknüpft ist.

4. Verfahren zur Herstellung eines Proteins, das die Aktivität einer beta-Galaktosidase (EC 3.2.1.23) hat, umfassend:
(a) Züchtung der Wirtszelle nach Anspruch 3 und
(b) Isolierung des hergestellten Proteins, das die Aktivität einer beta-Galaktosidase hat.

5. Protein, das die Aktivität einer beta-Galaktosidase (EC 3.2.1.23) hat, kodiert von dem Nukleinsäuremolekül nach Anspruch 1 oder hergestellt nach dem Verfahren nach Anspruch 4.

6. Protein nach Anspruch 5, das ein Fusionsprotein ist.

7. Zusammensetzung, umfassend die Nukleinsäure nach Anspruch 1, den Vektor nach Anspruch 2, die Wirtszelle nach Anspruch 3 und/oder das Protein nach Anspruch 5 oder 6 oder Kombinationen davon.

8. Zusammensetzung nach Anspruch 7, die eine Lebensmittelzusammensetzung, eine kosmetische Zusammensetzung oder eine pharmazeutische Zusammensetzung ist.

9. Lebensmittelzusammensetzung oder kosmetische Zusammensetzung nach Anspruch 8, wobei die Zusammensetzung Molke, Milch und/oder ein Milchprodukt umfasst.

10. Lebensmittelzusammensetzung nach Anspruch 9, wobei das Milchprodukt Joghurt, Quark, Käse, Eiscreme, Sahne, geschlagene Sahne oder "Dulce de leche" ist.

11. Pharmazeutische Zusammensetzung nach Anspruch 8 zur Verwendung in der Behandlung oder Prävention der Symptome einer Laktoseintoleranz.

12. Verwendung des Proteins nach Anspruch 5 oder 6 zur Hydrolyse eines beta-Galaktosids, beta-Glucosids und/oder der Synthese von beta-Galakto-Oligosacchariden.

13. Verwendung nach Anspruch 12, wobei das beta-Galaktosid beta-D-Galaktose oder Laktose ist.

14. Verfahren zur Verminderung der Laktosekonzentration in Milch oder einem Milchprodukt, umfassend das Inkontakbringen der Wirtszelle nach Anspruch 3, des Proteins nach Anspruch 5 oder 6 oder von Kombinationen davon mit Milch oder einem Milchprodukt.

15. Verfahren zur Herstellung von beta-Galakto-Oligosacchariden, umfassend das Inkontaktbringen der Wirtszelle nach Anspruch 3, des Proteins nach Anspruch 5 oder 6 oder von Kombinationen davon mit beta-D-Galaktose und/oder Laktose.

## Revendications

1. Molécule d'acide nucléique codant pour
(I) un polypeptide possédant l'activité d'une bêta-galactosidase (EC 3.2.1.23) laquelle bêta-galactosidase est **caractérisée en ce qu'**elle possède (i) une activité enzymatique suffisante dans la plage de 5 à 50 °C pour catalyser l'hydrolyse de β-galactosidases en monosaccharides, (ii) une activité enzymatique suffisante dans une plage de pH de 6 à 8 pour catalyser l'hydrolyse des β-galactosidase en monosaccharides, et (iii) une activité enzymatique qui n'est pas inhibée par les produits réactionnels tels que le galactose, ou d'autres produits substantiellement présents dans le lait, tels que le calcium, laquelle bêta-galactosidase est codée par
(a) une molécule d'acide nucléique codant pour un polypeptide comprenant ou constitué de la séquence d'acides aminés de SEQ ID NO : 1 ;
(b) une molécule d'acide nucléique comprenant ou constituée de la séquence nucléotidique de SEQ ID NO : 2 ;
(c) une molécule d'acide nucléique codant pour une bêta-galactosidase dont la séquence d'acides aminés est au moins identique à 70 % avec la séquence d'acides aminés de SEQ ID NO : 1 ;
(d) une molécule d'acide nucléique comprenant ou constituée d'une séquence nucléotidique qui est au moins identique à 70 % avec la séquence nucléotidique de SEQ ID NO : 2 ; ou
(e) une molécule d'acide nucléique correspondant à la molécule d'acide nucléique selon l'une quelconque de (a) à (d) dans laquelle T est remplacé par U ;
(II) un fragment du polypeptide de (I) possédant l'activité d'une bêta-galactosidase telle que définie en (I) et comprenant au moins 800 acides aminés.

2. Vecteur codant pour la molécule d'acide nucléique selon la revendication 1.

3. Cellule hôte transformée, transduite ou transfectée avec la molécule d'acide nucléique selon la revendication 1 ou le vecteur selon la revendication 2, et un élément régulateur de l'expression hétérologue qui est lié de façon fonctionnelle à la molécule d'acide nucléique telle que définie dans la revendication 1.

4. Méthode de production d'une protéine possédant l'activité d'une bêta-galactosidase (EC 3.2.1.23) comprenant
(a) la culture de la cellule hôte selon la revendication 3 et
(b) l'isolement de la protéine produite possédant l'activité d'une bêta-galactosidase.

5. Protéine possédant l'activité d'une bêta-galactosidase (EC 3.2.1.23) codée par la molécule d'acide nucléique selon la revendication 1 ou produite par la méthode selon la revendication 4.

6. Protéine selon la revendication 5 qui est une protéine de fusion.

7. Composition comprenant l'acide nucléique selon la revendication 1, le vecteur selon la revendication 2, la cellule hôte selon la revendication 3, et/ou la protéine selon la revendication 5 ou 6, ou des combinaisons de ceux-ci.

8. Composition selon la revendication 7, qui est une composition alimentaire, une composition cosmétique, ou une composition pharmaceutique.

9. Composition alimentaire ou composition cosmétique selon la revendication 8, dans laquelle la composition comprend du lactosérum, du lait et/ou un produit laitier.

10. Composition alimentaire selon la revendication 9, dans laquelle le produit laitier est du yaourt, du lait caillé, du fromage, de la glace, de la crème, de la crème fouettée, ou de la confiture de lait.

11. Composition pharmaceutique selon la revendication 8 pour une utilisation dans le traitement ou la prévention des symptômes d'une intolérance au lactose.

12. Utilisation de la protéine selon la revendication 5 ou 6, pour l'hydrolyse d'un bêta-galactoside, d'un bêta-glucoside et/ou la synthèse de bêta-galacto-oligosaccharides.

13. Utilisation selon la revendication 12, dans laquelle le bêta-galactoside est le bêta-D-galactose ou le lactose.

14. Procédé de réduction de la concentration de lactose dans le lait ou un produit laitier, comprenant la mise en contact de la cellule hôte selon la revendication 3, de la protéine selon la revendication 5 ou 6, ou de combinaisons de celles-ci avec le lait ou un produit laitier.

15. Procédé de production de bêta-galacto-oligosaccharides, comprenant la mise en contact de la cellule hôte selon la revendication 3, de la protéine selon la revendication 5 ou 6, ou de combinaisons de celles-ci, avec du bêta-D-galactose et/ou du lactose.


### SEQUENCE LISTING

<110> B.R.A.I.N. Biotechnology Research and Information Network AG
<120> Novel Beta-Galactosidases useful for the production of lactose depleted milk products
<130> T1842 EP
<160> 10
<170> PatentIn version 3.5
<210> 1
   <211> 1039
   <212> PRT
   <213> unknown
<220>
   <223> metagenome library 3J_33_AA
<400> 1
   Met Arg Gln Lys Leu Val Tyr Ile Pro Pro Ala Asn Gly Tyr Pro Glu
   1               5                   10                  15
   Trp Asp Asn Asn Pro Glu Leu Phe Gln Val Asn Arg Met Glu Ala His
               20                  25                  30
   Ala Thr Trp Ile Pro Phe Asp Thr Lys Glu Glu Ala Phe Ile Gly Asp
           35                  40                  45
   Phe Lys Ala Ser Ser Asn Tyr Leu Ser Leu Asn Gly Leu Trp Lys Phe
       50                  55                  60
   Ala Tyr Ala Asp Thr Pro Asp Lys Arg Ile Arg Ser Phe Tyr Glu Gln
   65                  70                  75                  80
   Asp Tyr Asp Cys Ser Ser Trp Ala Glu Leu Thr Val Pro Ser His Trp
                   85                  90                  95
   Gln Leu His Gly Tyr Asp Tyr Pro Gln Tyr Thr Asn Val Asn Tyr Pro
               100                 105                 110
   Trp Ser Glu Arg Glu Pro Glu Leu Lys Pro Pro Phe Ala Pro Ser Lys
           115                 120                 125
   Tyr Asn Pro Val Gly Ser Tyr Val Arg Thr Phe Thr Val Pro Glu Gly
       130                 135                 140
   Trp Val Glu Lys Pro Val Phe Ile Ser Phe Gln Gly Val Glu Ser Ala
   145                 150                 155                 160
   Phe Tyr Val Trp Val Asn Gly Asp Leu Val Gly Tyr Ser Glu Asp Thr
                   165                 170                 175
   Phe Thr Pro Ala Glu Phe Asp Ile Thr Pro Tyr Leu Thr Pro Gly Glu
               180                 185                 190
   Asn Lys Leu Ala Val Glu Val Tyr Lys Trp Cys Asp Ala Ser Trp Leu
           195                 200                 205
   Glu Asp Gln Asp Phe Trp Arg Leu Ser Gly Ile Phe Arg Asp Val Tyr
       210                 215                 220
   Leu Tyr Thr Ala Pro Glu Thr His Ile Tyr Asp Phe Phe Val Arg Thr
   225                 230                 235                 240
   Glu Leu Asp Asp Gln Tyr Arg His Ala Glu Leu Gln Leu Asp Val Lys
                   245                 250                 255
   Leu Lys Asp Tyr Phe Ala Arg Thr Thr Glu Ala Ile His Val Asp Ala
               260                 265                 270
   Glu Leu Tyr Asp His Asn Gln Asp Pro Val Phe Asp Asn Pro Leu Ser
           275                 280                 285
   Gln Ile Ile Asn Phe Asn Gly Ala Ser Asn Gln Thr Phe Ser Leu Ser
       290                 295                 300
   Ala Ser Val Leu Asp Pro Leu Lys Trp Ser Ala Glu Gln Pro Asn Leu
   305                 310                 315                 320
   Tyr Thr Leu Ile Leu Ser Leu Arg Asn Asp Asn Gly Asp Leu Leu Glu
                   325                 330                 335
   Val Val Arg Cys Arg Val Gly Phe Arg Lys Phe Glu Leu Lys Asp Gly
               340                 345                 350
   Leu Met Lys Ile Asn Gly Gln Arg Ile Val Phe Lys Gly Val Asn Arg
           355                 360                 365
   His Glu Phe Ser Cys Asp Thr Gly Arg Ala Ile Ser Val Asp Asp Met
       370                 375                 380
   Ile Gln Asp Ile Gln Leu Met Lys Ala His Asn Ile Asn Ala Val Arg
   385                 390                 395                 400
   Thr Ser His Tyr Pro Asn Gln Thr Thr Trp Tyr Glu Leu Cys Asp Glu
                   405                 410                 415
   Tyr Gly Leu Tyr Val Ile Asp Glu Thr Asn Leu Glu Thr His Gly Ser
               420                 425                 430
   Trp Ser Tyr Gly Gln Lys Glu Leu Gly Glu His Thr Val Pro Gly Ser
           435                 440                 445
   Lys Pro Glu Trp Arg Asp Asn Val Leu Asp Arg Cys Asn Ser Met Phe
       450                 455                 460
   Gln Arg Asp Lys Asn His Pro Ser Ile Ile Ile Trp Ser Leu Gly Asn
   465                 470                 475                 480
   Glu Ser Phe Gly Gly Asp Asn Phe Ile Ser Met Tyr Asp Phe Leu Lys
                   485                 490                 495
   Gln Glu Asp Pro Thr Arg Leu Val His Tyr Glu Gly Gln Phe His Tyr
               500                 505                 510
   Arg Glu Ser Ser Ala Ala Ser Asp Ile Glu Ser Thr Met Tyr Val Lys
           515                 520                 525
   Pro Val Asp Val Glu Lys Tyr Ala Leu Asn Asp Pro Lys Lys Pro Tyr
       530                 535                 540
   Ile Ile Cys Glu Tyr Ser His Ala Met Gly Asn Ser Cys Gly Gly Leu
   545                 550                 555                 560
   His Leu Tyr Trp Glu Leu Phe Glu Lys Tyr Asp Ile Leu Gln Gly Ala
                   565                 570                 575
   Phe Ile Trp Asp Trp Ile Asp Gln Ala Ile Arg Ile Glu Gln Ala Asp
               580                 585                 590
   Gly Thr Val Gln Met Ala Tyr Gly Gly Asp Phe Gly Glu Ser Pro His
           595                 600                 605
   Asp Gly Asn Phe Cys Gly Asn Gly Leu Ile Phe Ala Asp His Ile Val
       610                 615                 620
   Ser Pro Lys Leu Tyr Glu Val Lys Lys Cys Tyr Gln Asn Val Lys Phe
   625                 630                 635                 640
   Glu Ala Val Asp Ile Asp Ser Gly His Ile Arg Val Thr Asn Arg Asn
                   645                 650                 655
   Leu Phe Thr Asp Leu Ala Glu Tyr Asp Leu Lys Trp Glu Val Ala Cys
               660                 665                 670
   Asn Gly Gln Ile Val Gln Thr Gly Gly Ile Asp Leu Ala Val Pro Ala
           675                 680                 685
   Gly Asp Val Ala Glu Ile Thr Val Pro Phe Val His Val Asn Glu Ser
       690                 695                 700
   Thr Pro Asp Gly Glu Tyr Val Leu Thr Leu Ser Met Val Leu Lys Glu
   705                 710                 715                 720
   Ala Thr Leu Trp Ala Glu Val Gly His Glu Leu Ala Trp Glu Gln Phe
   725 730 735
   Val Leu Pro Asn Ser Arg His Glu Ala Leu Pro Thr Gln Thr Thr Ser
   740 745 750
   Ser Asp Ser Val Leu Val Lys Glu Gln Ala Asp Gln Leu Ile Val Gln
   755 760 765
   Val Ala Asp Val Ser Leu Gln Phe Asn Lys Ser Asn Gly Cys Leu Ser
   770 775 780
   Ser Ile Gln Asn Ala Gly Glu Glu Leu Leu Leu Glu Pro Val Arg Pro
   785 790 795 800
   Asn Phe Trp Arg Ala Val Thr Asp Asn Asp Leu Gly Asn Lys Leu Asn
   805 810 815
   Glu Arg Cys Ala Val Trp Arg Thr Val Trp Asp Thr Ser Thr Leu Asn
   820 825 830
   Ser Phe Glu Trp His Lys Asp Ser Asp Asp Val Ile Val Arg Ala Glu
   835 840 845
   Tyr Ser Leu His Thr Glu Pro Val Ser Ser Leu Thr Leu Glu Tyr Lys
   850 855 860
   Val Gly Ser Asn Gly Ser Ile Asn Val Phe Glu Glu Phe Ser Pro Gly
   865 870 875 880
   Lys Gly Leu Pro Glu Ile Pro Glu Ile Gly Met Leu Phe Val Leu Lys
   885 890 895
   Asp Ser Leu Asn Thr Val Ser Trp Tyr Gly Arg Gly Pro His Glu Asn
   900 905 910
   Tyr Trp Asp Arg Lys Thr Gly Ala Arg Leu Gly Tyr Phe Ser Gly Arg
   915 920 925
   Ile Gln Asp Gln Phe Val Pro Tyr Ile Arg Pro Gln Glu Cys Gly Asn
   930 935 940
   Lys Thr Asp Val Arg Tyr Ala Ser Ile Thr Gln Gly Thr Asn Gly Ser
   945 950 955 960
   Gly Ile His Ile Glu Gly Asp Pro Val Leu Glu Leu Asn Ala Leu Pro
   965 970 975
   Trp Thr Pro Glu Glu Leu Glu Ala His Asp His Val Tyr Lys Leu Pro
   980 985 990
   Ala Ser Asn Lys Thr Val Val Arg Val Asn Tyr Lys Gln Met Gly Val
   995 1000 1005
   Gly Gly Asp Asp Ser Trp Asn Ala Pro Thr His Glu Glu Phe Thr
   1010 1015 1020
   Leu Pro Ala Asp Lys Thr Tyr Gly Phe Asp Phe Thr Phe Arg Thr
   1025 1030 1035
   Ile
<210> 2
   <211> 3120
   <212> DNA
   <213> unknown
<220>
   <223> metagenome library 3J_33_DNA
<400> 2
   atgcgacaaa agcttgttta tataccccca gctaatggat atcccgagtg ggataataac      60
   ccagaattat ttcaggttaa ccgaatggaa gcacacgcca cttggatccc ctttgatacc     120
   aaagaggaag cattcattgg agactttaaa gcttcgtcta actatttgtc attaaatggt     180
   ctatggaaat tcgcctatgc agatacccca gataaacgca ttcgttcgtt ttatgaacaa     240
   gactatgact gcagttcttg ggctgaacta accgtccctt cccattggca attacatggc     300
   tacgattatc cacaatacac aaatgtgaac tacccgtgga gtgagcgaga acccgagctg     360
   aagccgccat ttgctccttc caaatacaat ccggtaggct cttatgtacg gaccttcact     420
   gttccagaag gatgggtgga aaaacccgta ttcattagtt ttcaaggtgt cgaatccgct     480
   ttctatgttt gggtcaacgg cgacttagtc ggttacagcg aagatacatt tactccagcg     540
   gaattcgata taactcctta tcttacccca ggagagaata agttagcggt tgaagtctac     600
   aaatggtgtg atgcaagctg gctggaggat caggatttct ggcgtcttag cggtattttt     660
   agagatgtgt atttatatac agcacctgag acacacatct atgatttctt tgttcgaacc     720
   gaactagatg atcaataccg acatgctgaa ctgcaactag atgtgaagct caaagattat     780
   ttcgcaagaa caacggaagc aatccatgta gatgccgaac tctatgatca caatcaggac     840
   ccggtgttcg ataatccact ttcacaaatt attaacttta atggcgcctc aaaccaaacg     900
   tttagcctgt cagcatctgt cttggatccg ttaaaatgga gtgccgaaca accaaatctg     960
   tataccttga tcctgtcact gcgtaatgac aatggtgatc ttctcgaagt agttcgctgc    1020
   cgtgtgggtt tccgaaagtt tgaactgaaa gacgggttaa tgaagatcaa tgggcaacgg    1080
   attgttttta aaggcgttaa tcgccatgaa ttttcatgtg atacgggcag agcgattagt    1140
   gtggatgaca tgatacaaga cattcaatta atgaaggccc acaacatcaa tgccgttcga    1200
   acctcacatt acccgaatca aacaacctgg tatgaactgt gtgatgaata tggactttat    1260
   gtcattgatg agacgaacct ggaaacgcat ggatcatgga gttatggaca gaaagaacta    1320
   ggtgaacaca cagttcctgg tagtaaacct gaatggcgcg acaatgtatt agaccggtgt 1380
   aattcaatgt ttcagcggga caaaaaccac ccctcgatta tcatctggtc actcggcaac 1440
   gaatcgtttg gtggcgacaa ttttatttct atgtatgatt tccttaagca ggaggatcct 1500
   acccggcttg tccattacga aggtcaattc cattatcgcg aaagtagtgc agcctctgat 1560
   atcgaaagta cgatgtacgt caaaccggtt gatgtggaga aatatgccct aaatgaccca 1620
   aaaaaacctt acattatatg cgagtatagt catgccatgg gtaactcttg tggtggcctg 1680
   catctgtatt gggagctctt tgagaaatat gatattttac aaggtgcgtt tatttgggac 1740
   tggatcgatc aagccattcg tattgagcaa gccgacggaa ctgtacaaat ggcttacggg 1800
   ggagacttcg gtgaatcacc tcatgacggg aatttttgcg gaaatggctt aatatttgct 1860
   gaccacatcg tatctccgaa gctttatgaa gtgaagaaat gctaccagaa cgtcaagttc 1920
   gaagcagttg atatagacag tggacatatc cgggtaacta accggaatct attcacggat 1980
   ctagcagagt atgatctaaa gtgggaagta gcctgcaatg ggcaaatagt ccaaactggc 2040
   gggatagatc ttgctgtacc agcaggagat gttgctgaaa ttactgttcc tttcgttcat 2100
   gttaatgaat cgacaccaga cggtgagtat gttctaacgc tcagtatggt acttaaagag 2160
   gctactcttt gggcagaagt gggtcatgaa ttggcctggg aacaattcgt ccttcctaat 2220
   tctaggcatg aggctctccc tacccagaca acaagcagtg acagtgttct tgtgaaggag 2280
   caggcagatc agttgatcgt tcaggttgca gatgtctcct tacaattcaa taagtcaaac 2340
   ggctgtctat cctcaattca gaacgcaggc gaagagcttc tacttgaacc cgtacgtcca 2400
   aacttctggc gtgctgttac ggataacgat ttgggtaaca agcttaacga acgttgcgct 2460
   gtttggagaa cggtatggga tacgagcaca ctcaactcgt ttgaatggca taaagatagt 2520
   gatgatgtta tcgttcgtgc cgagtactca ttgcataccg agcctgtttc ctcgcttaca 2580
   ttggagtaca aagtcggtag taatggatcc atcaacgtat ttgaagagtt ttcaccaggt 2640
   aaaggattgc ctgaaatccc cgagatcggg atgttatttg tgttaaaaga ttcgctcaac 2700
   accgtatcgt ggtatgggcg tggtcctcat gaaaactact gggatcgcaa aacaggagca 2760
   cgtcttggtt atttctctgg gcgtatccaa gatcaatttg ttccttacat ccggcctcaa 2820
   gagtgcggca acaagacgga tgttcgttat gctagcatca ctcaagggac gaatggatct 2880
   ggcatccata tcgaaggtga cccagtacta gaacttaatg cccttccatg gacaccagag 2940
   gaactagaag ctcacgatca tgtatataaa ctgcctgcta gtaataaaac ggtagtaaga 3000
   gtgaactaca aacaaatggg tgtaggtgga gacgatagct ggaatgcacc aacgcatgaa 3060
   gagttcaccc taccagcaga taaaacttat ggattcgact ttacgtttcg caccatttaa 3120
<210> 3
   <211> 1019
   <212> PRT
   <213> unknown
<220>
   <223> metagenome library 3J_37_AA
<400> 3
   Met Lys Leu Ala Val Thr Pro Ser Leu Ser Trp Leu Glu Asp Pro Gly
   1               5                   10                  15
   Val Phe Lys Val Asn Arg Leu Glu Ala His Ser Asp His Arg Tyr Tyr
               20                  25                  30
   Ser Asp Met Ala Lys Ala Glu Ala Gly Ala Pro Met Asp Met Arg Tyr
           35                  40                  45
   Ser Leu Asn Gly Ser Trp Lys Phe Ser Tyr Ala Pro Asn Pro Ala Ser
       50                  55                  60
   Arg Val Glu Ser Phe Phe Ala Glu Ser Tyr Asn Cys Ser Ala Trp Asp
   65                  70                  75                  80
   Ser Ile Gln Val Pro Gly His Ile Gln Leu Gln Gly Phe Gly Arg Ala
                   85                  90                  95
   Gln Tyr Val Asn Thr Met Tyr Pro Trp Asp Gly Leu Ser Asp Val Arg
               100                 105                 110
   Pro Pro His Ile Pro Gln Glu His Asn Pro Val Gly Ser Tyr Val Lys
           115                 120                 125
   Glu Phe Arg Leu Pro Ala Asn Leu Glu Asp Gly Pro Leu Tyr Ile Ser
       130                 135                 140
   Phe Gln Gly Val Glu Ser Ala Phe Tyr Val Trp Leu Asn Gly Glu Phe
   145                 150                 155                 160
   Val Gly Tyr Ala Glu Asp Ser Phe Thr Pro Ser Glu Phe Asp Leu Thr
                   165                 170                 175
   Pro Tyr Val Arg Thr Gly Lys Asn Lys Leu Ala Val Glu Val Tyr Gln
               180                 185                 190
   Arg Ser Thr Gly Gly Trp Leu Glu Asp Gln Asp Phe Trp Arg Phe Ser
           195                 200                 205
   Gly Ile Phe Arg Asp Val Tyr Leu Tyr Thr Val Pro Lys Gln His Ile
       210                 215                 220
   Arg Asp Leu Ala Val Arg Ala Glu Leu Asp Asp Thr Tyr Thr Gln Gly
   225                 230                 235                 240
   Ser Leu Asn Val Gln Met Gln Leu Leu Asp Thr Pro Gln Glu Asn Val
                   245                 250                 255
   Ser Ala Arg Ile Glu Leu Lys Asp Lys Ala Gly Gln Val Arg Leu Ser
               260                 265                 270
   Thr Glu Gly Gly Phe Ser Gly Arg Glu Leu Thr Leu Ser Gly Asp Ile
           275                 280                 285
   Ala Asp Val Leu Thr Trp Ser Ala Glu Asn Pro Tyr Leu Tyr Ser Leu
       290                 295                 300
   Cys Ile Ser Leu Leu Asp Ala Gln Gly Lys Leu Ile Glu Ala Ile Val
   305                 310                 315                 320
   Gln Lys Ala Gly Phe Arg Arg Phe Glu Leu Lys Asn Lys Leu Met Cys
                   325                 330                 335
   Ile Asn Gly Lys Arg Ile Val Phe Lys Gly Val Asn Arg His Glu Phe
               340                 345                 350
   Asn Cys Arg Thr Gly Arg Thr Ile Thr Arg Glu Asp Met Leu Trp Asp
           355                 360                 365
   Ile Arg Thr Leu Lys Arg Ser Asn Ile Asn Ala Val Arg Thr Ser His
       370                 375                 380
   Tyr Pro Asn Gln Thr Leu Trp Tyr Glu Leu Cys Asp Glu Tyr Gly Ile
   385                 390                 395                 400
   Tyr Val Ile Asp Glu Met Asn Leu Glu Thr His Gly Ser Trp Gln Lys
                   405                 410                 415
   Met Gly Ala Val Glu Pro Ser Trp Asn Ile Pro Gly Asn Asp Pro Asn
               420                 425                 430
   Trp Arg Asp Ile Val Leu Asp Arg Ala Glu Ser Met Leu Glu Arg Asp
           435                 440                 445
   Lys Asn His Pro Ser Ile Leu Ile Trp Ser Cys Gly Asn Glu Ser Tyr
       450                 455                 460
   Ala Gly Gln Val Ile Leu Asp Val Ala Asn Trp Phe Arg Ser Arg Asp
   465                 470                 475                 480
   Asn Ser Arg Leu Val His Tyr Glu Gly Val Phe Tyr Asp Arg Asn Tyr
                   485                 490                 495
   Asn Ala Thr Ser Asp Met Glu Ser Arg Met Tyr Ala Lys Pro Ala Asp
               500                 505                 510
   Ile Glu Glu Tyr Leu Asn Ser Asn Pro Asp Lys Pro Tyr Leu Ser Cys
           515                 520                 525
   Glu Tyr Met His Ala Met Gly Asn Ser Val Gly Gly Met His Lys Tyr
   530 535 540
   Thr Glu Leu Glu Asn Lys Tyr Pro Met Tyr Gln Gly Gly Phe Ile Trp
   545 550 555 560
   Asp Tyr Ile Asp Gln Ser Ile Ile Thr Lys Glu Ser Pro Thr Ala Ala
   565 570 575
   Ser Ile Tyr Ala Tyr Gly Gly Asp Phe Gln Asp Arg Pro Thr Asp Tyr
   580 585 590
   Ser Phe Cys Gly Asn Gly Ile Val Tyr Ala Asp Arg Gln Trp Thr Pro
   595 600 605
   Lys Met Gln Glu Val Lys Phe Leu Tyr Gln Asn Tyr Lys Leu Glu Pro
   610 615 620
   Gly Lys Thr Gly Ile Arg Leu Ile Asn Glu Ser Leu Phe Ala Gly Thr
   625 630 635 640
   Gly His Leu Gly Leu Glu Tyr Arg Leu Tyr Arg Glu Gly Glu Leu Leu
   645 650 655
   Gly Ala Trp Thr Thr Asp Val Asn Ala Ala Pro Gln Ser Glu Val Leu
   660 665 670
   Val Pro Val Glu Leu Pro Gln Ala Asp Gly Lys Pro Gly Glu Tyr Ser
   675 680 685
   Ile Gln Ala Ser Leu Leu Leu Lys Asp Ala Glu Leu Trp Ala Glu Ala
   690 695 700
   Gly Tyr Glu Ile Ala Phe Gly Glu Ser Val Phe Val Val Gln Glu Glu
   705 710 715 720
   Ala Glu Ala Glu Gln Pro Ala Gly Glu Leu Lys Val Val Glu Gly Asp
   725 730 735
   Val Gln Ile Gly Val Leu Gly Glu Gly Phe Thr Val Leu Phe Ser Lys
   740 745 750
   Gln Val Gly Ser Leu Val Ser Leu Asn Ile His Gly Arg Glu Met Ile
   755 760 765
   Ala Ala Pro Pro Ala Pro Leu Phe Trp Arg Ala Ala Thr Asp Asn Asp
   770 775 780
   Lys Gly Thr Asn Leu Gly Tyr Asn Ala Gly Cys Trp Tyr Ala Ala Ser
   785 790 795 800
   Leu Ser Arg Lys Cys Val Glu Val Ser Leu Ala Gln Glu Glu Gly Thr
                   805                 810                 815
   Val Thr Val Ser Phe Asp Tyr Arg Phe Ser Ile His Pro Gly Leu Ser
               820                 825                 830
   Val Gln Thr Ala Tyr Thr Val Tyr Gly Asp Gly Lys Val Gln Val His
           835                 840                 845
   Thr Arg Tyr Asn Gly Ala Ala Gly Leu Pro Asp Leu Pro Ile Leu Ala
       850                 855                 860
   Leu Ser Phe Lys Met Pro Ala Asp Tyr Asp Gln Thr Glu Trp Tyr Ala
   865                 870                 875                 880
   Met Gly Pro Glu Glu Asn Tyr Ile Asp Arg Ala Tyr Gly Ala Arg Leu
                   885                 890                 895
   Gly Val Phe Ser Arg Ser Val Gln Glu Leu Pro Ser Arg Tyr Leu Val
               900                 905                 910
   Pro Gln Glu Ser Gly Asn Arg Thr Gly Val Arg Arg Val Lys Ile Thr
           915                 920                 925
   Asp Lys Ser Gly Val Gly Leu Gln Ile Ser Ala Pro Ala Thr Thr Pro
       930                 935                 940
   Val Glu Cys Asn Leu Ser Pro Tyr Thr Ala Phe Glu Leu Glu His Ala
   945                 950                 955                 960
   Ser His Ala Phe Glu Leu Pro Ala Val His Tyr Thr Val Val Thr Val
                   965                 970                 975
   Ala Gly Arg Gln Met Gly Val Gly Gly Asp Asp Ser Trp Gly Ala Pro
               980                 985                 990
   Val Tyr Lys Glu Tyr Thr Ile Pro  Ala Glu Gln Ser Leu  Glu Tyr Glu
           995                 1000                1005
   Phe Phe  Ile Ser Ser Val Gly  Thr Glu Glu Lys
       1010                 1015
<210> 4
   <211> 3060
   <212> DNA
   <213> unknown
<220>
   <223> metagenome library 3J_37_DNA
<400> 4
   atgaaattgg cagttacccc aagtttgagc tggctggagg acccgggtgt gttcaaggtg 60
   aaccggctgg aggcccattc ggaccaccgt tattattcag acatggctaa ggcagaggcg 120
   ggtgcgccga tggatatgcg ctatagcctt aacggcagct ggaaattcag ctatgcgccg     180
   aatccggcca gccgtgtaga gtcgtttttt gcggaatcct ataattgctc cgcatgggac     240
   agcatccagg ttcccggaca tatccagctg cagggcttcg gccgggcaca atatgtcaat     300
   acgatgtatc cgtgggatgg actaagtgat gtccgcccgc cgcatatccc gcaggagcat     360
   aacccggtcg gcagttatgt gaaggaattc cgtctcccgg cgaacctgga ggacgggccg     420
   ctctatatct ccttccaagg ggtggagtcg gctttttatg tctggcttaa cggggaattt     480
   gtcggctacg ccgaggatag ctttacaccg tcggaatttg atctgacccc gtatgtgcgt     540
   acaggcaaaa acaagctggc cgtcgaggta taccagcgca gtaccggagg ctggctggag     600
   gatcaggatt tctggcggtt ctcgggtatt ttccgcgacg tctatttgta tactgttccg     660
   aagcagcata tccgggatct cgcggtgcgg gcggagcttg acgatactta tacgcaagga     720
   agcctgaatg ttcaaatgca gctgctggat actccgcagg agaacgttag cgctagaatt     780
   gagctcaagg ataaagcagg acaggtccgt ctgagtacgg agggcgggtt cagcggacgt     840
   gaactgacct tgtctggaga cattgcggat gttctgacgt ggagtgcaga gaatccgtat     900
   ctgtattcgc tctgcatctc gctgctcgat gcacagggga agctcattga agcgattgtc     960
   cagaaggcgg ggttccgccg gttcgagctg aagaacaagc tgatgtgcat caacggcaag    1020
   cggattgtat tcaaaggcgt caaccggcat gaattcaact gccgcaccgg ccggacgatt    1080
   acccgtgagg acatgctgtg ggacatccgt acgctgaagc gcagcaacat taacgccgta    1140
   cgtacgtcgc attatcccaa ccaaacacta tggtatgagc tgtgcgatga atacggcatt    1200
   tacgtcatcg acgaaatgaa tctggaaacc catggctcgt ggcaaaagat gggggctgtt    1260
   gagccttcgt ggaacattcc cggcaacgat ccgaattggc gcgatatcgt gctggaccgg    1320
   gcagagtcta tgctggaacg ggataagaat catccatcca ttctaatctg gtcctgcggc    1380
   aacgagtcat acgcgggtca ggtcattctg gatgtggcca attggttccg cagccgtgat    1440
   aatagccgcc ttgtccatta tgaaggtgtg ttctatgacc gtaattataa tgccacgagc    1500
   gatatggaga gccggatgta tgccaaaccg gcggatatcg aagagtattt gaacagcaat    1560
   ccggacaagc cttatctcag ctgcgaatac atgcatgcga tgggcaattc ggtgggcggc    1620
   atgcataagt atacggagct ggagaataag tacccgatgt accagggtgg cttcatttgg    1680
   gattatatcg atcagtcgat tattactaag gaatcgccta cggccgcgag tatttatgct    1740
   tacggcggcg atttccagga ccgtccgacg gactacagct tctgcggcaa tggcattgtc    1800
   tacgccgacc ggcagtggac gccgaagatg caggaggtta aattcctgta ccagaactac    1860
   aagctggagc ctggtaaaac aggcattcgg ctcattaatg aaagcttgtt cgcaggaacc    1920
   ggacatctgg gattggaata ccggctgtat cgtgaaggcg agctgctcgg cgcttggaca    1980
   actgacgtca atgccgcacc gcaaagcgaa gtgctggtgc cggtcgagct gccgcaagca    2040
   gacgggaaac cgggcgaata cagcattcag gcctcattgc tgctgaagga tgcggagctc    2100
   tgggctgagg ccggatatga aattgccttt ggagagtccg tcttcgtagt gcaagaggaa    2160
   gctgaagcag agcagccagc cggcgaactg aaggttgtgg aaggggatgt acagatcggc 2220
   gtccttggtg agggcttcac cgtgctgttc tctaaacagg ttggttctct ggtgtccttg 2280
   aacatccacg gacgagaaat gattgcggct cctccggccc cgctgttctg gcgggcggcg 2340
   accgacaacg acaaaggaac caatctcggc tacaatgccg gatgctggta tgctgcaagc 2400
   ctgtcgcgca aatgtgtaga ggtgagcctt gcacaggagg aaggcacagt cacggtgagc 2460
   tttgattacc ggttcagcat ccatcccggg ctgtcggttc aaaccgccta tacggtctac 2520
   ggcgacggaa aggtacaggt gcatacccgc tacaatggcg ctgccgggct tccggatctg 2580
   ccgatcctgg cattgtcctt caaaatgccg gccgactacg accagacgga atggtatgcg 2640
   atgggaccag aagagaatta tatcgaccgt gcgtacggtg ccagacttgg cgtgttcagc 2700
   cggagcgtac aggagctgcc gtcccgctac cttgttccgc aggaatccgg taaccgcacc 2760
   ggtgtacgcc gcgtgaagat taccgataaa tcgggagtgg gcctgcaaat ctcggcccct 2820
   gcaaccacgc ctgtggaatg taatttgtcg ccgtataccg cgttcgagct ggagcatgcc 2880
   agccatgcgt ttgagcttcc ggcggttcat tacaccgtag tgacagtagc aggaagacaa 2940
   atgggcgtag gcggagacga tagctggggc gcaccagtat ataaggaata caccattcct 3000
   gcagagcaaa gtcttgaata tgagtttttc atttcaagcg tgggaacgga agagaaatag 3060
<210> 5
   <211> 1039
   <212> PRT
   <213> unknown
<220>
   <223> metagenome library 3J_11_AA
<400> 5
   Met Arg Lys Lys Leu Val His Thr Pro Pro Ala Asn Gly Tyr Pro Glu
   1               5                   10                  15
   Trp Asn Asn Asn Pro Glu Thr Phe Gln Leu Asn Arg Leu Pro Ala His
               20                  25                  30
   Ala Ser Met Val Ala Phe Pro Ser Ile Thr Glu Ala Leu Ser Asn Asp
           35                  40                  45
   Ser Ser Ala Ser Pro Trp Tyr Glu Ser Leu Asn Gly Gln Trp Lys Phe
       50                  55                  60
   Ala Phe Ala Glu Thr Pro Glu Gln Arg Ile Thr Ser Phe Tyr Glu Asn
   65                  70                  75                  80
   Asn Tyr Asp Ser Ser Asp Trp Asp Glu Ile Pro Val Pro Ser Asn Trp
                   85                  90                  95
   Gln Leu Gln Gly Tyr Asp Tyr Pro His Tyr Thr Asn Met Thr Tyr Pro
               100                 105                 110
   Trp Val Glu Arg Glu Pro Glu Leu Lys Pro Pro Phe Ala Pro Thr Thr
   115 120 125
   Tyr Asn Pro Val Gly Ser Tyr Ile Arg Thr Phe Thr Val Pro Ala Asp
   130 135 140
   Trp Lys Asp Arg Pro Val Leu Leu His Phe Glu Gly Val Glu Ser Ala
   145 150 155 160
   Phe Tyr Val Trp Val Asn Gly Glu Leu Val Gly Tyr Ser Glu Asp Thr
   165 170 175
   Phe Thr Pro Ala Glu Phe Asp Ile Thr Ser Tyr Leu Thr Glu Gly Glu
   180 185 190
   Asn Lys Leu Ala Val Glu Val Tyr Arg Trp Cys Asp Ala Ser Trp Leu
   195 200 205
   Glu Asn Gln Asp Phe Trp Arg Leu Ser Gly Ile Phe Arg Gly Val Tyr
   210 215 220
   Met His Ser Pro Ser Pro Val Gln Ile Ala Asp Phe Phe Val Arg Thr
   225 230 235 240
   Glu Leu Asp Asp Ala Tyr Gln Asp Ala Glu Leu Leu Leu Asn Leu Lys
   245 250 255
   Phe Phe Asn His Asn Ala Pro Gln Asn Thr Ala Gly Leu Ser Val Gln
   260 265 270
   Ala Gln Leu Tyr Asp Ala Gln Gln Gln Thr Val Leu Lys Gln Pro Leu
   275 280 285
   Thr Ala Ala Val Thr Phe Gln Gly Glu Asp Glu Leu Ser Phe Asn Leu
   290 295 300
   Ser Ala Glu Val Ile Gln Pro Leu Leu Trp Ser Ala Glu Ser Pro His
   305 310 315 320
   Leu Tyr Thr Leu Val Leu Ser Ile Gln Asp Glu Ser Gly Glu Thr Leu
   325 330 335
   Glu Ala Val Arg Ser Arg Ile Gly Phe Arg Lys Phe Glu Leu Lys Asp
   340 345 350
   Gly Leu Met Gln Ile Asn Gly Lys Arg Ile Val Phe Lys Gly Val Asn
   355 360 365
   Arg His Glu Phe Ser Pro Asp Lys Gly Arg Ala Ile Gly Arg Glu Asp
   370 375 380
   Met Ile Arg Asp Ile Glu Leu Met Lys Ser Tyr Asn Val Asn Ala Val
   385                 390                 395                 400
   Arg Thr Ser His Tyr Pro Asn Gln Ser Leu Trp Tyr Glu Leu Cys Asp
                   405                 410                 415
   Glu Tyr Gly Leu Tyr Val Ile Asp Glu Thr Asn Leu Glu Thr His Gly
               420                 425                 430
   Thr Trp Glu Tyr Gly Gln Lys Glu Met Asn Glu Asn Asn Ile Pro Ala
           435                 440                 445
   Ser Lys Pro Glu Trp Arg Asn Asn Val Ile Asp Arg Cys Asn Ser Met
       450                 455                 460
   Phe Gln Arg Asp Lys Asn His Ala Ser Val Ile Ile Trp Ser Leu Gly
   465                 470                 475                 480
   Asn Glu Ser Phe Gly Gly Asn Asn Phe Ile Ala Met Tyr Asp Tyr Leu
                   485                 490                 495
   Lys Gln Val Asp Pro Thr Arg Leu Val His Tyr Glu Gly Thr Phe His
               500                 505                 510
   Tyr Arg Pro Ser Asp Ser Ala Ser Asp Ile Glu Ser Thr Met Tyr Ile
           515                 520                 525
   Ser Pro Gln Asp Val Glu Gln Tyr Ala Arg Met Lys Gly Pro Lys Lys
       530                 535                 540
   Pro Tyr Ile Ile Cys Glu Tyr Ser His Ala Met Gly Asn Ser Cys Gly
   545                 550                 555                 560
   Gly Leu His Leu Tyr Trp Asp Leu Phe Asp Lys Phe Asp Val Leu Gln
                   565                 570                 575
   Gly Ala Phe Ile Trp Asp Trp Val Asp Gln Ser Ile Arg Thr Thr Thr
               580                 585                 590
   Ala Asp Gly Val Glu Tyr Phe Ala Tyr Gly Gly Asp Phe Gly Glu Ser
           595                 600                 605
   Pro His Asp Gly Asn Phe Cys Gly Asn Gly Leu Ile Leu Ala Asp Lys
       610                 615                 620
   Thr Val Thr Pro Lys Leu Glu Glu Val Lys Lys Cys Tyr Gln Asn Val
   625                 630                 635                 640
   Arg Met Glu Thr Val Asp Val Lys Asp Gly Leu Leu Arg Ile Lys Asn
                   645                 650                 655
   Gln Phe Leu Phe Thr Asp Leu Ser Glu Tyr Ser Leu Val Trp Thr Val
               660                 665                 670
   Thr His Asp Gly Lys Ser Val Glu Asn Gly Thr Leu Asp Ile Ala Val
           675                 680                 685
   Pro Pro Gly Glu Ser Val Glu Val Arg Ile Pro Tyr Thr Pro Ser Ser
       690                 695                 700
   Asp Leu Phe Gln Glu Ala Val Leu Thr Val Ser Leu Val Thr Lys Val
   705                 710                 715                 720
   Ala Thr Lys Trp Ala Gly Val Gly His Glu Ile Ala Trp Asp Gln Phe
                   725                 730                 735
   Val Val Ser Pro Arg Leu Arg Pro Ile Gln Ser Ile Arg Gln Gly Gln
               740                 745                 750
   Asp Asn Ala Pro Gln Val Gln Asp Leu Gln Asp Val Leu Lys Val Ala
           755                 760                 765
   Thr Gly Gln Val Thr Leu Ser Phe Asn Pro Ala Thr Gly Ala Leu Thr
       770                 775                 780
   Ser Tyr Gln Ile Asn Asn Gln Glu Gln Leu Leu Ala Pro Val Arg Pro
   785                 790                 795                 800
   Asn Phe Trp Arg Ala Met Thr Asp Asn Asp Met Gly Asn Arg Leu Asn
                   805                 810                 815
   Glu Arg Ser Ala Phe Trp Arg Asp Ala His Ala Thr Ser Arg Leu Ile
               820                 825                 830
   Arg Phe Glu His His Ala Asp Glu Gln Gly Phe Leu Val Thr Thr Asp
           835                 840                 845
   Tyr Thr Trp Asp Gln His Pro Gly Cys Thr Leu Ser Ile Thr Tyr Arg
       850                 855                 860
   Ile Asp Ala Asp Gly Val Leu Glu Ile Ser Gln Thr Leu Ile Pro Gly
   865                 870                 875                 880
   Val Gly Leu Pro Asp Leu Pro Glu Phe Gly Met Leu Leu Gln Leu Asn
                   885                 890                 895
   Asn Arg Leu Asp Thr Ile Ser Trp Tyr Gly Arg Gly Pro His Asp Asn
               900                 905                 910
   Tyr Ala Asp Arg Leu Thr Ser Ala Arg Leu Gly Tyr Tyr Thr Gly Ala
           915                 920                 925
   Val Arg Asp Gln Phe Val Pro Tyr Leu Lys Pro Gln Glu Cys Gly Asn
   930 935 940
   Lys Thr Asp Val Arg Phe Ala Glu Ile Thr Ser Ala Asp Gly Gln Leu
   945 950 955 960
   Gly Leu His Val Glu Ala Thr Ile Pro Phe Glu Ile Asn Ala Leu Pro
   965 970 975
   Trp Thr Pro Glu Glu Leu Glu Ala Asn Asp His Val Tyr Lys Leu Pro
   980 985 990
   Gln Ser Thr Gln Thr Val Ala Arg Ile Asn Tyr Lys Gln Met Gly Val
   995 1000 1005
   Gly Gly Asp Asp Ser Trp Ser Ala Arg Thr His Val Glu Tyr Thr
   1010 1015 1020
   Leu Pro Ala Asn Arg Ala Tyr His Phe Thr Phe Thr Val Arg Pro
   1025 1030 1035
   Val
<210> 6
   <211> 3120
   <212> DNA
   <213> unknown
<220>
   <223> metagenome library 3J_11_DNA
<400> 6
   atgcgaaaga aactggtaca cacccctccg gcaaatggat acccggaatg gaacaataat 60
   cccgagactt ttcaattaaa ccgtctaccc gcacatgctt ctatggtagc gtttccatct 120
   ataacagaag cattgtccaa tgattccagc gcatcgccat ggtacgaatc actgaatggt 180
   caatggaagt ttgccttcgc ggagacaccg gagcaacgga ttacatcctt ttatgagaac 240
   aactatgatt cgagtgactg ggacgagatc cccgttcctt ccaactggca gctacaaggg 300
   tacgattatc cccactatac aaatatgacg tacccgtggg tcgagcgtga gcctgaactg 360
   aagccaccat ttgcaccaac aacttataat ccggtgggtt cgtacatccg cacgtttacg 420
   gttcctgctg actggaaaga ccggcctgtc ctgctgcact ttgagggcgt tgaatctgcc 480
   ttttatgtat gggtgaacgg ggagctggtc ggttatagcg aagacacgtt cacacccgca 540
   gaattcgata tcacttcgta tctaacggaa ggtgagaaca agctcgctgt ggaggtatat 600
   cgctggtgtg atgcgagctg gctggagaat caggatttct ggcggttaag cggaatcttc 660
   cgtggtgtat acatgcattc accttcaccg gttcagatcg ccgatttctt tgttcgtact 720
   gaactggatg acgcgtatca ggatgcggag ctcctgctga atttgaaatt ttttaatcat 780
   aatgccccgc agaacactgc cggattgtcc gttcaggcac agctctatga tgcacaacag     840
   cagactgtac tgaagcaacc acttactgcg gccgttactt tccagggtga ggatgaactt     900
   tcatttaatt tgtcagcaga ggttattcaa ccgcttctat ggagcgccga atcccctcat     960
   ctgtatacac ttgtgctgtc cattcaggac gaatcaggcg aaacactgga agcggtccgc    1020
   agccggatcg gattccgcaa gtttgaactg aaggacggcc tgatgcaaat caatggcaaa    1080
   cgcattgtgt tcaaaggcgt caatcgtcat gaattttccc cggataaggg tcgagccatt    1140
   gggcgggaag acatgatccg tgacatcgag ttgatgaagt cctataacgt taacgcggtg    1200
   cgtacatccc attatccgaa tcagtctctc tggtacgaac tgtgtgatga atatggcctt    1260
   tatgtcattg acgaaacgaa tctggaaact cacggcacct gggaatatgg gcaaaaggaa    1320
   atgaatgaaa acaatattcc cgcgagcaaa ccggaatggc gtaataacgt aatagatcgc    1380
   tgtaactcga tgttccagcg ggacaaaaac catgcgtccg ttattatctg gtctctgggt    1440
   aatgaatcct tcggcggaaa taacttcatc gccatgtacg attatctgaa acaagtcgat    1500
   ccaactcgtc tcgttcatta tgaaggtacc ttccattatc gcccttctga ttcggcaagt    1560
   gatattgaat cgacaatgta tatcagtcca caagatgtgg agcaatacgc gcgcatgaag    1620
   ggtccgaaga aaccttatat tatctgcgaa tacagccatg ccatgggtaa ctcctgtgga    1680
   ggtttgcatc tgtattggga tttgttcgat aaatttgatg tgttgcaggg tgcattcatc    1740
   tgggactggg ttgatcagtc cattcgtacc actacggcag acggtgtaga atattttgct    1800
   tatggtggcg atttcggtga atctcctcat gatggcaatt tctgcgggaa cggattgatt    1860
   ctggccgata agacagttac accgaagctg gaagaagtga agaaatgtta tcagaacgtt    1920
   cgtatggaaa ccgttgatgt caaagacggc ctgctgcgca tcaaaaacca gttcctgttc    1980
   acggatctga gcgaatattc gctcgtatgg acagtaactc acgatggcaa atctgtagag    2040
   aacggtacgt tggatatcgc ggtacctcct ggcgaatcgg ttgaagtccg aatcccttat    2100
   acaccatctt ccgatctgtt ccaggaagcg gtgctgactg tatctctggt taccaaagtg    2160
   gcaaccaaat gggcaggagt aggtcatgag attgcctggg atcagttcgt ggtatctccg    2220
   agattgcgtc caatccaatc gatacgtcaa ggacaagaca atgcgccgca ggtacaggat    2280
   ctgcaagatg tattgaaagt ggctacaggc caagtcacgt tgagcttcaa tccagccact    2340
   ggcgcgttaa catcctatca gattaacaat caggagcaat tgctcgcacc tgtccggccg    2400
   aatttttgga gagccatgac agacaatgat atggggaacc gcctgaatga gcgttctgcc    2460
   ttctggagag atgctcacgc taccagcaga ttaattcgct ttgaacacca tgcagacgaa    2520
   caaggttttc ttgtaacgac cgattatacg tgggatcaac atccgggatg tacgctgtcc    2580
   atcacatacc gaattgatgc ggatggtgta ttggaaatca gtcaaaccct tattccaggc    2640
   gtaggcttac ctgatctacc ggaatttggt atgctgctac aactgaataa ccgcttggat    2700
   acaatctctt ggtacggcag aggcccgcat gacaattatg cagaccgtct aaccagtgca    2760
   cgtctcggct attacacagg tgcggttcga gatcaatttg ttccatatct gaagccacaa    2820
   gagtgtggta acaaaacgga tgtacgcttt gcagaaatta cgtcagcgga tggtcagctt     2880
   ggccttcatg ttgaagccac tatacccttt gaaattaatg cgttgccgtg gacaccggaa     2940
   gaactggaag caaacgacca tgtgtacaaa ttacctcaga gcacacagac tgtcgcccgc      3000
   atcaattaca aacaaatggg tgttggcgga gatgacagct ggagcgcacg tacccatgtc      3060
   gaatacacct tgccagccaa ccgcgcgtat cacttcacct ttacggtaag acctgtataa      3120
<210> 7
   <211> 1036
   <212> PRT
   <213> unknown
<220>
   <223> metagenome library 186_B1/330_C6_AA
<400> 7
   Met Arg Asn Lys Phe Ile Tyr Gln Pro Pro Ala Asn Gly Tyr Pro Glu
   1               5                   10                  15
   Trp Asn Asn Asn Pro Asp Ile Phe Gln Leu Asn Arg Gln Gln Ala His
               20                  25                  30
   Ser Tyr Met Met Pro Phe Lys Thr Glu Ser Glu Ala Leu Thr Glu Glu
           35                  40                  45
   His Ala Ser Ser Pro Tyr Tyr Arg Ser Leu Asn Gly Thr Trp Lys Phe
       50                  55                  60
   Ala Phe Ala Glu Thr Pro Asp Gln Arg Ile Lys Pro Phe Tyr Glu Ala
   65                  70                  75                  80
   Asn Tyr Asp Ser Asn Asn Trp Ala Asp Met Pro Val Pro Ser His Trp
                   85                  90                  95
   Gln Leu Gln Gly Tyr Asp Tyr Pro Gln Tyr Thr Asn Val Gln Tyr Pro
               100                 105                 110
   Trp Ser Val Ser Glu Pro Glu Leu Lys Ala Pro Phe Ala Pro Thr Lys
           115                 120                 125
   Tyr Asn Pro Val Gly Ser Tyr Ile Arg Ser Phe Ser Val Pro Glu Glu
       130                 135                 140
   Trp Ala Gly Gln Pro Val Phe Ile Ser Phe Gln Gly Val Glu Ser Ala
   145                 150                 155                 160
   Phe Tyr Val Trp Ile Asn Gly Glu Leu Val Gly Tyr Ser Glu Asp Thr
                   165                 170                 175
   Phe Thr Pro Ala Glu Phe Asp Leu Thr Pro Tyr Leu Ile Ala Gly Glu
               180                 185                 190
   Asn Lys Leu Ala Val Glu Val Tyr Arg Trp Cys Asp Ala Ser Trp Leu
   195 200 205
   Glu Asp Gln Asp Phe Trp Arg Leu Ser Gly Ile Phe Arg Asp Val Tyr
   210 215 220
   Leu Tyr Ser Val Pro Pro Val His Ile Ala Asp Phe Phe Val Arg Ser
   225 230 235 240
   Asn Leu Asp Asp Lys Tyr Arg Asp Ala Glu Leu Ile Ile Asp Ala Lys
   245 250 255
   Val Ala Asn Tyr Phe Asn Asp Ser Val Gly Ser Leu His Leu Glu Thr
   260 265 270
   Phe Leu Phe Asp Gln Asp Lys His Pro Val Gln Lys Gln Ser Leu Leu
   275 280 285
   Ile Lys Glu Gly Met Thr Asp Glu Gln Asn Phe Gln Leu Ser Met Lys
   290 295 300
   Val His Asn Pro Ala Lys Trp Ser Ala Glu Thr Pro Asn Leu Tyr Thr
   305 310 315 320
   Leu Val Leu Ser Leu Lys Asp Ala Leu Gly Asn Cys Leu Glu Ala Ile
   325 330 335
   Ser Cys Arg Thr Gly Phe Arg Gln Phe Glu Leu Lys Asp Gly Leu Met
   340 345 350
   Lys Ile Asn Gly Lys Arg Ile Val Phe Lys Gly Val Asn Arg His Glu
   355 360 365
   Phe Ser Cys Asp Thr Gly Arg Ala Leu Ser Lys Glu Asp Met Ile Lys
   370 375 380
   Asp Ile Lys Leu Met Lys Ala Tyr Asn Ile Asn Ala Val Arg Thr Ser
   385 390 395 400
   His Tyr Pro Asn Gln Ser Tyr Trp Tyr Glu Leu Cys Asp Glu Tyr Gly
   405 410 415
   Leu Tyr Val Ile Asp Glu Thr Asn Leu Glu Thr His Gly Thr Trp Glu
   420 425 430
   Tyr Gly Lys Lys Gly Leu Asn Asp Lys Thr Val Pro Gly Ser Lys Pro
   435 440 445
   Glu Trp Arg Glu Asn Val Leu Asp Arg Cys Asn Ser Met Phe Gln Arg
   450 455 460
   Asp Lys Asn His Pro Ser Ile Val Ile Trp Ser Leu Gly Asn Glu Ser
   465 470 475 480
   Tyr Gly Gly Asp Asn Phe Ile Ala Met His Asp Phe Leu Lys Gln Ala
   485 490 495
   Asp Pro Thr Arg Leu Val His Tyr Glu Gly Ile Phe His Tyr Arg Glu
   500 505 510
   Ser Glu Ala Ala Ser Asp Ile Glu Ser Thr Met Tyr Ile Ser Pro Gln
   515 520 525
   Asp Val Glu Lys Tyr Ala Arg Ser Asn Pro Gln Lys Pro Tyr Ile Ile
   530 535 540
   Cys Glu Tyr Ser His Ala Met Gly Asn Ser Cys Gly Gly Leu His Leu
   545 550 555 560
   Tyr Trp Glu Val Phe Glu Arg Tyr Asp Ile Leu Gln Gly Ala Phe Ile
   565 570 575
   Trp Asp Trp Val Asp Gln Ala Ile Arg Thr Thr Thr Ser Glu Asp Ile
   580 585 590
   Val Tyr Leu Ala Tyr Gly Gly Asp Phe Gly Glu Thr Pro His Asp Gly
   595 600 605
   Asn Phe Ser Gly Asn Gly Leu Ile Phe Ala Asp Arg Ser Val Thr Pro
   610 615 620
   Lys Leu Asp Glu Val Lys Lys Cys Tyr Gln Asn Val Lys Phe Glu Gly
   625 630 635 640
   Ile Gln Leu Asp Glu Gly Arg Ile Cys Val Thr Asn Arg Phe Leu Phe
   645 650 655
   Thr Asp Leu Ser Asp Tyr Leu Leu Lys Trp Glu Ile Ser Leu Glu Gly
   660 665 670
   Glu Ala Ala Gln Ser Gly Thr Leu Pro Ile Ser Leu Pro Pro Gly Asp
   675 680 685
   Asn Arg Glu Ile Val Ile Pro Tyr Ser Pro Val Asn Thr Tyr Asp Lys
   690 695 700
   Glu Ala Ile Leu Thr Val Ser Phe Val Thr Ile Glu Ser Ser Lys Trp
   705 710 715 720
   Ala Ser Ala Gly His Glu Val Ala Trp Glu Gln Phe Ala Leu Thr Ser
   725 730 735
   Tyr Leu Pro Ala Arg Arg Glu Thr Asp Leu Asn Gln Ala Pro Ile Asn
               740                 745                 750
   Leu Thr Glu Leu Glu Glu Asp Val Leu Ile Ser Ala Gly Asp Val Ser
           755                 760                 765
   Leu Lys Phe Ser Arg Ile Thr Gly Asp Leu Ser Ser Tyr Val Ser Ser
       770                 775                 780
   Gly Lys Glu Cys Leu Leu Glu Pro Val Arg Pro Asn Phe Trp Arg Ala
   785                 790                 795                 800
   Val Thr Asp Asn Asp Leu Gly Asn Lys Leu Pro Glu Arg Cys Gly Val
                   805                 810                 815
   Trp Lys Ala Ala Pro Asp Asn Arg Gln Leu Ile Ser Phe Lys Trp Lys
               820                 825                 830
   Leu Glu Gly Leu Cys Cys Val Ile Thr Ser His Tyr Thr Ile Ala Thr
           835                 840                 845
   Asn Pro Ile Thr Ser Leu Thr Ile Glu Tyr Gln Ile Val Ala Asp Gly
       850                 855                 860
   Thr Ile His Ile His Glu Gln Leu Ile Pro Gly Asn Gly Leu Pro Glu
   865                 870                 875                 880
   Ile Pro Glu Phe Gly Met Leu Phe Val Leu Asp Asn Arg Leu Asp Thr
                   885                 890                 895
   Val Asp Trp Tyr Gly Arg Gly Pro His Glu Asn Tyr Trp Asp Arg Asn
               900                 905                 910
   Thr Gly Ala Arg Ile Gly Arg Tyr Ser Gly Lys Val Gln Asp Gln Phe
           915                 920                 925
   Val Pro Tyr Leu Lys Pro Gln Glu Cys Gly Asn Lys Thr Asp Val Arg
       930                 935                 940
   Phe Ala Ser Leu Thr Glu Gly Thr Asn Gly Thr Gly Leu Gln Ile Val
   945                 950                 955                 960
   Ala Pro Ser Leu Met Glu Leu Cys Ala Leu Pro Trp Thr Pro Asp Glu
                   965                 970                 975
   Leu Glu Ala Ser Asp His Val Tyr Lys Leu Pro Pro Ser Asn Lys Thr
               980                 985                 990
   Val Leu Arg Val Asn Tyr Lys Gln  Met Gly Val Gly Gly  Asp Asn Ser
           995                 1000                1005
   Trp Gly Ala Arg Thr His Glu Pro Phe Thr Leu Pro Ser Asn Arg
   1010 1015 1020
   Ser Tyr Ser Phe Ala Phe Thr Leu Gln Pro Leu Met Pro
   1025 1030 1035
<210> 8
   <211> 3111
   <212> DNA
   <213> unknown
<220>
   <223> metagenome library 186_B1/330_C6_DNA
<400> 8
   atgcgcaata aatttattta tcagccgcct gctaacggct atcccgaatg gaataataac      60
   cctgacattt ttcaattaaa tcgccagcaa gcccattctt acatgatgcc tttcaagact     120
   gaatcggaag cgcttactga agaacatgcc agttcccctt attacaggtc actgaatgga     180
   acatggaaat tcgcttttgc agaaacgccg gatcagcgca tcaaaccttt ctacgaggca     240
   aactatgatt ccaacaattg ggcggacatg cccgtaccgt cgcactggca gcttcaaggc     300
   tatgactatc cccaatatac gaatgtacag tacccatggt ccgtttcgga accggaattg     360
   aaagcgcctt ttgctccaac caaatacaat cccgtaggct cctatatccg ttccttctcc     420
   gttccggaag agtgggcagg gcaacctgtt tttattagct ttcaaggtgt tgaatccgct     480
   ttctatgtct ggattaacgg tgaattggtt ggatacagcg aagatacttt tacacctgcc     540
   gaattcgacc ttacccctta tctgattgcc ggcgagaata agcttgccgt tgaagtatac     600
   cgttggtgcg atgccagctg gcttgaggac caagacttct ggaggctgag cggtattttt     660
   cgcgatgtct acctttactc cgtccctcct gtccatatcg cagatttttt cgtgcggtca     720
   aatctagatg acaaataccg agacgcagaa ctaataatag acgctaaagt agccaattat     780
   ttcaatgata gcgttggctc tcttcatctt gagacgtttc tattcgatca agacaagcac     840
   ccggtccaga aacaatccct cctgattaag gaaggtatga cggatgaaca gaacttccag     900
   ctttccatga aggtacataa ccctgcgaag tggagcgcag aaactccaaa tctgtatacg     960
   cttgttttgt cattaaagga cgctctcggc aattgccttg aagcgatcag ctgccggacg    1020
   ggtttccgcc aatttgagct caaagacgga ttgatgaaaa ttaatggcaa aagaattgtt    1080
   tttaaaggcg tgaacagaca tgagttttcc tgcgatacag ggagagcatt aagtaaagaa    1140
   gatatgatta aagacattaa gctgatgaaa gcctataata tcaatgccgt acgcacctca    1200
   cactatccga atcaatccta ctggtacgag ctttgcgatg agtacggctt gtatgtcatc    1260
   gacgaaacga atttggaaac acatggtact tgggaatatg ggaaaaaggg tctgaacgac    1320
   aaaaccgtgc cgggcagcaa acccgaatgg cgtgagaacg tactggatcg ctgcaactct    1380
   atgtttcaga gagacaagaa ccatccctct atcgtcattt ggtcgctagg caacgaatcc    1440
   tacggaggag acaacttcat tgccatgcat gattttctta aacaagcgga tccgacaaga    1500
   ctcgttcatt atgaaggcat attccattac agggagtcag aagcggctag tgatatcgag 1560
   tctaccatgt atatctctcc gcaagacgta gagaaatacg ccagaagcaa ccctcagaaa 1620
   ccttatatca tatgtgaata tagtcacgct atgggtaact cctgcggcgg acttcatctg 1680
   tattgggaag tgttcgagcg ctatgatatt ctacaaggtg cttttatttg ggactgggta 1740
   gatcaagcga ttcgaacgac aacgagcgaa gacattgtat atttggctta cggcggtgat 1800
   tttggtgaaa caccccatga cggcaatttc agtggaaacg ggttaatctt cgccgatcgc 1860
   agtgtcaccc ctaaactcga tgaagtgaaa aaatgctacc agaatgtgaa gttcgaaggt 1920
   attcagctcg atgaaggacg catttgcgtt acgaatcgtt ttttatttac ggatcttagc 1980
   gactatcttc tgaaatggga gattagcctt gagggggaag cagcccaaag tggaacgctg 2040
   cctatttcac ttcctcctgg tgacaataga gaaatcgtca ttccttattc gccagttaat 2100
   acctacgaca aagaagcaat cttgaccgtt tctttcgtaa ccatagaaag ctcgaagtgg 2160
   gcttctgctg gccatgaagt cgcatgggag cagtttgctc tcacgtctta tttaccggca 2220
   agacgggaaa ccgacctcaa tcaggctcct atcaacctaa ctgagcttga agaggatgtt 2280
   ctcatctccg caggagatgt ttccttgaag tttagccgaa tcactggaga tctgtcgtct 2340
   tacgtttctt cgggcaagga atgcttattg gagccggtaa gaccgaattt ctggcgcgcc 2400
   gtgaccgata atgatttggg taataagctg cctgaacgtt gcggtgtatg gaaggctgcc 2460
   cccgataatc gacagttgat ttcattcaaa tggaaactcg aaggcctatg ttgcgtcatt 2520
   acaagtcatt acacaattgc aacgaatccc attaccagcc tcacaatcga gtatcaaatt 2580
   gttgccgatg gaaccatcca tatccatgaa caattgatac ctggtaatgg attgccggaa 2640
   attccggaat tcggcatgct attcgtgcta gataatagac tcgacaccgt tgattggtat 2700
   ggtcgcggac cgcatgagaa ctattgggat cgcaataccg gagcccgtat cggtcgttat 2760
   tctgggaagg tgcaggatca gtttgtccct tacttgaaac cgcaagaatg cggcaacaaa 2820
   actgacgtca ggtttgccag ccttactgaa ggtaccaatg gaactggctt gcagattgta 2880
   gccccatctt taatggaatt atgtgccctt ccatggacac cagatgagtt ggaagccagc 2940
   gatcatgttt acaagctgcc gccaagcaat aaaaccgttt tgcgcgtcaa ttacaagcaa 3000
   atgggtgtag gcggcgataa cagctggggc gcccgcacgc atgagccatt tactcttcct 3060
   tccaatcgtt cgtacagctt tgcgtttacg ttgcagcctc taatgccata g 3111
<210> 9
   <211> 1037
   <212> PRT
   <213> Paenibacillus sp. JDR-2
<400> 9
   Met Arg Ala Lys Leu Asn Tyr Gln Pro Pro Ala Asn Gly Tyr Pro Glu
   1 5 10 15
   Trp Asn Asn Asn Pro Glu Ile Phe Gln Ile Asn Arg Leu Pro Ala His
   20 25 30
   Ala Ser Met Met Ser Tyr Ser Ser Val Glu Glu Ala Leu Lys Gly Asp
   35 40 45
   Lys Thr Ala Ser Glu Arg Tyr Leu Ser Leu Asn Gly Val Trp Lys Phe
   50 55 60
   Ala Phe Ala Glu Thr Pro Asp Gln Arg Ile Gln Asn Phe Tyr Gln Ser
   65 70 75 80
   Asp Tyr Asp Ala Ser Ser Trp Thr Asp Met Pro Val Pro Ser His Trp
   85 90 95
   Gln Phe His Gly Phe Asp Tyr Pro Gln Tyr Thr Asn Ile Arg Tyr Pro
   100 105 110
   Trp Ile Glu Ser Glu Pro Glu Leu Gln Pro Pro Phe Ala Pro Thr Lys
   115 120 125
   Tyr Asn Pro Val Gly Ser Tyr Ile Arg Ser Phe Gln Val Pro Gln Ser
   130 135 140
   Trp Ser Gly Gln Pro Val Tyr Leu Ser Phe Gln Gly Val Glu Ser Ala
   145 150 155 160
   Phe Tyr Val Trp Leu Asn Gly Glu Leu Val Gly Tyr Ser Glu Asp Thr
   165 170 175
   Phe Thr Pro Ser Glu Phe Asp Ile Thr Pro Tyr Leu Thr Ser Gly Glu
   180 185 190
   Asn Lys Leu Ala Val Glu Val Tyr Arg Trp Cys Asp Ala Ser Trp Leu
   195 200 205
   Glu Asp Gln Asp Phe Trp Arg Leu Ser Gly Ile Phe Arg Asp Val Tyr
   210 215 220
   Val Tyr Ser Lys Pro Lys Val Ser Ile Thr Asp Phe Phe Ala Arg Thr
   225 230 235 240
   His Leu Asp Glu Glu Tyr Arg Asn Ala Glu Leu Ala Val Asn Val Thr
   245 250 255
   Leu Glu Asn Tyr Phe Asn Glu Ala Ser Glu Gly Ile Arg Val Glu Ala
   260 265 270
   Gln Leu Tyr Asp Ala Asn Gln Gln Ala Val Trp Thr Glu Pro Val Ala
   275 280 285
   Val Thr His Ser Phe Ala Ser Asp Gly Glu Gln Gln Leu Ser Leu Ser
   290                 295                 300
   Gly His Val Glu Asn Pro Leu Lys Trp Ser Ala Glu Ala Pro His Leu
   305                 310                 315                 320
   Tyr Thr Leu Val Leu Ser Val Lys Asn Asn Ser Gly Leu Ile Glu Thr
                   325                 330                 335
   His Ser Ser Lys Val Gly Phe Arg Thr Phe Gly Ile Lys Asp Gly Leu
               340                 345                 350
   Met Arg Ile Asn Gly Lys Arg Ile Ile Phe Lys Gly Ala Asn Arg His
           355                 360                 365
   Glu Phe Ser Cys Asp Thr Gly Arg Ala Leu Ser Lys Glu Asp Met Ile
       370                 375                 380
   Arg Asp Ile Thr Leu Met Lys Ser His Asn Ile Asn Ala Val Arg Thr
   385                 390                 395                 400
   Ser His Tyr Pro Asn Gln Ser Val Trp Tyr Glu Leu Cys Asp Glu Tyr
                   405                 410                 415
   Gly Leu Tyr Val Ile Asp Glu Thr Asn Leu Glu Thr His Gly Ser Trp
               420                 425                 430
   Thr Phe Gly Gln Gln Asp Leu Pro Ala His Thr Val Pro Gly Ser His
           435                 440                 445
   Pro Glu Trp Arg Ala Asn Val Leu Asp Arg Cys Asn Ser Met Phe Gln
       450                 455                 460
   Arg Asp Lys Asn His Pro Ser Val Ile Ile Trp Ser Leu Gly Asn Glu
   465                 470                 475                 480
   Ser Phe Gly Gly Asp Asn Phe Ile Ala Met His Asp Tyr Leu Lys Glu
                   485                 490                 495
   Ala Asp Pro Thr Arg Ile Val His Tyr Glu Gly Val Phe His Tyr Arg
               500                 505                 510
   Pro Ser Glu Ala Ala Ser Asp Ile Glu Ser Thr Met Tyr Val Arg Pro
           515                 520                 525
   Asn Glu Val Glu Gln Tyr Ala Leu Asn Asn Pro Lys Lys Pro Tyr Ile
       530                 535                 540
   Leu Cys Glu Tyr Ser His Ala Met Gly Asn Ser Cys Gly Gly Leu His
   545                 550                 555                 560
   Lys Tyr Ala Glu Leu Phe Asp Lys Tyr Glu Ile Leu Gln Gly Gly Phe
   565 570 575
   Ile Trp Asp Trp Val Asp Gln Ala Ile Arg Thr Lys Thr Ala Asp Gly
   580 585 590
   Val Glu Tyr Leu Ala Tyr Gly Gly Asp Phe Gly Glu Ser Pro His Asp
   595 600 605
   Gly Asn Phe Ser Gly Asn Gly Leu Leu Phe Ala Asp Arg Ser Val Thr
   610 615 620
   Pro Lys Leu Leu Glu Val Lys Lys Cys Tyr Gln Asn Ile Ser Ile Ser
   625 630 635 640
   Asp Ile Asn Leu Ala Ala Gln Thr Ile Arg Val Thr Asn Lys Asn Leu
   645 650 655
   Phe Thr Asp Leu Gly Val Tyr Asp Val Lys Trp Glu Ile Gln His Glu
   660 665 670
   Gly Lys Thr Val Gln Lys Asp Val Leu Lys Val Ser Leu Ala Pro Gly
   675 680 685
   Glu Thr Thr Glu Val Thr Ile Pro Tyr Ser Pro Ser Phe Cys Glu Gln
   690 695 700
   Glu Ala Val Leu Thr Ile Ser Phe Val Ile Pro Glu Glu Ser Lys Trp
   705 710 715 720
   Ala Glu Ala Gly His Glu Ile Ala Trp Glu Gln Phe Val Leu Ser Pro
   725 730 735
   Arg Val Val Glu Val Tyr Ser Asn Val Thr Asp Ala Val His Ala Pro
   740 745 750
   Leu Gln Leu Asn Glu Glu Ser Glu Ser Leu Gln Ile Ser Asn Asp Val
   755 760 765
   Leu Ser Val Thr Phe Asn Arg Leu Thr Gly Asp Ile Val Ser Tyr Arg
   770 775 780
   Ser Gly Asp Lys Glu Lys Leu Leu Glu Pro Ile Arg Pro Asn Phe Trp
   785 790 795 800
   Arg Ala Ala Thr Asp Asn Asp Phe Gly Asn Lys Met Pro Glu Arg Cys
   805 810 815
   Ser Ile Trp Lys Asp Ala Gly Ser Thr Lys Arg Leu Ile Gln Phe Thr
   820 825 830
   Trp Thr Ala Ala Gln Asp Arg Cys His Val Thr Thr Val His Gln Leu
   835 840 845
   Ala Thr Asn Pro Val Ser Leu Leu Thr Ile Gly Tyr Thr Val His Ser
   850 855 860
   Asn Gly Ser Ile Asp Ile His Glu Gln Leu Leu Pro Gly Gly Asp Lys
   865 870 875 880
   Leu Pro Glu Ile Pro Glu Phe Gly Met Leu Leu Val Leu Asp Gly Asn
   885 890 895
   Leu Asp Thr Ile Ser Trp Tyr Gly Leu Gly Pro Gln Asp Asn Tyr Trp
   900 905 910
   Asp Arg Lys Thr Gly Ala Lys Leu Gly Gln Tyr Ser Gly Thr Val Lys
   915 920 925
   Asp Gln Phe Val Pro Tyr Leu Arg Pro Gln Glu Cys Gly Asn Lys Thr
   930 935 940
   Asp Val Arg Tyr Ala Glu Leu Thr Asn Gly Ala Asp Gly Asn Gly Ile
   945 950 955 960
   Arg Phe Asp Ser Ala Ala Val Met Glu Ile Asn Ala Leu Pro Trp Thr
   965 970 975
   Pro Asp Glu Leu Glu Ala Ser Asp His Pro Tyr Lys Leu Pro Val Ser
   980 985 990
   Phe Lys Thr Val Val Arg Val Asn Asp Lys Gln Met Gly Val Gly Gly
   995 1000 1005
   Asp Asp Ser Trp Gly Ala Pro Ile His Pro Glu Tyr Lys Leu Phe
   1010 1015 1020
   Ala Asn Arg Thr Tyr Ser Leu Ser Phe Thr Leu Ser Leu Ile
   1025 1030 1035
<210> 10
   <211> 3114
   <212> DNA
   <213> Paenibacillus sp. JDR-2
<400> 10
   ttgcgagcca aacttaatta tcagccacct gcaaacggct atccggaatg gaacaataat    60
   cctgaaatct tccaaatcaa ccgtcttcct gcacacgctt ccatgatgtc gtacagctct   120
   gtggaagaag ctttgaaggg tgacaaaacg gcttccgaac gttatctgtc gttaaacggg   180
   gtttggaaat tcgcctttgc cgagacgccg gatcagcgca ttcagaactt ctatcaatcg   240
   gattacgatg ccagcagctg gaccgacatg cccgttccgt cccattggca gttccacggc   300
   ttcgattatc cgcaatatac gaatatccgt tatccatgga tcgaatcgga accggaattg   360
   cagccgccct ttgctccgac caaatataat ccggttggtt cctatatccg cagctttcaa 420
   gttccgcaat cctggagtgg tcaaccggta tacttgagct tccaaggcgt tgaatccgcc 480
   ttctacgtct ggttgaacgg cgagttggtc ggctatagcg aggacacctt taccccgtcc 540
   gaattcgata tcactcctta tttaacaagc ggcgagaata agctggccgt agaagtgtac 600
   cggtggtgcg atgcaagctg gctggaggat caggacttct ggcgcctcag cggtattttc 660
   cgcgatgtct acgtgtattc caaaccaaag gtgagtatca cggacttctt tgcgcgtacc 720
   cacttggatg aagagtaccg taatgcagaa ttagcagtta acgtcactct cgaaaattat 780
   tttaacgaag catcggaagg catccgcgtt gaagcccaat tatatgatgc taatcaacag 840
   gccgtatgga cggagcctgt ggcagttacc cattcgttcg caagcgacgg cgagcaacag 900
   ctatcgctgt ccggtcatgt ggagaaccct cttaaatgga gcgccgaagc acctcattta 960
   tatacgcttg tgttatcggt taagaataat tccggcttga tcgaaacgca tagcagcaag 1020
   gtgggcttcc gaacgttcgg gatcaaggac ggattgatgc ggattaacgg caaacggatt 1080
   attttcaaag gcgcaaaccg ccatgaattt tcctgcgata ccggccgggc tttatccaaa 1140
   gaggatatga tccgggatat cacgctcatg aaatcccata acattaatgc ggttcgtacc 1200
   tcccactacc cgaatcaatc ggtctggtat gagctatgcg acgaatacgg tttatacgtc 1260
   attgatgaaa cgaatctgga aacgcacggt tcctggacct tcggtcagca ggatttgccc 1320
   gcgcatacgg ttccgggcag ccatccggag tggagagcca acgtgctgga tcgctgcaat 1380
   tcgatgtttc agcgggacaa gaatcatcct tcggttatca tctggtcgct tggcaatgaa 1440
   tcctttggcg gcgataactt tatcgctatg catgattatt tgaaggaagc cgaccctact 1500
   agaattgtgc attacgaagg cgtgttccat taccgtcctt ccgaagctgc gtcggatatc 1560
   gaatccacga tgtatgttcg tcctaacgag gtagagcaat atgccttgaa taatccgaaa 1620
   aaaccttata ttctgtgcga atactcccat gcgatgggca actcttgcgg cgggcttcat 1680
   aaatacgcgg agctgttcga caaatacgag attttgcaag gcggatttat ctgggattgg 1740
   gtagatcagg cgatccggac caaaacagct gacggcgtgg aatatctggc ctatggcggc 1800
   gatttcggcg aatccccgca tgacggcaac ttcagcggca acggcctgct gtttgctgac 1860
   cgctccgtaa ctccaaagct gttagaagtg aagaaatgct accagaacat ttcgatttcg 1920
   gatatcaatc tcgcggctca gacgattcgc gtaacgaata aaaacctgtt cacggacctt 1980
   ggcgtttacg atgtgaaatg ggaaatccaa catgaaggta aaacggttca gaaggatgtt 2040
   ctgaaggtgt cgctggcacc aggcgagact acggaggtaa caatacctta tagcccttcg 2100
   ttctgcgagc aagaagcggt gctgactatt tccttcgtta ttccggagga atcgaaatgg 2160
   gcggaagccg gtcatgaaat cgcttgggag cagtttgtat tgtcgccgcg cgtggtggag 2220
   gtttacagca atgttaccga tgccgtccat gctcctctgc aattaaacga agaatccgag 2280
   tcgctgcaaa tcagcaatga cgtgttgtcg gttaccttta accgtctgac tggcgatatc 2340
   gtatcctatc gctcgggaga caaagagaaa ttgcttgaac cgattcgtcc taacttttgg 2400
   cgtgcagcta ccgataatga cttcggaaac aaaatgccgg agcgctgctc gatttggaag      2460
   gatgccggaa gcacaaagcg tcttattcag tttacttgga ccgcggcaca agaccgctgc      2520
   catgtcacaa ccgtacacca gctggcaaca aatcctgtat ctttgcttac gatcggctat      2580
   acggttcact ccaacggctc gattgatatt catgaacagt tgctgcctgg cggcgacaag      2640
   cttcctgaaa ttccggaatt cggcatgctg ctcgttctcg acggcaatct cgatactatc      2700
   tcctggtacg gcttagggcc gcaggataat tactgggatc gtaaaacagg cgctaaatta      2760
   ggccaataca gcggtacggt taaagaccaa tttgtccctt acctgcgccc tcaggaatgc      2820
   ggcaataaga cggatgtccg ttatgcagag cttacaaacg gtgctgatgg gaacggtatt      2880
   cgctttgaca gcgctgcagt aatggagatc aatgcccttc catggacgcc ggatgagctt      2940
   gaagctagcg accacccgta caaactgcct gtctcgttca aaacggtcgt acgcgtaaac      3000
   gacaaacaaa tgggcgttgg cggcgatgac agctggggtg ctcctattca tccggaatat      3060
   aagctattcg ctaaccggac ttatagctta tccttcaccc tttctttgat ctaa            3114